(19)

Europäisches
Patentamt
European
Patent Office
Office européen
des brevets

(11)  **EP 2 468 764 A1**

(12)  # EUROPEAN PATENT APPLICATION

(43) Date of publication:
**27.06.2012 Bulletin 2012/26**

(51) Int Cl.:
***C07K 14/435*** (2006.01)      ***C07K 14/00*** (2006.01)

(21) Application number: **10252230.7**

(22) Date of filing: **24.12.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
• **Rijksuniversiteit te Groningen
9712 CP  Groningen (NL)**
• **European Molecular Biology Laboratory (EMBL)
69117 Heidelberg (DE)**
• **Fundació Privada
Centre de Regulació Genòmica (CRG)
08003 Barcelona (ES)**
• **Fundació Privada Institució Catalana De
Recerca I Estudis Avançants
08010 Barcelona (ES)**

(72) Inventors:
• **Quax, Wilhelmus Johannes
9707 PT Kropswolde (NL)**

• **Tur, Vicente R.
69117 Heidelberg (DE)**
• **Serrano, Luis
69117 Heidelberg (DE)**
• **Van der Sloot, Albert Martinus ,**

**08003 Barcelona (ES)**
• **Cool, Robbert H.,**

**9713 AV Groningen (NL)**
• **Van Assen, Aart H.G. ,**

**9712 CP Groningen (NL)**

(74) Representative: **Goodfellow, Hugh Robin
Carpmaels & Ransford
One Southampton Row
London
WC1B 5HA (GB)**

(54)    **TNF family ligand variants**

(57)    The present invention relates to variants of TNF family ligands which have been mutated at the ligand trimerisation interface so that they are not capable of assembling into trimers, and either assemble into dimers or remain as monomers. Such ligands bind to the TNF receptor but are unable to activate it, effectively functioning as competitive inhibitors. The invention also relates to nucleic acids encoding the variants of TNF family ligands, vectors and host cells comprising the nucleic acid and methods for the treatment of diseases associated with aberrant signalling through a TNF receptor.

Figure 2

EP 2 468 764 A1

**Description**

**Background**

The Tumor Necrosis Factor ligand (TNF) family is a family of ligands which are involved in a wide range of biological activities, including cell proliferation and apoptosis. There is a complex balance between immunostimulatory and immunoregulatory functions within this family that ensures that an individual is capable of appropriate immune responses. Genetic polymorphisms or other mutations in the TNF ligand receptor family can result in deregulation of immune homeostasis, which is implicated in pathogenesis. For this reason, the TNF family represents a prime target for therapeutic intervention.

Each of the TNF family of ligands interacts with its cognate receptor(s) to trigger a number of signalling pathways that are important for immune tolerance, in addition to providing both protective and pathogenic effects on tissues (35,36,37). Examples of such proteins include ligands such as Receptor Activator of NF-Kappa Beta-Ligand (RANKL), TNF-related apoptosis-inducing ligand (TRAIL), B-Cell Activating Factor (BAFF), A Proliferation-inducing Ligand (APRIL), TNFalpha, CD30L, CD40L, FasL, Light, and Tumor necrosis factor-like Weak inducer of apoptosis (Tweak), which are implicated in disease conditions such as rheumatoid arthritis, autoimmune diabetes, systemic lupus erythematosus (SLE), Sjörgen's syndrome, experimental autoimmune encephalomyelitis (EAE), inflammatory bowel disease (IBD), autoimmune lymphoproliferative syndrome (ALPS), multiple sclerosis, and cancers such as breast cancer.

RANKL is particularly implicated in disorders associated with reduced bone density, such as osteoporosis, bone lesions due to rheumatoid arthritis (RA), Paget's disease and malignancy induced bone disease because signalling through RANKL results in increased production of bone-resorbing osteoclast cells. Binding of RANKL to its cognate receptor Receptor Activator of NF-Kappa Beta (RANK) expressed on osteoclast progenitor cells is crucial for the differentiation of these progenitor cells into mature osteoclasts, while binding of RANKL to RANK expressed on mature osteoclasts prevents these cells undergoing apoptosis and stimulates their adherence to bone cells. Increased signalling through RANKL and the production of an increased number of bone-resorbing osteoclasts disrupts the delicate homeostatic balance between osteoclasts and bone tissue producing osteoblasts, leading to disorders associated with reduced bone density, such as those mentioned above.

More recently, RANKL has been implicated in breast cancer. Here it is responsible for proliferative changes to the mammary epithelium which can lead to tumorigenesis (45,46). The inventors therefore hypothesise that inhibiting the interaction of RANKL with its cognate receptor RANK could be used for the treatment of cancer.

All TNF ligand family members consist of anti-parallel β-sheets, which self-trimerise into the active form of the ligand. Sequence homology is highest between the residues found at the trimer interface. Once formed, a ligand trimer binds three cognate receptors, with each receptor either binding in the groove between two adjacent ligands or binding directly to one of the ligands. (33,34).

As well as binding to their cognate receptors in order to promote signalling, TNF family ligands also bind to naturally occurring decoy receptors. These decoy receptors are either membrane bound or soluble pseudo-receptors, to which the TNF ligand can bind, without activating cell signalling. Whilst bound to the decoy receptor, the TNF ligand is sequestered away from the active receptor, and receptor-mediated signalling is inhibited.

Osteoprotegerin (OPG) is a soluble decoy receptor for RANKL. It is produced by osteoblasts and binds RANKL, thus preventing RANKL binding to and activating RANK, and resulting in a reduction of osteoclast activity. Likewise, DcR1 and DcR2 are decoy receptors for TRAIL, and DcR3 is a decoy receptor for FasL. These receptors sequester TRAIL or FasL, respectively, away from the active signalling receptors, effectively acting as endogenous competitive inhibitors.

Recently, a number of groups have begun studying the TNF ligand family in an attempt to manipulate aberrant signaling which is often associated with pathogenesis. With regard to inhibition of receptor signaling, much of this work has been modeled on the role of the decoy receptors, and has focused on increasing the sequestration of TNF ligands in order reduce receptor-mediated signaling.

One approach is to use the extracellular ligand binding domain of the ligand's endogenous receptor and fuse it with the Fc domain of IgG. These chimeric soluble receptors function as artificial decoy receptors and prevent the endogenous ligand binding to the endogenous receptor by sequestering it. One well known example is Etanercept (Enbrel®), a chimera between the extracellular ligand binding domain of TNFR2 (p75) and the Fc domain of IgG1 and which is clinically used in the treatment of RA and other immune diseases (39,40). A similar approach has also been used with the RANKL decoy receptor OPG (44).

An alternative approach requires the administration of a trimeric TNF ligand mutant incapable of receptor binding but capable of subunit exchange with the wild-type TNF ligand. This may result in the formation of mixed mutant/ endogenous ligand trimers which are either incapable of receptor binding; as are the trimeric mutant forms, or, if bound, incapable of activating the receptor once bound(47). The trimeric wild-type ligand is essentially "poisoned" by the mutant variant and as a result, inactivated. A drawback of this approach is the heterogeneity of the preparation containing different ratios of wild-type to mutant monomers in the trimer, which makes the effects unpredictable.

Another strategy which has been successfully applied is the administration of anti-ligand antibodies (e.g Denosumab, also known as AMG 162 and Prolia®) (42,43). These antibodies bind to RANKL and prevent it from binding to its cognate receptor, therefore blocking receptor signaling.

Recently, blocking peptides have also been used to block signaling through the RANK/RANKL pathway by binding directly to RANK and blocking the ligand binding site (31,32).

### Description of the invention

The present invention relates to variants of TNF family ligands which have been mutated at the ligand trimerisation interface so that they are not capable of assembling into trimers, and either assemble into dimers or remain as monomers. Such ligands bind to the TNF receptor but are unable to activate it, effectively functioning as competitive inhibitors.

The invention also relates to nucleic acids encoding the variants of TNF family ligands, vectors and host cells comprising the nucleic acid and methods for the treatment of diseases associated with aberrant signalling through a TNF receptor.

Here, the inventors show that disrupting the trimerisation of TNF ligands prevents the formation of an active signaling complex. Furthermore, maintaining the ability of the ligand to bind to its cognate receptor, either in the form of a monomer or a dimer, blocks the receptor, competitively inhibiting signaling. TNF ligand variants which are not capable of trimerisation have the advantage that they are structurally similar to wild-type ligands, and are therefore less prone to proteolytic degradation than blocking peptides. The TNF ligand variants of the present invention therefore have potentially greater stability than blocking peptides due to a decreased rate of degradation and clearance from the body. Further, such a system exploits the TNF ligand signaling pathway in a manner distinct from pre-existing therapies as this approach targets the receptor and not the ligand and it can therefore be used in combination with pre-existing therapies.

#### *Variants of TNF family ligands*

In one aspect the present invention includes a variant of a TNF family ligand which is mutated such that it is not capable of assembling into a trimer, wherein the variant retains the ability to bind to one or more of its cognate receptor(s), but wherein binding to the receptor does not activate the receptor.

Within the context of the present invention, the term "mutated" encompasses substitution to any natural or non-natural amino acid residue, deletion, insertion and addition of amino acid residues.

In another embodiment the TNF ligand variant may include one or more post-translational modifications. Suitable modifications include pegylation, acetylation, formylation, alkylation such as methylation, and glycosylation, as well as labeling with flurophores, radioisotopes, PET, etc. Such modifications may decrease immunogenicity, improve pharmacokinetics, and allow for certain specific applications of the variants (e.g. diagnostics).

As discussed above, TNF ligand trimerisation is required to effect receptor trimerisation, which is in turn required for signalling. Therefore, a variant of a TNF family ligand which is incapable of trimerisation, but able to assemble into a dimer or remain monomeric, and still able to bind its TNF receptor, will effectively block its receptor and therefore inhibit downstream signalling. The variant is therefore functioning as a competitive inhibitor.

#### *Variants capable of assembling into dimers*

In one embodiment, the variant of a TNF family ligand may be capable of assembling into a dimer with another variant of the same TNF family ligand.

This will lead to formation of a TNF ligand dimer (homodimer or heterodimer), which is capable of binding to its cognate receptor, but will not be capable of activating the receptor because receptor trimerisation cannot occur when there is only a ligand dimer present.

In one embodiment the ligand variant has at least 10 fold, at least 100 fold, at least 1000 fold or more higher affinity for assembling into a dimer than assembling into a trimer.

In another embodiment, the ligand variant may have an affinity of at least $10^8$M, at least $10^9$M, at least $10^{10}$M or greater for its cognate receptor(s). Affinity may be defined using the association constant ($K_a$), which is determined using the following formulae:

$$Ka = \frac{[C]}{[R]\,[L]}$$

Wherein [C] is the concentration of the complex, [R] is the concentration of unbound receptor and [L] is the concentration of unbound ligand.

In another embodiment, the ligand variant dissociation constant ($K_d$) of at least $10^{-8}$M, at least $10^{-9}$M, at least $10^{-10}$M or less. Kd is determined using the following formula:

$$Kd = \frac{[R]\,[L]}{[C]}$$

Wherein [C] is the concentration of the complex, [R] is the concentration of unbound receptor and [L] is the concentration of unbound ligand.

In comparison to previous approaches, in particular the trimeric TNF ligand mutants discussed above, the present invention potentially ensures a faster response with a lesser dose, as it allows improvement of the Kd to provide stronger antagonists.

As discussed above, many TNF family receptors bind to their cognate ligands in the groove formed between two ligands of the trimer (e.g. RANK, TNFR1, TNFR2, FAS, CD40, CD27, CD30, DR4 and DR5). In this case a dimer will be required in order to stably bind, and therefore block, a single TNF receptor. Only one receptor will be bound by such a dimer due to the presence of only one cleft in which a receptor can be bound. The receptors will therefore be blocked, and no signalling will occur.

As also discussed above, some TNF family receptors bind their cognate ligands within the structure of the TNF-ligand monomer, generally the solvent exposed surface of the TNF ligand monomer (e.g. APRIL and BAFF). In this embodiment, the dimer is formed of two variant TNF ligands that bind to two TNF receptors. However, receptor activation will not occur due to the absence of the third ligand, which is required for receptor trimerisation.

In one embodiment, the TNF family ligand from which the variant is derived may selected from the group consisting of RANKL (human accession no. AAB86811, mouse accession no. 035235), TRAIL (accession no. P50591), APRIL (accession no. BAE16556), BAFF (accession no: Q9Y275), TNFalpha (accession no. NP_000585), TNFbeta (accession no. P01374.2), CD30L (NM_001244), CD40L (NP_000065), FasL (NP_000630), Light (accession no. 043557), Tweak (BAE16557), GITRL (accession no. AAQ89227), EDA-A1 (accession no. NP_034229.1), and OX40L (accession no. P23510.1). Within this embodiment, the TNF family ligand may be of mammalian origin. More specifically, the TNF family ligand may be human, camel, dog, cat, horse, cow, pig, sheep, camelid, mouse, rat, rabbit, hamster, guinea pig, pig, sheep, and so on.

The variant of a TNF family ligand may be mutated at one or more positions. In certain embodiments, the variant may be mutated at 1, 2, 3, 4, 5, 6, 7, 8, 9, 10, 11, 12, 13, 14, 15, 16, 17, 18, 19, 20 or more positions.

*RANKL variants*

The inventors have illustrated the principle of preparing TNF ligand variants which are incapable of forming trimers using RANKL. In a preferred embodiment the TNF family ligand is RANKL.

The results described herein using RANKL can be equally applied to other TNF family ligands including TRAIL, APRIL, BAFF, TNFalpha, TNFbeta, CD30L, CD40L, FasL, Light, and Tweak, GITRL, EDA-A1, EDA-A2 and OX40L.

In one embodiment the RANKL variant may comprise or consist of a mutation at one or more of positions 168, 194, 212, 220, 229, 256, 271, and 279 in the mouse RANKL sequence, or positions equivalent thereto in orthologous proteins. In the human sequence, these positions are 169, 195, 213, 221, 230, 257, 272 and 280. Mutations at such positions may be to any natural or non-natural amino acid, although mutations to more hydrophilic amino acids are preferred because residues positioned at the trimer interface will not be surface exposed following formation of a trimer, but at least some will be surface exposed for a TNF ligand variant incapable of forming a trimer.

In one embodiment a murine RANKL variant may comprise or consists of one or more of the mutations T168V, K194D, F212Y, C220S, C220A, D229K, S229K, K256D, F271Y and F279Y. In the human RANKL variant, these mutations are T169V, K195D, F213Y, C221S, S230K, K257D, F272Y and F280Y. Therefore, the variant may comprise or consist of 1, 2, 3, 4, 5, 6, 7 or 8 of these mutations.

In one embodiment the RANKL variant may comprises or consist of mutations at positions K194, C220 and F271, or equivalent positions (in the human these are K195, C221 and F272).

In one embodiment the RANKL variant may comprises or consist of the mutations K194D, C220S and F271Y, or equivalent mutations at equivalent positions (in the human these are K195D, C221 S and F272Y).

In another embodiment the RANKL variant may comprise or consist of mutations at positions F212, K256 and F279, or equivalent positions (in the human these are F213, K257 and F280).

In another embodiment the RANKL variant may comprise or consist of the mutations F212Y, K256D and F279Y, or equivalent mutations at equivalent positions (in the human these are F213Y, K257D and F280Y).

In another embodiment the RANKL variant may comprise or consist of mutations at positions T168, S229 and F271, or equivalent positions (in the human these are T169, S230 and F272).

In another embodiment the RANKL variant may comprise or consist of the mutations T168V, S229K and F271Y, or equivalent mutations at equivalent positions (in the human these are T169V, S230K and F272Y).

In another embodiment the RANKL variant may comprise or consist of mutations at positions T168, F212, C220, S229, K256 and F279 or equivalent positions (in the human these are T169, F213, C221, S230, K257 and F280).

In another embodiment the RANKL variant may comprise or consist of the mutations T168V, F212Y, C220A, S229K, K256D and F279Y or equivalent mutations at equivalent positions (in the human these are T169V, F213Y, C221A, S230K, K257D and F280Y).

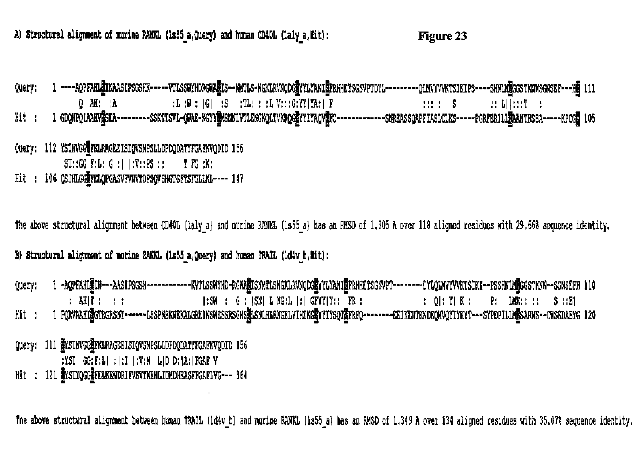

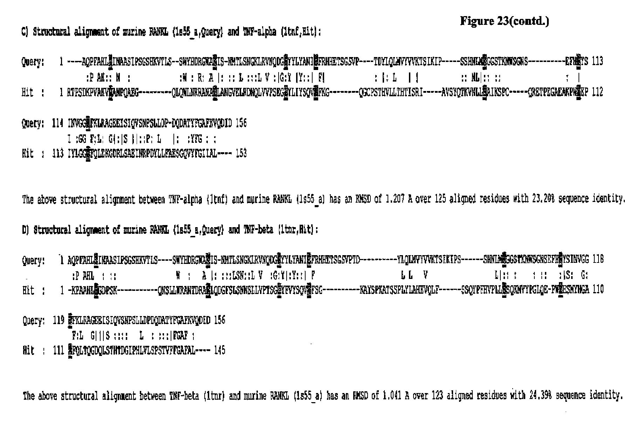

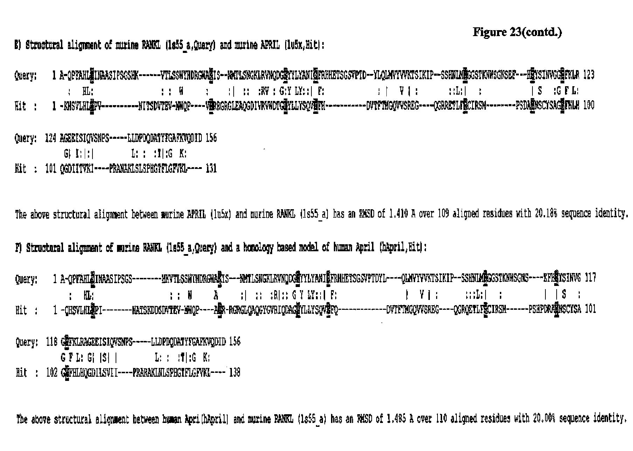

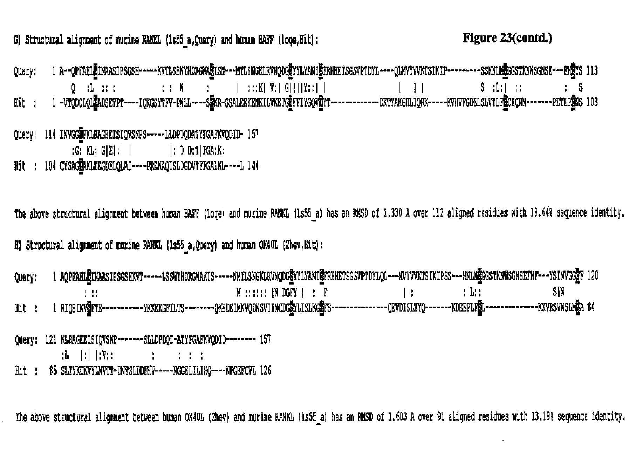

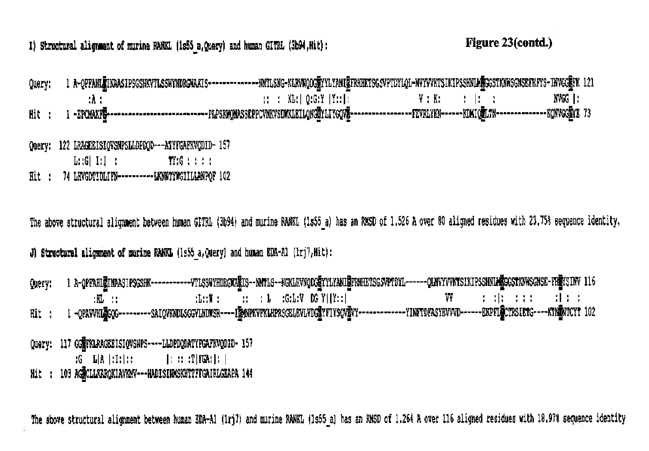

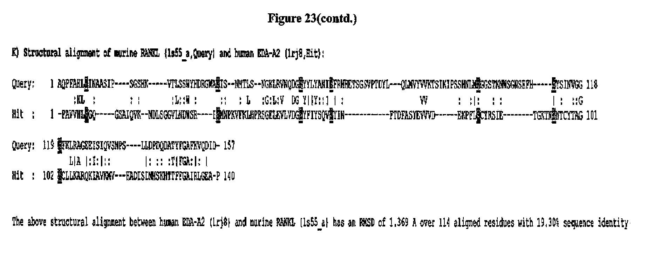

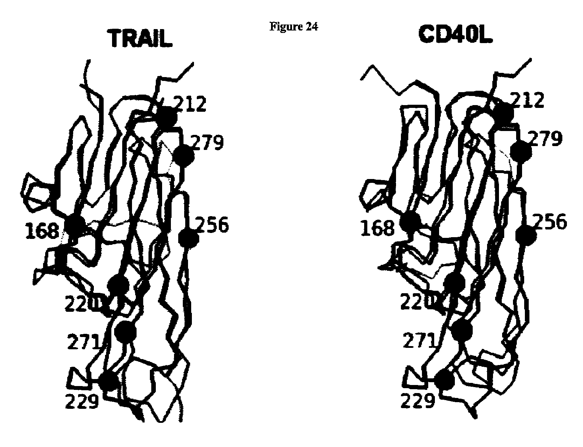

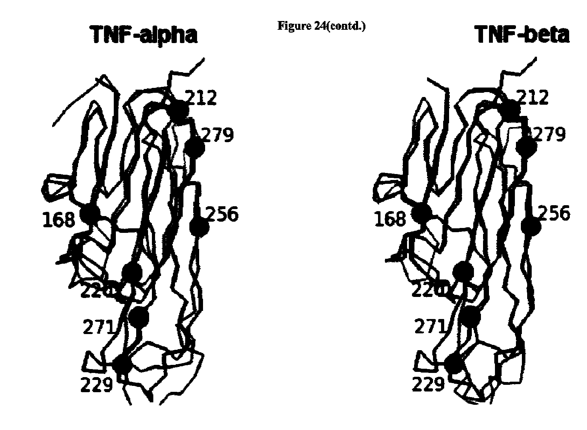

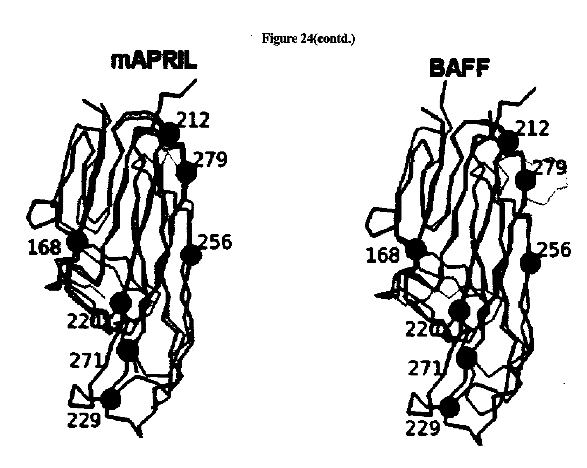

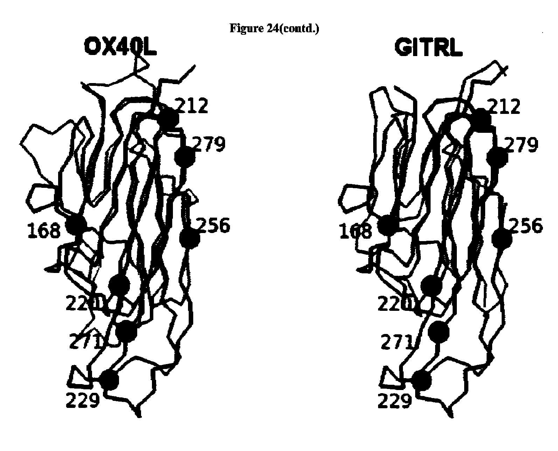

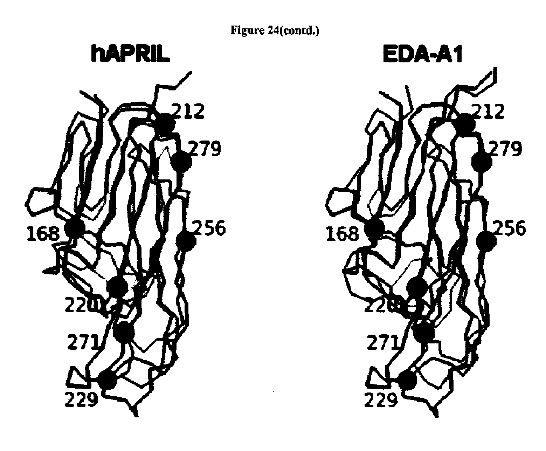

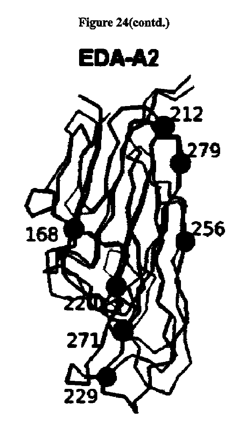

As described above, the present is not limited to RANKL, but can be extended to all members of the TNF family ligand. Therefore, the present invention includes a variant of a TNF ligand variant having mutations at positions equivalent to those discussed above in relation to RANKL. Such equivalent positions are detailed in Table 1, below. These positions are derived from figures 23 and 24.

Table 1 - Structurally equivalent mutations positions in other TNF family ligands

| Mutations position in murine RANKL | Equivalent mutations positions in other TNF family ligands | | | | | | | | | |
|---|---|---|---|---|---|---|---|---|---|---|
| | Human RANKL | Human TRAIL | Human BAFF | Murine APRIL | TNF$\alpha$ | TNF$\beta$ | Human CD40L | Human APRIL | Human OX40L | Human GITRL |
| T168 | T169 | T127 | I150 | V112 | V93 | I68 | I127 | V121 | Q65 | G63 |
| K194 | K195 | F163 | F172 | L132 | L112 | F87 | T147 | L142 | F100 | L94 |
| F212 | F213 | F181 | Y192 | I152 | L131 | I106 | L168 | V162 | Y108 | A102 |
| C220 | C221 | Y189 | L200 | L160 | L139 | V114 | T176 | L170 | Q128 | T126 |
| D229 | S230 | - | - | - | - | - | - | - | - | - |
| K256 | K257 | K224 | R231 | R185 | S157 | S150 | R207 | F194 | V140 | T139 |
| F271 | F272 | L239 | N242 | Y198 | Y191 | L164 | Q220 | Y208 | - | - |
| F279 | F280 | 1247 | 1250 | V206 | V199 | A172 | V228 | V216 | - | - |

In one embodiment the invention includes the TNF ligand variants of SEQ ID NOs: 33-76.

*Variant dimers*

In one aspect the invention includes a dimer comprising or consisting of two of the variants of TNF family ligands of the invention. Such a dimer may include any two TNF ligand variants of the same ligand described above. In a preferred embodiment the dimer comprises or consists of two of the RANKL variants discussed above.

In one embodiment, the dimer of two variants of TNF family ligands may be a heterodimer. The two variants included in the dimer may therefore contain different mutations to each other, relative to the wild-type sequence.

In one embodiment the two ligand variants may be present as a single peptide chain. Within this embodiment the sequences of the two ligand variants may be directly linked, or they may be linked through a linker sequence comprising or consisting of, for example, 1, 2, 3, 4, 5, 6, 7, 8, 9, 10, 15, 20, 30, 40, 50 or more amino acids.

In one embodiment the dimer may comprise or consist of a variant comprising or consisting of mutations at positions C220 and F271 or equivalent positions (human is C221 and F272), and a variant comprising or consisting of mutations at positions F212, K256 and F279 or equivalent positions (human is F213, K257 and F280).

In another embodiment the dimer may comprise or consist of a variant comprising or consisting of the mutations C220S and F271Y or equivalent mutations at equivalent positions (human is C221S and F272Y), and a variant comprising or consisting of the mutations F212Y, K256D and F279T or equivalent mutations at equivalent positions (human is F213Y, K257D and F280T).

In another embodiment the dimer may comprise or consist of a variant comprising or consisting of mutations at positions T168, S229 and F271 or equivalent positions (human is T169, S230 and F272) and a variant comprising or consisting of mutations at positions T168, F212, C220, S229, K256 and F279 or equivalent positions (human is T169, F213, C221, S230, K257 and F280).

In another embodiment the dimer may comprise or consist of a variant comprising or consisting of the mutations T168V, S229K and F271Y or equivalent mutations (human is T169V, S230K and F272Y) at equivalent positions and a variant comprising or consisting of the mutations T168V, F212Y, C220A, S229K, K256D and F279Y or equivalent mutations at equivalent positions (T169V, F213Y, C221A, S230K, K257D and F280Y).

In one embodiment the TNF ligand variant dimer may comprise or consist of two of the variants of SEQ ID NOs: 35-77, with the proviso that the two variant monomers forming the dimer are variants of the same TNF ligand.

In a preferred embodiment, the two ligand variants are SEQ ID NOs: 35 and 36, SEQ ID NOs: 37 and 38. SEQ ID NOs: 39 and 40 or SEQ ID NOs: 41 and 42.

*Variants incapable of assembling into dimers*

In one embodiment, the variant of a TNF family ligand may not capable of assembling into a dimer with other variants of the same TNF ligand. In this embodiment, the variant will not be able to bind a receptor which binds within the cleft formed between two ligands because a ligand dimer will not form and there will therefore be no cleft into which a receptor can bind.

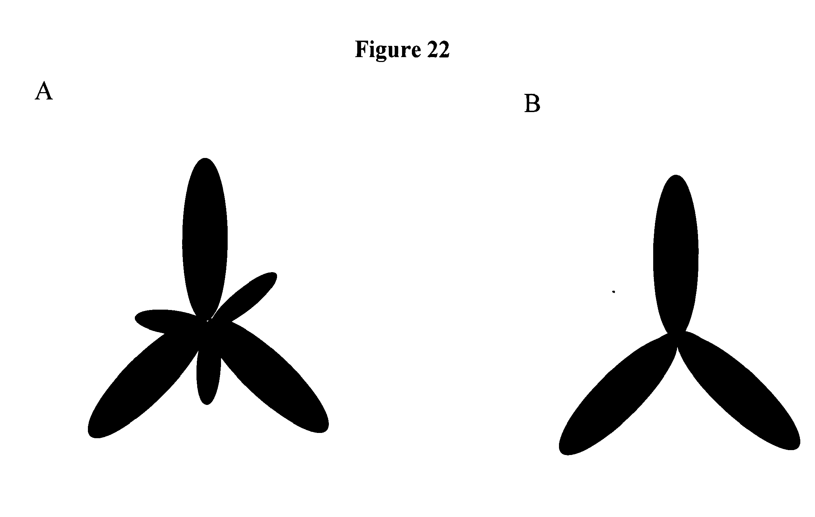

In one embodiment, the ligand variant binds its cognate receptor on the solvent exposed surface rather than in the cleft between two adjacent ligand monomers, as shown in figure 22. This will enable the ligand variant to bind to its cognate receptor without any requirement for the ligand variant to dimerise. Examples of TNF family ligands which bind in such a mode to their cognate receptor, and are therefore useful within this embodiment of the invention are APRIL, BAFF and most likely Tweak.

*Receptor binding*

As discussed above, TNF ligand variants of the invention bind to the TNF receptor but are unable to activate it.

The TNF ligand variants of the invention are considered to "bind to the TNF receptor" if they demonstrate more than 50% of the receptor binding observed with the wild-type ligand at saturating concentration. In other embodiments the ligand variants may demonstrate more than 60%, more than 70%, more than 80%, more than 90%, more than 91%, more than 92%, more than 93%, more than 94%, more than 95%, more than 96%, more than 97%, more than 98%, more than 99%, more than 99.5%, more than 99.6%, more than 99.7%, more than 99.8%, more than 99.9%, more than 99.95%, or more than 99.99% of the receptor binding observed with the wild-type ligand.

The TNF ligand variants of the invention are considered "unable to activate the receptor" if they demonstrate less than 50% of the receptor activation observed with the wild-type ligand at saturating concentration. In other embodiments the ligand variants may demonstrate less than 40%, less than 30%, less than 20%, less than 10%, less than 9%, less than 8%, less than 7%, less than 6%, less than 5%, less than 4%, less than 3%, less than 2%, less than 1%, less than 0.5%, less than 0.4%, less than 0.3%, less than 0.2%, less than 0.1%, less than 0.05%, or less than 0.01% of the receptor activation observed with the wild-type ligand.

In one embodiment, receptor activation may be assessed by measuring the levels of signalling through the receptor.

In one embodiment, the variant of a TNF family ligand according to the invention may have an increased binding affinity for one or more of its cognate receptor(s), compared to the wild-type TNF family ligand. The ligand variant may have increased binding affinity for 1, 2, 3, 4, 5 or more of its cognate receptors.

In one embodiment, the binding affinity for one or more of the cognate receptors may be increased by substituting one or more amino acids residues of the TNF family ligand to any other natural or non-natural amino acid, deleting one or more amino acid residues from the TNF family ligand or inserting one or more amino acid residues into the TNF family ligand.

Within this embodiment, binding affinity may be increased by substituting one or more of the amino acid residues present at the receptor binding interface of the ligand to any other natural or non-natural amino acid, or deleting any of the residues present at the receptor binding interface or inserting one or more additional residues at the receptor binding interface.

In another embodiment, the ligand variant may have an increased binding affinity for one or more of its cognate target receptors, compared to the wild-type TNF family ligand. In this context a "target" receptor refers to a receptor through which signalling can take place, i.e. not a decoy receptor, and which should be inhibited in order to produce a cellular response. Non-target receptors refer to active receptors which are not the main target of inhibition and/or decoy receptors.

Within this embodiment, affinity for the cognate target receptor may be increased by any of the methods discussed above.

In order to increase the binding affinity of a ligand variant for one or more of its cognate target receptors above its affinity for one or more of its decoy receptors, the ligand variant may include one or more amino acid residue substitutions or deletions which decrease the affinity of the ligand variant for one or more if its cognate decoy receptors. In order to increase the binding affinity of a ligand variant for one or more of its cognate target receptors above its affinity for one or more of its non-target receptors, the ligand variant may include one or more amino acid residue substitutions, insertions or deletions which decrease the affinity of the ligand variant for one or more of its non-target cognate receptors. Such a receptor selective effect may be useful in cases where it is beneficial to inhibit the signalling mediated by one cognate receptor but not the signalling mediated by the other receptor(s) of the ligand. For example, in the treatment of Rheumatoid arthritis, it may be beneficial to block TNFalpha mediated signalling via TNFR1 but leave TNFalpha mediated TNFR2 signalling unperturbed. Such receptor selectivity is not possible, or at least not as readily possible, with the approaches used in the prior art.

*Sequence identity, fragments and truncations*

The TNF ligand variants of the invention may have at least 50% sequence identity to any one of the TNF ligands variants described herein and to SEQ ID NOs: 35-78. In one embodiment the variant may have at least 60%, at least 70%, at least 80%, at least 85%, at least 90%, at least 95%, at least 96%, at least 97%, at least 98%, at least 99%, at least 99.5%, at least 99.9% or more sequence identity to SEQ ID NOs: 35-78. The variant may retain the ability to bind its cognate receptor(s).

In one embodiment, the invention also encompasses fragments of the TNF ligand variants described herein and of SEQ ID NOs: 35-78. Such a fragment may be 7, 8, 9, 10, 20, 30, 40, 50, 60, 70, 80, 90, 100, 150, 200, 250, 300 or more amino acids in length. The variant fragment may retain the ability to bind its cognate receptor(s).

In another embodiment, the invention also encompasses TNF ligand variants as described herein which have a truncation at the N-terminus and/or the C-terminus. The truncation may be 1, 2, 3, 4, 5, 6, 7, 8, 9, 10, 11, 12, 13, 14, 15, 16, 17, 18, 19, 20, 30, 40, 50, 60, 70, 80, 90, 100, 150, 200 or more amino acids in length. The truncation may be an internal deletion with the same characteristics. The variant truncate may retain the ability to bind its cognate receptor(s).

In one preferred embodiment, the ligand variant may be a soluble ligand variant. Therefore, the ligand may have been mutated to delete the transmembrane domain. The ligand variant may therefore comprise or consist of amino acid residues 158 - 316 or 160-316 of murine RANKL, and variants around this sequence, such as those described above. The ligand variant may comprise or consist of amino acid residues 159 - 317 or 161-317 of human RANKL, and variants around this sequence, such as those described above. The ligand variant may therefore comprise or consist of amino acid residues 114-281 of human TRAIL, and variants around this sequence, such as those described above.

*Fusion proteins*

In one embodiment the ligand variants of the invention may be fused to a heterologous peptide. This may allow purification of the ligand variant during production, or may confer an additional therapeutic property to the variant for therapeutic or diagnostic use such as increasing retention time in the circulatory system by allow weak binding to abundant blood proteins such as serumalbumin. A particularly suitable heterologous peptide for purification is a hexahistidine tag or a Flag tag.

For heterodimers comprising two differing ligand variants, the heterologous peptide maybe be fused to one of the monomers constituting the heterodimer or to both, alternatively two different heterologous peptide sequences may be fused to the two monomers constituting the heterodimer.

*Nucleic Acids*

In one aspect, the invention includes a nucleotide sequence encoding a variant of a TNF family ligand according to the invention. The nucleic acid may encode any of the variants, truncates or fragments described above. The nucleic acid may be DNA including chromosomal DNA and cDNA or RNA, including mRNA. The mRNA or DNA sequences may or may not include one or more introns.

The nucleic acid may include one or more regulatory sequences such as a promoter, an enhancer, an internal ribosomal entry site (IRES), a Kozak sequence or a ribosomal binding site (RBS) sequence.

The monomers comprising the heterodimeric variants might be encoded in two separate nucleic acid molecules.

One or more ligand variants of the same TNF ligand may be encoded by a single nucleic acid molecule. In one embodiment the nucleic acid molecule may encode two ligand variants of the same ligand. In this embodiment expression of the two ligand variants may be under the control of the same or different promoters, IRES sites or other regulatory sequences.

In another aspect, the invention includes a vector comprising a nucleotide sequence encoding any of the variants, fragments or truncates of the invention. Vectors suitable for use in the method of the invention include plasmids and viruses (including both bacteriophage and eukaryotic viruses), as well as other linear or circular DNA carriers, such as those employing transposable elements or homologous recombination technology. Particularly suitable viral vectors include baculovirus-, lentivirus-, adenovirus- and vaccinia virus- based vectors.

In yet another aspect, the invention includes a host cell comprising a nucleic acid or a vector encoding one or more of the ligand variants of the invention. Suitable examples of host cells include prokaryotic host cells such as bacterial cells, yeast host cells and mammalian host cells. Particularly preferred host cells include *E. coli,* CHO, HEK293 PerC7 cells, *B. subtilis,* insect cells, and yeast such as *S. cerevisiae,* or.

**Receptor-variant complexes**

The invention encompasses complexes which comprise or consist of one or more TNF ligand variants and one or more cognate TNF family receptors.

In one embodiment the complex may comprise or consist of a dimer formed of two TNF ligand variants which are incapable of assembing into a trimer and one or two cognate TNF family receptors.

In another embodiment the complex may comprise or consist of one or two TNF ligand variants which are incapable of assembling into dimers or trimers and one or two cognate TNF family receptors.

**Transgenic animals**

In another aspect, the invention includes a transgenic animal which has been engineered to express one or more of the TNF family variants or dimers disclosed herein.

The animal may be any animal which can be engineered to express one or more TNF ligand variants, for example a mammal. Examples of suitable animals include monkeys, mice, cows, sheep, rabbit, rat, hamster, guinea pig, goat, horses, donkey, pigs, cats, dogs and camels.

**Pharmaceutical compositions**

In one aspect the invention includes a pharmaceutical composition comprising one or more of the variants of a TNF family ligand, the dimers, the nucleotide sequences, the vectors or the host cells of the invention.

In one embodiment the pharmaceutical composition may additionally comprise a pharmaceutically-acceptable carrier, excipient, diluent or buffer. Suitable pharmaceutically acceptable carriers, excipients, diluents or buffers may include liquids such as water, saline, glycerol, ethanol or auxiliary substances such as wetting or emulsifying agents, pH buffering substances and the like. Excipients may enable the pharmaceutical compositions to be formulated into tablets, pills, capsules, liquids, gels, or syrups to aid intake by the subject. A thorough discussion of pharmaceutically acceptable carriers is available in Remington's Pharmaceutical Sciences (38).

The pharmaceutical composition may include a therapeutically effective amount of one or more of the variants of a TNF family ligand, the dimers, the nucleotide sequences, the vectors or the host cells of the invention. A pharmaceutically effective amount is an amount able to treat the disease. The actual amount will depend on a number of factors including the size, weight, age, gender, and health of an individual, and the rate of blood clearance, and will be decided by a clinical practitioner. Generally a pharmaceutically effective amount will be between 1g/kg body weight and 1mg/kg body weight or less.

In one embodiment the pharmaceutical composition may include an additional therapeutic component, in particular, a component useful for the treatment of osteoporosis, rheumatoid arthritis, Paget's disease or malignancy induced bone disease. Such a component may include Denosumab, antiresorptive agents, including bisphosphonates, the selective estrogen-receptor modulator (SERM), raloxifene, calcitonin, denosumab, teriparatide, calcium or vitamin D. In another embodiment the additional therapeutic component may be useful for the treatment of cancer, and may include chemotherapeutics, ERMs, SERMs, or specific biological targeted therapies e.g. Herceptin. In another embodiment the additional therapeutic component may be useful for the reducing inflammation any may include infliximab, etanercept, immunosupresives such as metroxate, and other anti-inflammatory agents such as NSAIDs.

The invention also includes any medical device which may have the pharmaceutical composition of the invention inserted into it or coated onto it. Such devices include, stents, pins, rods, meshes, beads, syringes, plasters, microchips, micro fluidic devices, and stitches.

### Methods of treatment

In another aspect, the invention provides a variant of a TNF family ligand, a dimer, a nucleotide sequence, a vector of, a host cell or the pharmaceutical composition of the invention for use in medicine.

In one embodiment the invention includes a variant of a TNF family ligand, a dimer, a nucleotide sequence, a vector, a host cell or a pharmaceutical composition according to the invention for use in the treatment of osteoporosis, rheumantoid arthritis, Paget's disease, malignancy induced bone disease or cancer, such as breast cancer.

In another embodiment the invention includes a method of treating osteoporosis, rheumatoid arthritis, Paget's disease, malignancy induced bone disease or cancer such as breast cancer comprising administering a pharmaceutically effective amount of a variant of a TNF family ligand, a dimer, a nucleotide sequence, a vector of, a host cell or the pharmaceutical composition of the invention to a patient in need of treatment.

As used herein, the term "treatment" encompasses therapy, and can be prophylactic or therapeutic.

A pharmaceutically effective amount is an amount able to treat the disease. The actual amount will depend on a number of factors including the size, weight, age, gender, health of an individual, and the rate of blood clearance, and will be decided by a clinical practitioner. Generally a pharmaceutically effective amount will be between 1 g/kg body weight and 1 mg/kg body weight or less.

In another embodiment the invention includes the use of a variant of a TNF family ligand, a dimer, a nucleotide sequence, a vector, a host cell or a pharmaceutical composition according to the invention in the manufacture of a medicament for the treatment of osteoporosis, rheumatoid arthritis, Paget's disease, malignancy induced bone disease or cancer such as breast cancer.

The variant of a TNF family ligand, dimer, nucleotide sequence, vector, host cell or pharmaceutical composition according to the invention may be used for the treatment of disease in any animal. The animal may be a mammal such as a camel, dog, cat, horse, cow, pig, sheep, camelid, mouse, rat, rabbit, hamster, guinea pig, pig, sheep. In one embodiment, the mammal may be a human.

The variant of a TNF family ligand, dimer, nucleotide sequence, vector, host cell or pharmaceutical composition according to the invention may be administered to a patient using any one or more of a number of modes of administration which will be known to a person skilled in the art. Such modes of administration may include parenteral injection (e.g. intravenously, subcutaneously, intraperitoneally, intramuscularly, or to the interstitial space of a tissue), or by rectal, oral, vaginal, topical, transdermal, intradermal, intrathecal, intranasal, ocular, aural, pulmonary or other mucosal administration. The precise mode of administration will depend on the disease or condition to be treated.

### Methods of designing variants of TNFfamily ligands

The invention includes a method for producing a variant of a TNF family ligand comprising the steps of:

    a) identifying amino acids in the TNF family ligand that are located in the trimerisation interface as candidates for mutation;

    b) substituting each of one or more residues in the trimerisation interface; and

    c) selecting amino acid substitutions which have a neutral or positive effect on the stability of the dimer but a negative effect on the stability of the trimer.

In one embodiment, the most hydrophilic amino acid substitution at newly solvent exposed positions may be favoured.

In another embodiment the method may further comprise one or more of the following steps:

d) selecting amino acid substitutions to increase the affinity for one or more of the target receptor(s);
e) selecting amino acid substitutions to increase selectivity for one or more of the target receptors, either by increasing affinity for one or more of the target receptor(s) or decreasing affinity for one or more of the non-target receptors, including decoy receptors.
f) producing a variant of a TNF family ligand; and
g) Modifying the variant to improve its properties such as to decrease its immunogenicity or improve its pharmacokinetics. Such modifications may include one or more of pegylation, acetylation, formylation, alkylation such as methylation, and glycosylation and other possible modifications.

In one embodiment, the method may be a method of producing an inhibitory variant of a TNF family ligand.

**Brief description of the Figures**

Figure 1A shows a model of the conversion of a RANKL agonistic trimer into a RANKL antagonistic dimer.

Figure 1B shows the location of the mutated residues in RANKL dimer 1.

Figure 1C shows the location of the mutated residues in RANKL dimer 2.

Figure 2 is a schematic which shows the binding of a TNF family ligand trimer to 3 receptors, and the binding of a TNF family ligand dimer to a single receptor at the only cleft formed between two monomers.

Figure 3 shows capillary electrophoresis of A) wild-type RANKL, B) dimer 1, and C) dimer 2.

Figure 4 shows a surface Plasmon resonance binding curve of A) WT RANKL and B) RANKL D1 and C) RANKL D2 binding to immobilized RANK-FC receptor Figure 5 shows the results of a competitive ELISA using RANKL dimer 2.

Figure 6 shows osteoclastogenesis determination by TRAP (tartrate resistant acid posphatase) assay using A) 100ng/ml wild-type RANKL, B) 100ng/ml RANKL dimer 2, C) 100ng/ml wild-type RANKL and 100ng/ml RANKL dimer 2, and D) untreated.

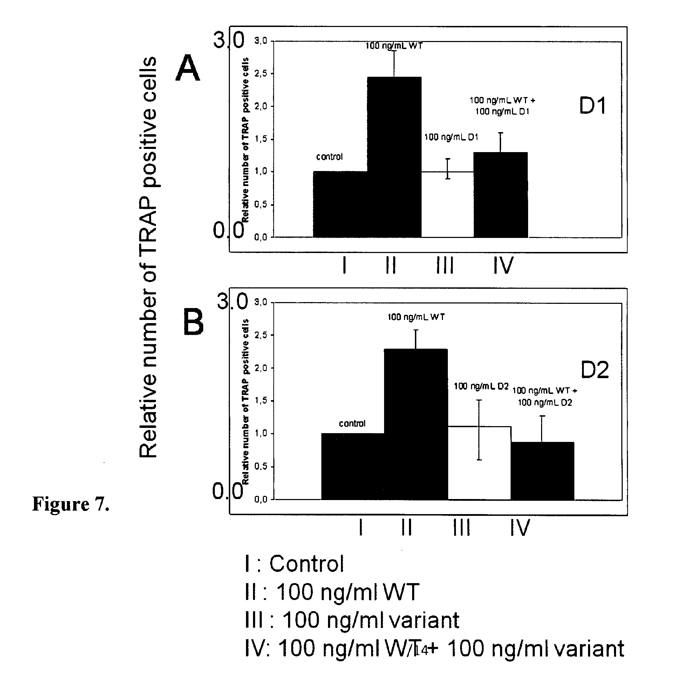

Figure 7 is a graphical representation of the results of Figure 6, A) and B) are duplicates of the same experiment.

Figure 8 shows a circular dichroism wavelength scan of A) dimer 1, and B) dimer 2.

Figure 9 shows a circular dichroism temperature scan of A) dimer 1, and B) dimer 2.

Figure 10 shows analytical gel filtration chromatography for A) flag wild-type RANKL, B) RANKL dimer 2, and C) flag RANKL dimer.

Figure 11 shows analytical gel filtration chromatography, where F is flag wild-type RANKL, E and C are RANKL dimer 1 and D is flag RANKL dimer 1.

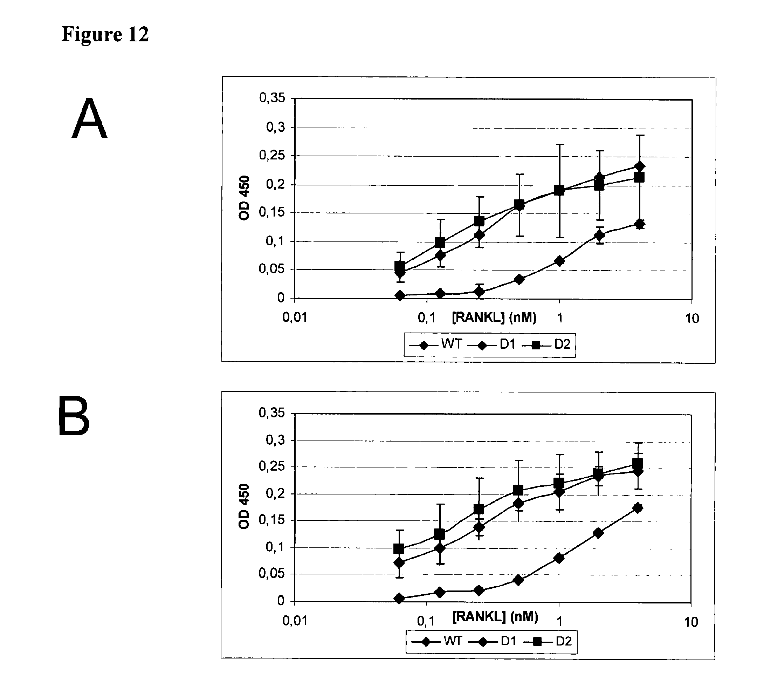

Figure 12 shows ELISA of WT, D1 and D2 binding to immobilized RANK-Fc receptor. A and B are duplicates.

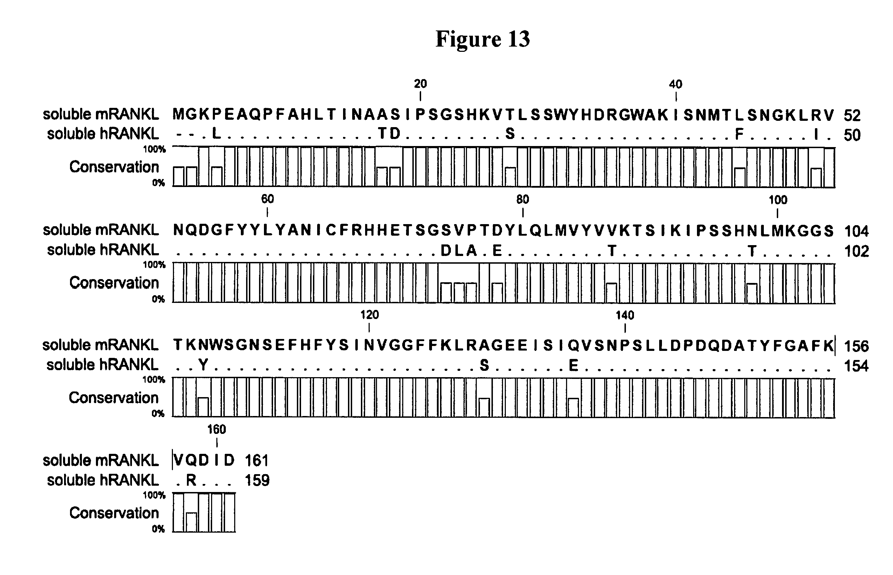

Figure 13 shows the conservation between human and murine RANKL. 143 of the 149 residues (89%) are identical.

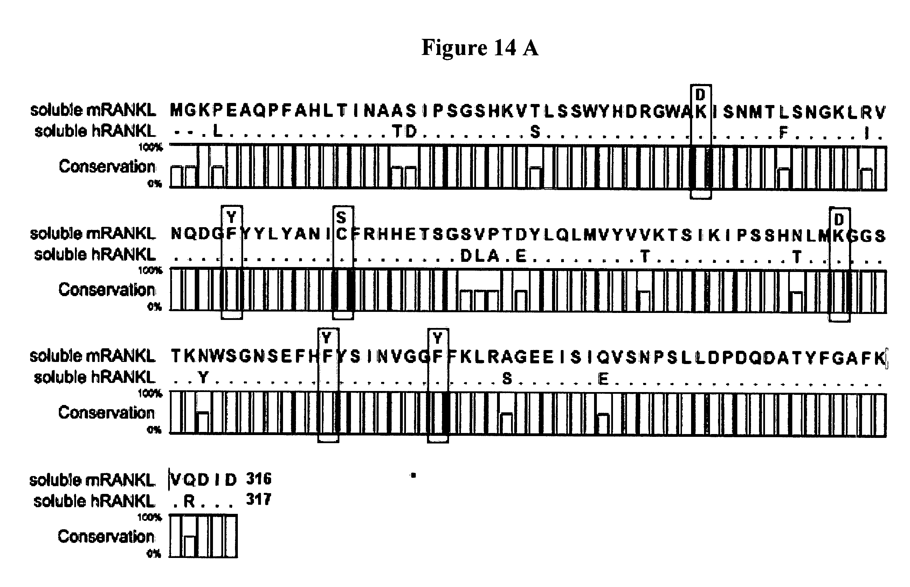

Figure 14A shows the sequence conservation between the human and murine RANKL sequences of dimer 1. Mutations in dimer A are shown in dark blue, mutations in dimer B are shown in light cyan, and mutations shared between dimers A and B are shown in dark green.

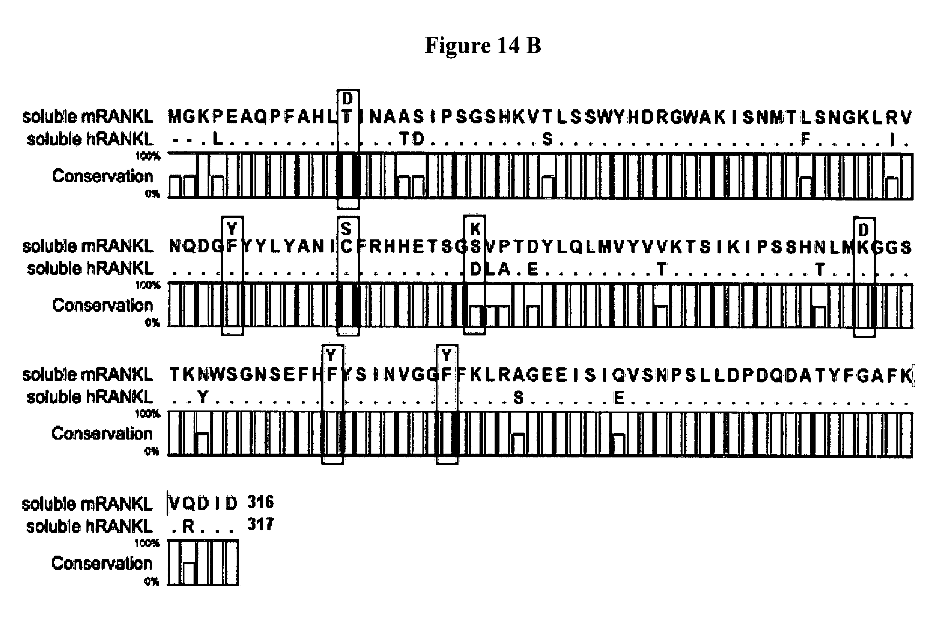

Figure 14B shows the sequence conservation between the human and murine RANKL sequences of dimer 2. Mutations in dimer A are shown in dark blue, mutations in dimer B are shown in light cyan, and mutations shared between dimers A and B are shown in dark green.

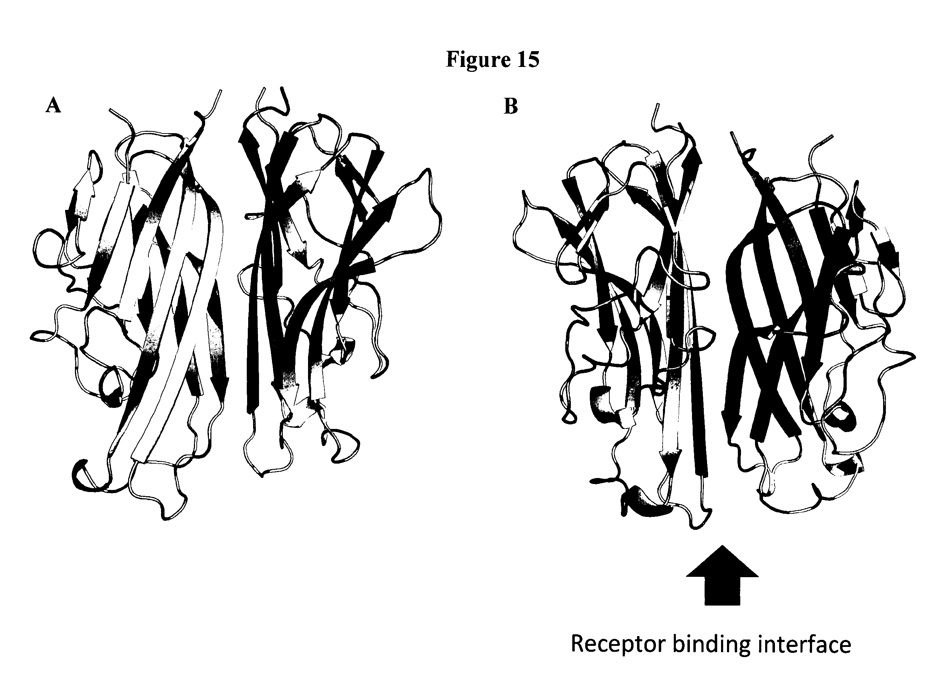

Figure 15A shows the non-conserved residue positions (red) between the human and murine RANKL sequences mapped on dimer.

Figure 15B shows the conserved trimer interface between the human and murine RANKL sequences.

Figure 16 shows conformation of stoichiometry by Native Gel for Dimer 1 and 2. Making use of a NativePAGE™ Novex® 4-16% Bis-Tris Gel samples were loaded and the size was checked. Visible is one band at the expected size and two bands higher, the sizes do not correspond to the size of a trimer or monomer.

Figure 17 shows conformation of stoichiometry by DLS for Dimer 1 & 2. A) % Intensity plotted versus the radius measured for dimer 1. The first peak has a polydispersity of 35% for a measured radius of 2.8 nm corresponding to a 38 kDa protein, the second and third peak correspond to a radius which is to big to be a protein. B) % Intensity plotted versus the radius measured for dimer 2. The first peak has a polydispersity of <0.1% for a measured radius of 3.0 nm corresponding to a 46 kDa protein, the second and third peak correspond to a radius which is to big to be a protein.

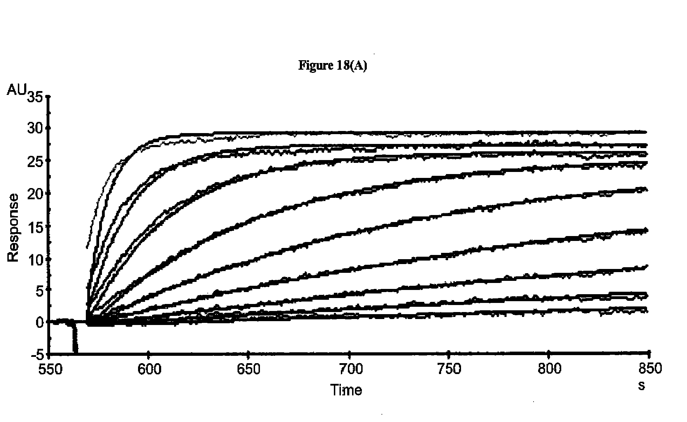

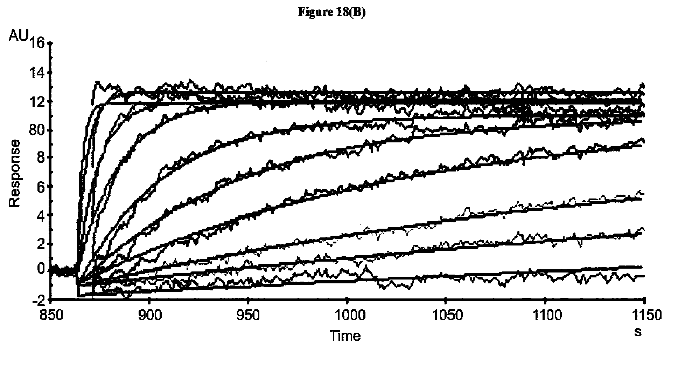

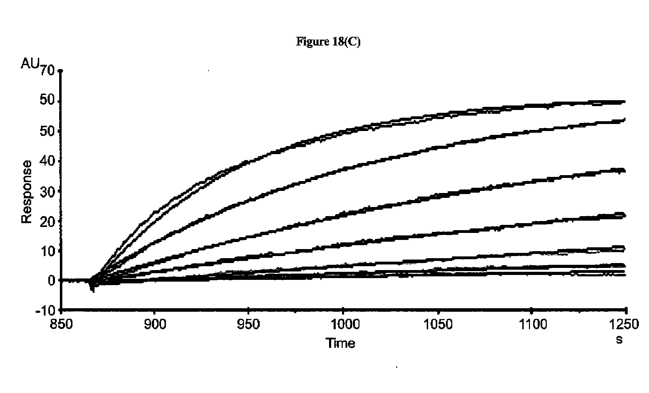

Figure 18 shows representative curves of receptor binding of wtRANK-L trimer and RANK-L Dimer 1 and Dimer 2 as determined with SPR. Concentration dependent association was reached for both dimers and for wildtype. The maximum response units are as expected for the interaction between dimeric RANK-L and one monomeric unit of RANK-FC, as can be calculated with the formula {[MWbound/MWcaptured] x RU RANK-FCcaptured} which gives the expected maximum RU value. In the experiment with RANK-L Dimer 1 57 RU of RANK-FC was captured, the expected maximum RU value would be 34. For the experiment with RANK-L Dimer 2 25 RU RANK-FC was captured, the expected RU value would be 15. It is visible that both values are almost reached. For the trimeric RANK-L 37RU FC was captured, the maximum RU value expected here would be (105/60*37=) 65 RU. This value is the calculated maximum reached in the experiment shown.

Figure 19 shows binding of D1 and D2 to OPG as shown by SPR. Directly immobilized OPG on a CM5 chip, concentration range RANKL (4 nM - 31.25 pM). Concentration dependent binding, almost no dissociation.

Figure 20 shows the results of an ELISA to measure the binding of WT/D1/D2 to A) RANK and B) OPG.

Figure 21 shows dimer 1 treated cells (concentration 100 ng/mL), showing the formation of bigger osteoclasts.

Figure 22 shows the association of wild-type TNF ligands with their congnate receptor, when ligands bind either A) in the cleft formed between two receptors, or B) to the surface exposed surface of the receptor, wherein the large blue ovals represent receptor monomers and the small red ovals represent ligand monomers.

Figure 23 shows an alignment of the extracellular ligand binding domain of various TNF ligands against murine RANKL. Structurally equivalent positions of mRANKL mutations are shown in bold and underlined. All sequences shown are based on the X-ray structures, except human APRIL which is based on a homology model with murine April as template.

Figure 24 shows a structural alignment of the extracellular ligand binding domain of various TNF ligands against murine RANKL. All sequences shown are based on the X-ray structures, except human APRIL which is based on a homology model with murine April as template.

Various aspects and embodiments of the present invention will now be described in more detail by way of example. It will be appreciated that modification of detail may be made without departing from the scope of the invention.

### Examples

### *Example 1- In silico design of RANKL dimers*

RANKL, like most other TNF ligand family members, binds the RANK receptor in the cleft between two adjacent monomers and RANKL induced trimerization of the RANK receptor results in activation of downstream signalling. We reasoned that by engineering a dimeric RANKL variant having only one intact receptor binding interface a competitive antagonist could be created that binds RANK receptor but is unable to trimerize and activate it.

Several crystal structures are available of the extracellular domain of murine RANKL are available (20) but none of human RANKL. The two highest resolution structures of murine RANKL (PDB codes: 1IQA and 1S55; 2.20 and 1.90 Å respectively) were used for the design (20). Both structures contain the biological active RANKL homotrimer in the asymmetric unit comprising of three RANKL monomer chains (A, B and C). A dimeric structure was constructed by deleting monomer C.

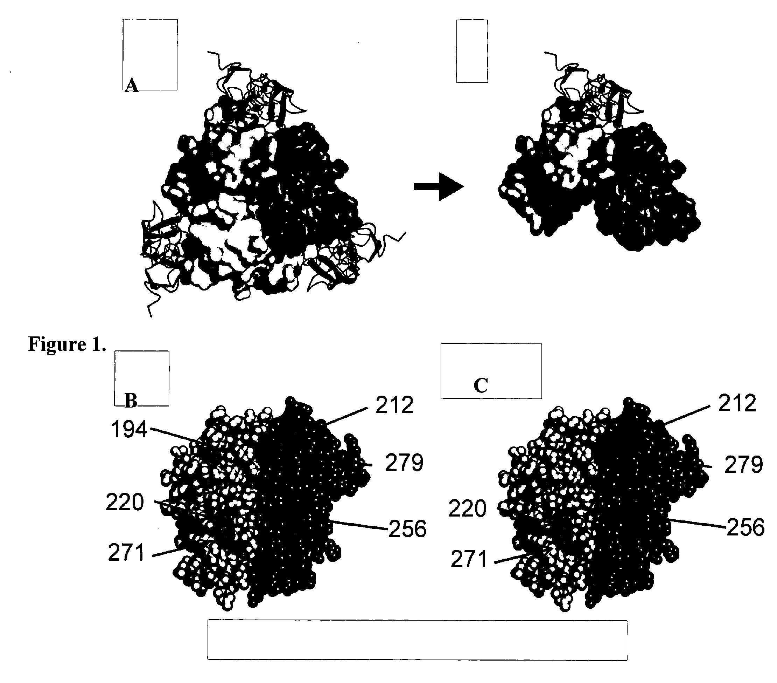

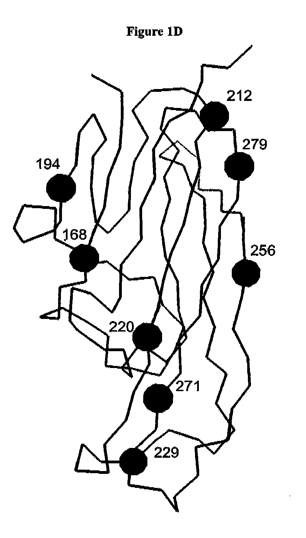

Next, an *in silico* amino acid mutagenesis scan using the FoldX protein design algorithm was performed on amino acid residues comprising the newly created solvent exposed interface of the dimer (Figure 1) (25). FoldX *in silico* mutagenesis and energy calculation were performed as described before. (25, 24, 15) Amino acids substitutions that had a neutral or positive effect on the stability of the dimer but a negative effect on the stability of the trimeric structure were retained. Favourable substitutions were combined and when possible the most hydrophilic amino acid substitution at newly solvent exposed positions was favoured. Remaining hydrophobic patches were modified by introducing energetically favourable hydrophilic substitutions.

Two different designs: D1 (Dimer 1) and D2 (Dimer 2), each comprising of a monomer A and B, were proposed for experimental characterization. The mutations made in these two dimers are shown in Table 2, below.

Table 2A - Mutations introduced into RANKL dimer 1

| Position | Monomer A | Monomer B |
|----------|-----------|-----------|
| K194 | D | |
| F212 | | Y |
| C220 | S | |
| K256 | | D |
| F271 | Y | |
| F279 | | Y |

Table 2B - Mutations introduced into RANKL dimer 2

| Position | Monomer A | Monomer B |
|----------|-----------|-----------|
| T168 | V | V |
| F212 | | Y |
| C220 | | A |
| S229 | K | K |
| K256 | | D |
| F271 | Y | |
| F279 | | Y |

Due to the three fold symmetry axis of the RANKL trimer, construction of a heterodimer consisting of a monomer A and B, was required. For example, residue 271 in monomer A is in the interface that previously interacted with the third monomer and substituting wild-type Phe271 for a tyrosine residue reduces the affinity for the third monomer. On the other hand, in monomer B this residue is part of the interface that interacts with monomer A and here the F271 Y substitution would severely affect the stability of the dimer. By designing a hetero dimer it was possible to change positions in the interface with the third monomer to disfavor binding while the equivalent position in the other monomer that is part of the dimer interface could remain unchanged.

Dimer 1 was predicted to destabilize trimer formation by ~9 kcal/mol, while trimer destabilization by dimer 2 was predicted to be ~7 kcal/mol. FoldX calculation also showed that dimer 2 was 1.5 kcal/mol more stable than dimer 1.

*Example 2 - Site directed mutagenesis, Expression and Purification of the dimers*

Mutations were introduced using several round of site directed mutagenesis in a cDNA encoding soluble murine RANKL (aa 160-316). (18) The mutated PCR product corresponding to monomer A was cloned into the first multiple cloning site (MCS) of a pCDF-Duet-1 bicistronic co-expression vector (Merck) using *Bam*HI and *Hind*III. Monomer B was cloned into the second MCS using *Nde*I and *Xho*I. Monomer A is fused to a hexahistidine-tag to aid purification.

A recombinant negative *E.coli* BL21 derivate, *E.coli* BL21 BLR, was transformed with the expression vector encoding Dimer 1 and Dimer 2. The *E.coli* BL21 BLR cells were grown in 2xLB medium at 37°C and 250 rpm until OD 0.5 was reached. Streptomycin (100 ug/mL) (Duchefa) or spectomycin (100 ug/mL) (Duchefa) was used as selective agent. After reaching OD 0.5 the cultures were induced with 1 mM IPTG (Duchefa) and the temperature was lowered to 20°C. The cells were incubated overnight and subsequentlyharvested by centrifugation at 10,000g for 15 minutes. The pellet was washed with PBS and resuspended in 20 mM sodium phosphate (Merck) buffer, containing 10% glycerol (Duchefa), 150 mM NaCl (Merck), and 7 mM $\beta$-mercaptoethanol (Sigma) , pH 7.5 (buffer A).

Cells were disrupted by sonicating the resuspended pellet for three minutes. The extract was clarified by centrifugation at 42,000g for one hour.

The supernatant was loaded on a Ni Sepharose HP Column (HisTRAP HP GE Healthcare). The column was washed with buffer A and the his-tag containing dimer was eluted with a gradient 0-100% of elution buffer (buffer A containing 1 M imidazol (Sigma) pH 7.5). Fractions containing his-tag protein were combined, concentrated and loaded on a gelfiltration column (Superdex 75 16/60 GE Healthcare). Buffer A was used as elution buffer. Purified proteins were >90% pure as determined using a collodial Coomassie Brilliant Blue-stained SDS-PAGE gel. Purified protein solutions were flash frozen in liquid nitrogen and stored at -80°C.

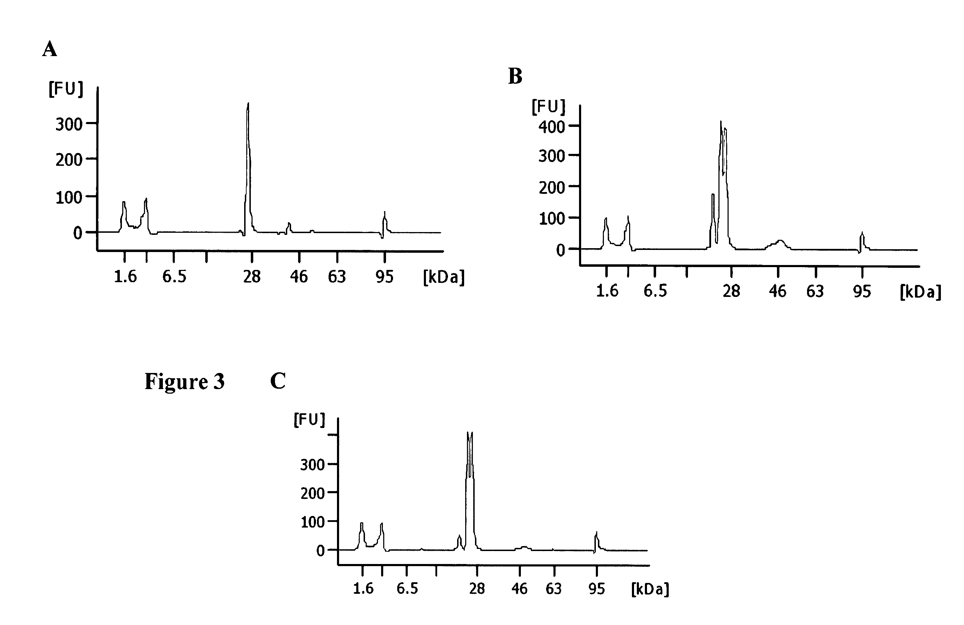

Mass spectroscopy (MALDI and MS-MS peptide sequencing) confirmed that both monomers of the heterodimer were present in the purified samples. Analysis by capillary electrophoresis also showed (Figure 3) that monomer A and monomer B were present in the same quantities.

*Example 3 - Biophysical characterization and conformation of stoichiometry*

Circular Dichroism and thermal decay experiments were performed as described before (26). Samples were dialyzed against 20 mM Sodium phosphate buffer containing 250 mM NaCl and 10% glycerol pH 7.3 and were measured at 0.2 mg/mL.

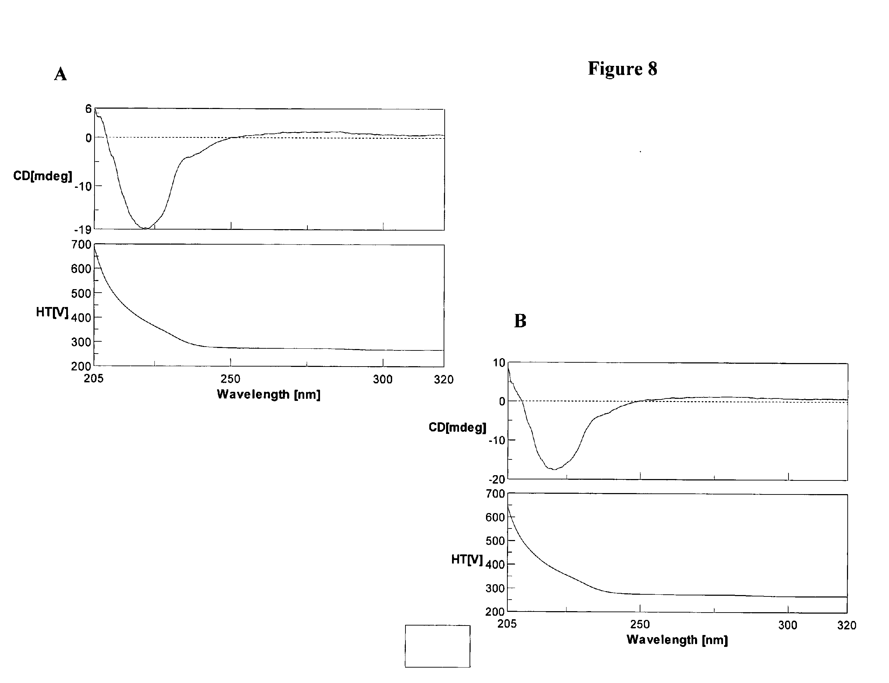

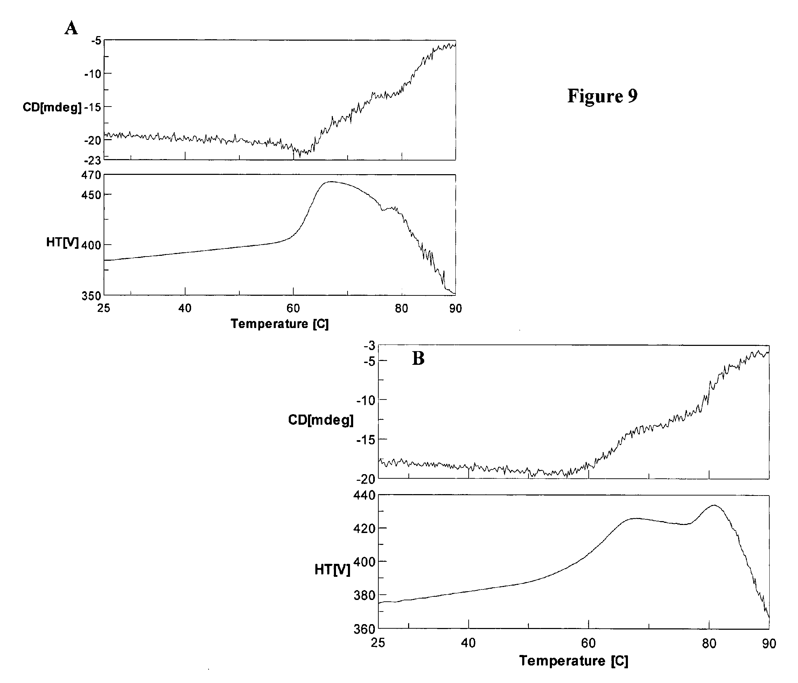

Circular dichroism measurements indicated that both dimers are properly folded and have secondary structures consistent with an all $\beta$-sheet protein (Figure 8). Heat induced unfolding showed that both variants were structurally stable up to 60 °C. No significant difference in stability between the variants could be determined (Figure 9).

Analytical gelfiltration was performed with 100 $\mu$L sample with a concentration of 0.6 mg/mL. Samples were run either on a Superdex 16/60 200 (GE Healthcare) or on a Superdex 16/60 75 (GE Healthcare). Both columns were calibrated with protein standards of known size. Buffer A was used as elution buffer with a flow rate of 0.5 or 1 mL/min. The retention time was related to the retention times of the calibration standards, as shown in Table 3, below.

Table 3 - Elution volumes from gel filtration assay

|  | Ve(ml) | Kav | ln(x) | MW(calc) |
|---|---|---|---|---|
| dimers |  |  |  |  |
| dRANKL D2+flag | 10.91 | 0.19 | 10.64 | 41821.92 |
| dRANKLD2 | 10.98 | 0.19 | 10.61 | 40596.64 |
| dRANKLD2 | 10.96 | 0.19 | 10.62 | 40943.01 |
| Trimers |  |  |  |  |
| flagRANKL wt | 10.5 | 0.16 | 10.82 | 49778.63 |
| flagRANKL wt | 10.5 | 0.16 | 10.82 | 49778.63 |
| flagRANKL wt | 10.49 | 0.16 | 10.82 | 49990.53 |

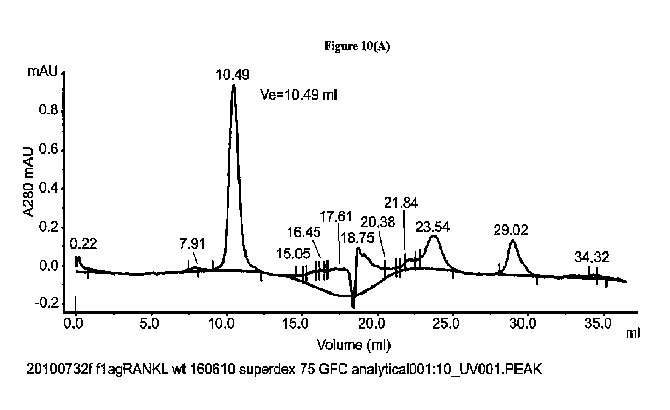

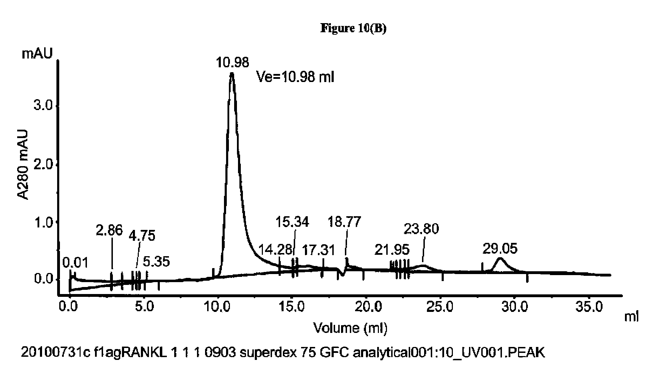

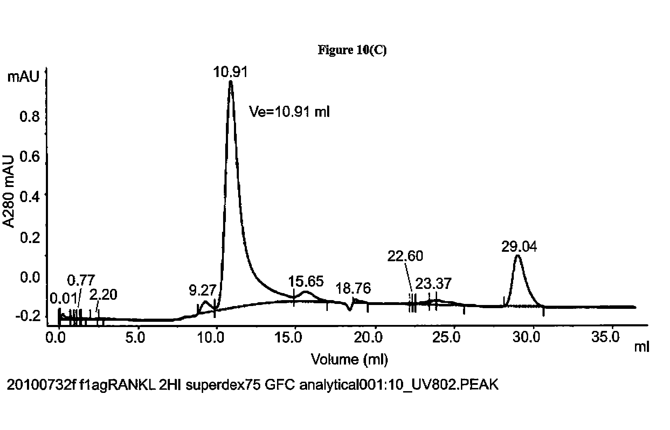

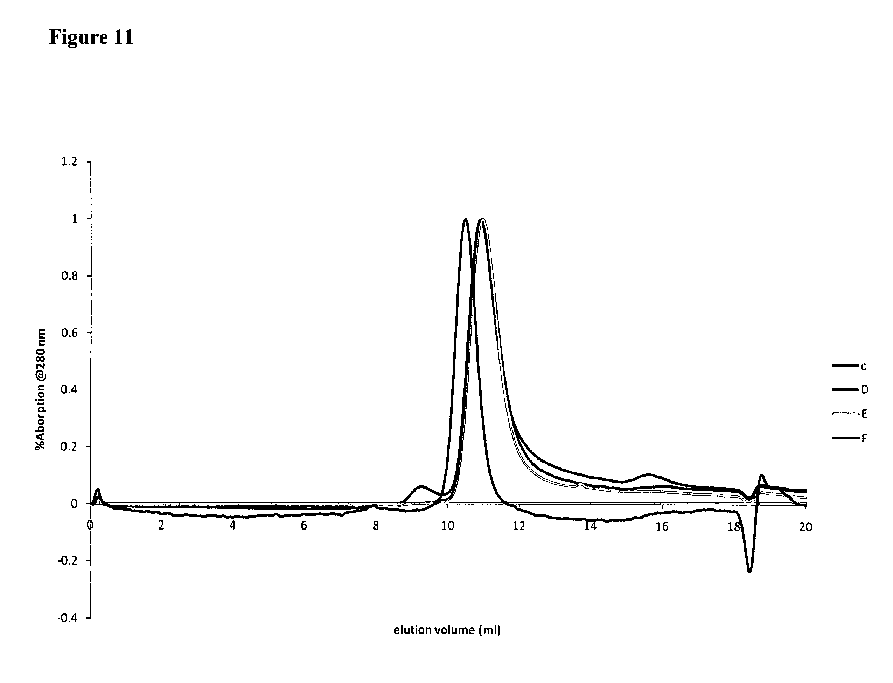

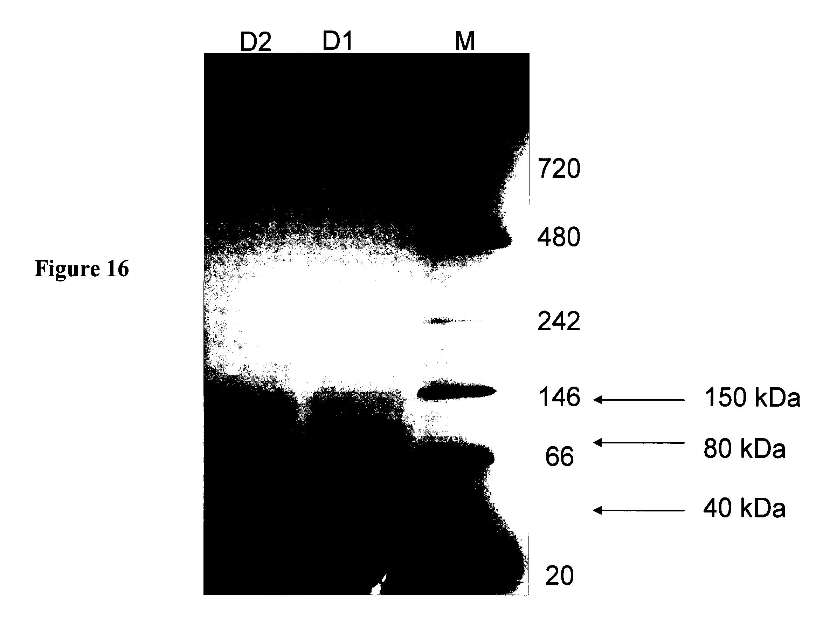

Analytical size exclusion chromatography showed that the dimer 1 and dimer 2 are not in the trimeric form as the elution volume is increased relative to RANKL wt (Figures 10 and 11). To confirm this a blue native PAGE was performed, the native gel showed a band at 35-40 kDa; the calculated size for the dimeric variants, but also two bands above 66 kDa suggesting some aggregation. No bands observed where wild-type RANKL (54 kDa) would be expected or where monomers should run (18 kDa) (Figure 16).

Samples were also analyzed by Dynamic Light Scattering (DLS). Purified samples (0.5-2 mg/mL) were before measurements filtered over a 0.2 $\mu$m filter. 20 measurements per sample were recorded. Data analysis was performed using Dynapro software, the data was analyzed as being bimodal. Blue native gel electrophoresis (NativePage 12% Invitrogen) was performed according to manufacturers instructions.

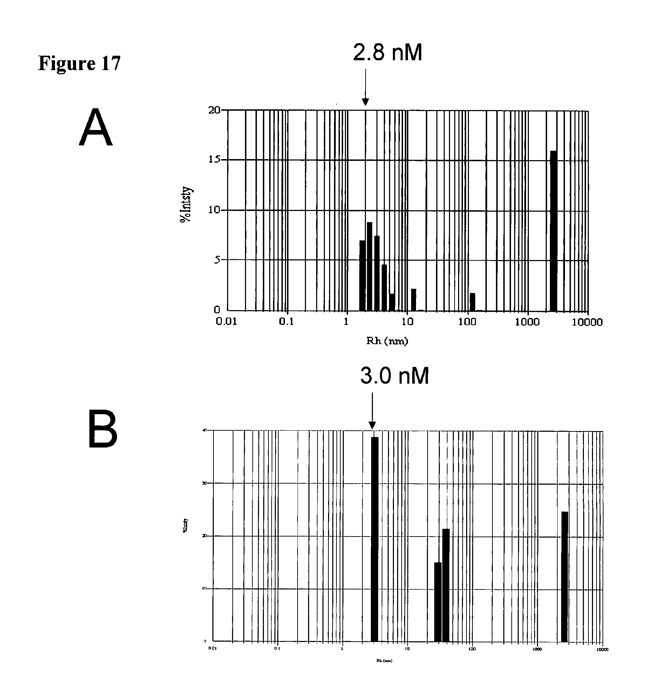

The calculated values from the DLS experiment are 38 kDa and 46 kDa for dimer 1 and dimer 2, respectively (Figure 17). While RANKL dimer 2 shows a minimal polydispersity (<0.1%), the polydispersity of dimer 1 was considerably larger at 35%. This is in line with the possible aggregation as observed in the BN-PAGE experiment described above. As for size exclusion chromatography, the mass calculated in the DLS experiments depends on the shape of the protein and this might explain why the values are slightly higher than would be expected (29).

A Surface Plasmon Resonance receptor binding assay was used as a shape independent method to determine the molecular weight of the RANKL dimers. In contrast to GFC and DLS which measure the oligomerization state of relatively concentrated samples (> 100 ug/ml), this assay has the added benefit that it measures the MW; and hence the oligomerization state, at physiologically relevant concentrations (< 1 ug/ml). The maximum response as recorded in a SPR assay depends on the MW and the amount (in response units [RU]) of the receptor coupled to the sensor chip and the MW of the analyte when observing a 1:1 binding (Equation 1).

$$RU_{Ligand(Maximum)} = \frac{MW_{Ligand}}{MW_{Re\,ceptor}} xRU_{\text{Re}\,ceptorCaptured}$$ .

Kinetic parameters were determined using a Surface Plasmon Resonance based assay on a Biacore200 system (GE Healthcare). A Cl chip (GE Healthcare) was coated with 1000 response units of protein A (Sigma) using standard amine coupling chemistry as described before (15,27,28).

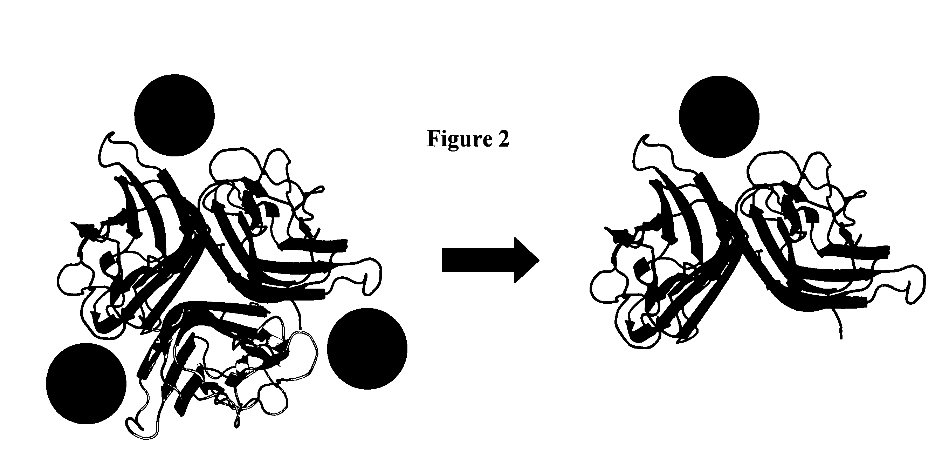

The calculated $R_{max}$ for dimeric RANKL variant D1 at a receptor capture level of **57** RU is **34** RU and for dimeric variant D2 at a capture level of 25 RI is **15** RU. The measured levels are 30 RU and 14 RU, respectively. The measured $R_{max}$ for RANKL wild-type is (**65** RU) at a capture level of 37 RU. Thus the measured values of the dimmers and wt closely agree with the calculated theoretical values. Hence, the oligomerization state of the RANKL variants is dimeric (Figure 2b). $R_{max}$ for RANKL wild-type.

### Example 4 - Determination of RANK-Fc and OPG binding

To demonstrate that RANKL dimers were still able to bind RANK or OPG with high affinity an ELISA experiment was performed.

Wells were coated with RANK-FC or OPG-FC fragments and a concentration range (100 pM to 562 nM) of the RANKL dimers was added. Both dimer 1 and dimer 2 still bound to RANK-FC with similar high affinity, as shown in Table 4, below.

Table 4 - Binding kinetics of D1, D2 and trimeric wtRANKL to RANK-Fc as shown by SPR

|  | ka (1/Ms) | kd (1/s) | KA(1/M) | KD(pM) |
|---|---|---|---|---|
| D1 | 4.52 (+/- 1) E6 | 1.97 (+/-0.5) E-4 | 2.33 (+/- 0.7) E10 | 44.6 (+/- 7) |
| D2 | 8.55 (+/- 1.5) E6 | 5.54 (+/- 2.5) E-4 | 1.80 (+/- 0.8) E10 | 61.9 (+/- 25) |
| WT | 8.27 (+/-1.8) E5 | 8.97 (+/-1.74) E-5 | 9,23 (+/- 0,2) E9 | 110 (+/- 4) |

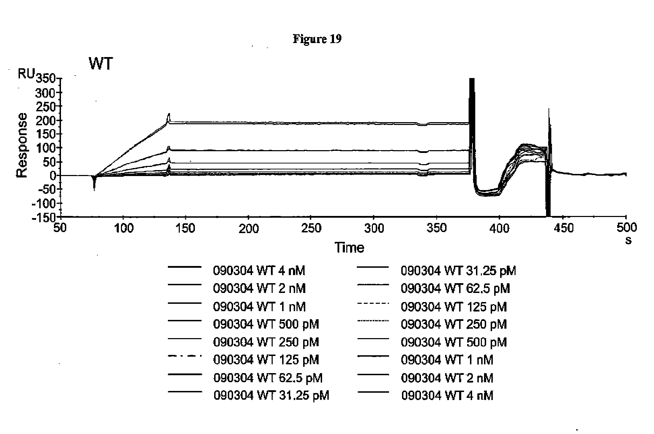

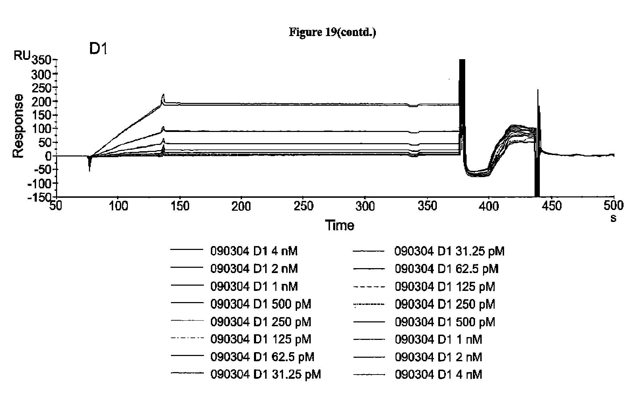

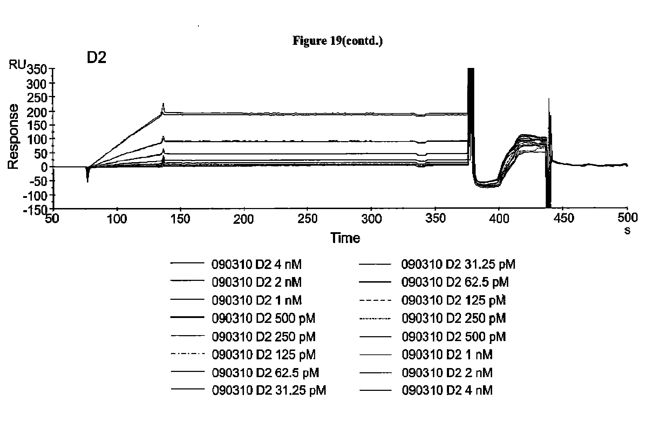

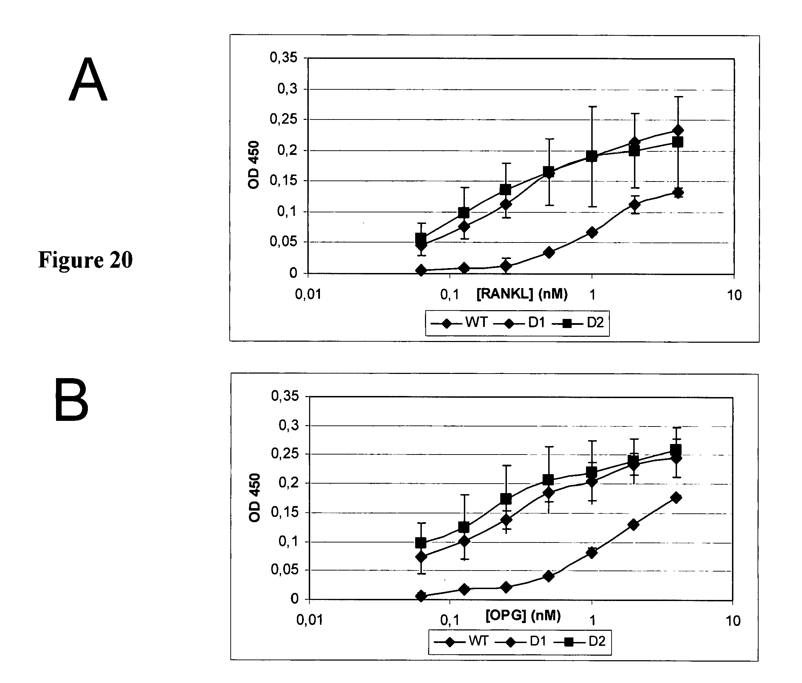

Binding was show to be concentration concentration dependent, using biacore and ELISA studies (Figures 19 and 20A). Similar results were observed for OPG binding; both dimer 1 and dimer 2 bind in a concentration dependent fashion with similar affinities to OPG (Figure 20B).

RANK-FC (Sigma) was captured in flow cell 2 at a level of 40 RU by a 60 s injection pulse of a 0.7 ug/ml RANK-FC solution at a flow rate of 50 uL/min. Flow cell 1 served as reference cell. Subsequently, serial dilutions ranging from 562 nM to 100 pM of RANKL wt (R&D systems), or the purified RANKL dimers were injected over both flow cells at 50 uL/min and 37°C. Binding was measured in real time. After each cycle the protein A surface was regenerated with a 30 s pulse of 10 mM Glycine pH 1.5. KD values were calculated by fitting the data to a langmuir 1:1 binding model using the BIAevaluation software as described before (30).

OPG-Fc (R&D systems) was immobilized directly on the surface of a CM5 chip (GE Healthcare) using a standard amine coupling procedure according to manufacturer's instructions. RANKL dimers or RANKL wt were injected at 4 nM at a flow of 70 uL/min and binding was measured in real time. The temperature was set at 25°C during the measurements. Between cycles the surface was regenerated with a 30s injection of 10 mM Glycine, pH 1.5. Association and dissociation was visualized with BIAevaluation software from Biacore.

Kinetic binding parameters were measured in real time using SPR. RANKL dimer binding curves were recorded using concentrations ranging from 100 pM to 562 nM dimer 1 or dimer 2, trimeric wild-type RANKL was used as a control. Measurements were performed at 37°C. The dissociation constants ($K_D$) were determined by fitting the data to a Langmuir 1:1 interaction model. The $K_D$ values for wild-type, dimer 1 and dimer 2 were 110 (+/-40) pM, 45 (+/- 7) pM and 62 (+/- 25) pM, respectively. The association rate constants ($k_{on}$) were ~5 fold (dimer 1) and ~10 fold (dimer 2) faster than the association rate constant of wild-type RANKL, as shown in Table 4, above.

The binding off-rate constant ($k_{off}$) of wild-type RANKL was ~2 to 6 fold slower than for dimer 1 and dimer 2. SPR binding measurements to OPG at 25°C showed similar results as RANK binding (Figure 20B).

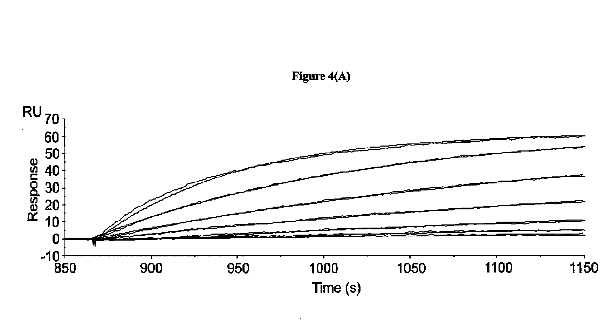

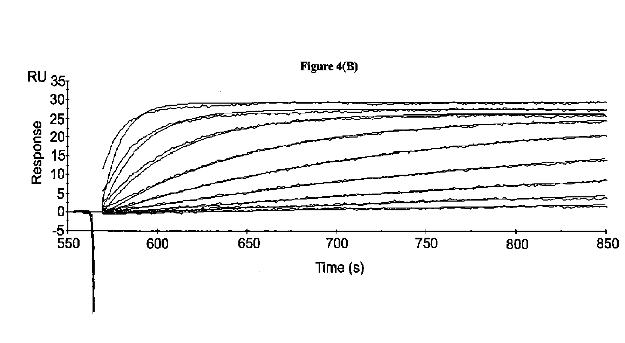

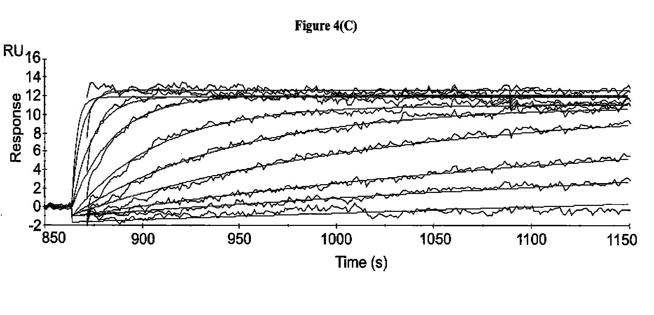

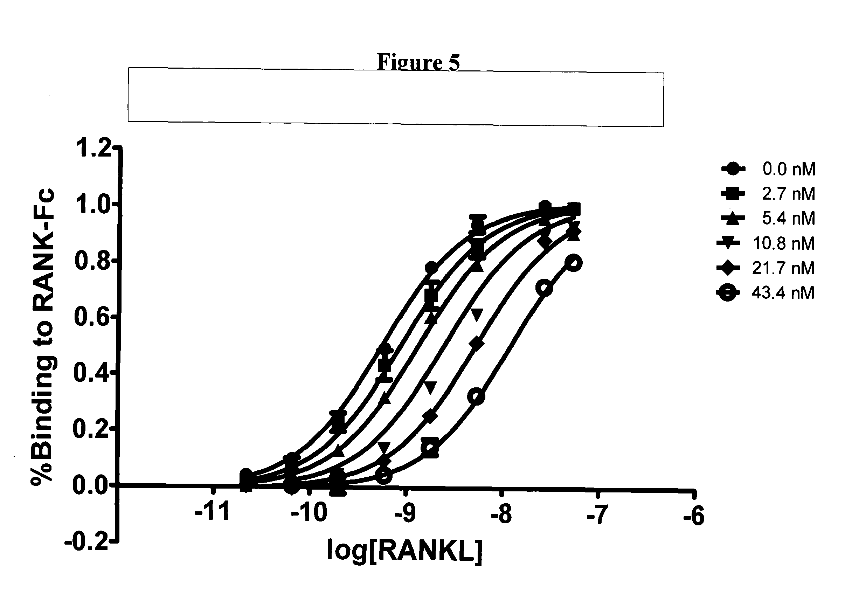

The ability of dimer 1 and dimer 2 to compete with wild-type RANKL for binding to RANK was investigated using a competitive ELISA. Serial dilutions of wild-type RANKL containing an N-terminal flagtag (flagRANKL) (0-53 nM) and a fixed concentration of dimer 1 or dimer 2 (0, 2.7, 5.4, 10.8, 21.7 or 43.4 nM) were added to RANK-Fc coated wells. After washing off unbound RANKL, RANK bound flagRANKL was detected by an anti-flag antibody. A RANKL dimer concentration dependant decrease in RANK binding and right shift in the $EC_{50}$ of flagRANKL was observed (Figures 4 and 5), indicating a competitive inhibition based mechanism. $IC_{50}$ values for dimer 1 and dimer 2 were calculated to be 4 nM by a global fit of the individual dose response curves using a four parameter logistic model. No significant differences were revealed between dimer 1 and dimer 2.

### Example 5 - Inhibition of osteoclastogenesis

To test the antagonistic properties of the RANKL dimers, the ability of RANKL dimers to block wild-type RANKL induced multinucleation of osteoclast precursor RAW 264.7 cells was determined.

Murine RAW 264.7 cells were cultured as described (19). The cells were sub-cultured every third or fourth day by a 1:10 dilution in fresh medium. Cells were seeded at a density of 1,000 cells/well in 100 $\mu$L in a 96 well plate. The wells bordering the edge of the plate were filled with 100 $\mu$L water to prevent evaporation of medium of wells containing cells. At day three the medium was changed for Alpha-Mem medium (Invitrogen) and RANKL dimers or wtRANKL trimer (Antigenix America) were added at 50, 100 and 150 ng/mL. Inhibition of wtRANKL by dimer 1 or dimer 2 was measured by adding 100 ng/mL wtRANKL and 100 ng/mL of the dimeric variant. At day 5 the medium was replaced and wild-type or variant RANKL was added at the same concentrations.

At day 7 osteoclastogenesis was determined using the tartrate resistance acid phosphatase (TRAP) assay (Sigma) according to manufacturer's instructions. The number of osteoclasts in each well was counted. The number of osteoclasts in the reference wells was set to one and the number of osteoclasts in the treated wells was calculated relative to the reference well. This allowed comparison between independent assays having different amount of cells at the start of the assay.

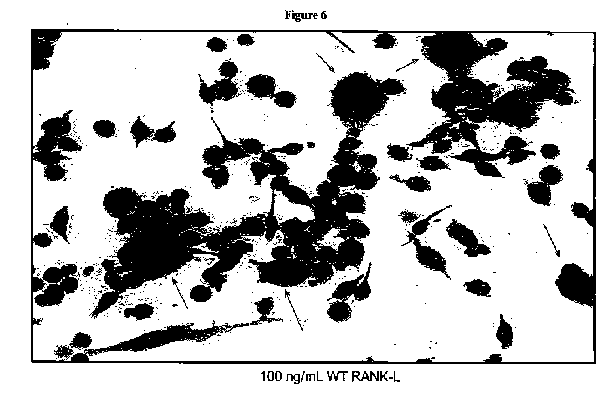

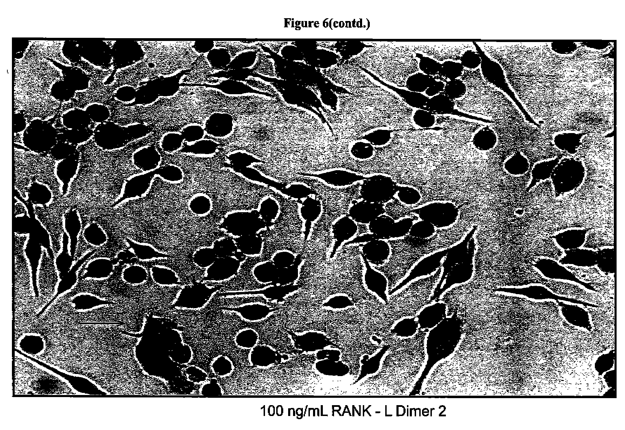

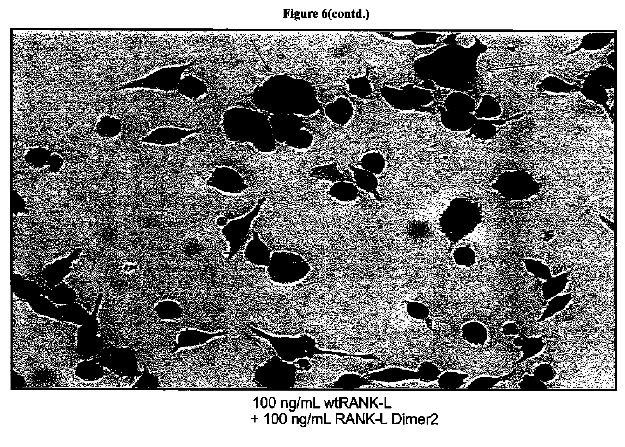

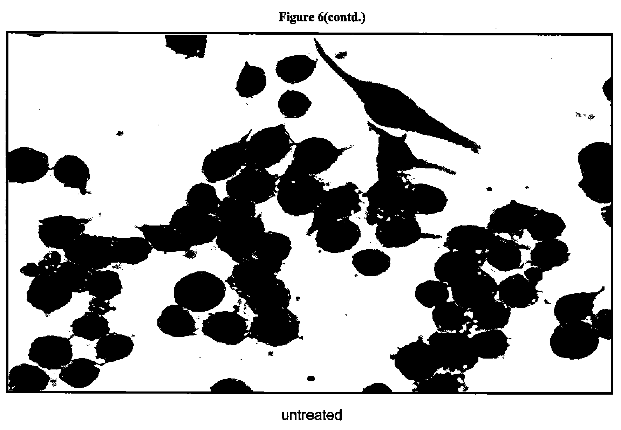

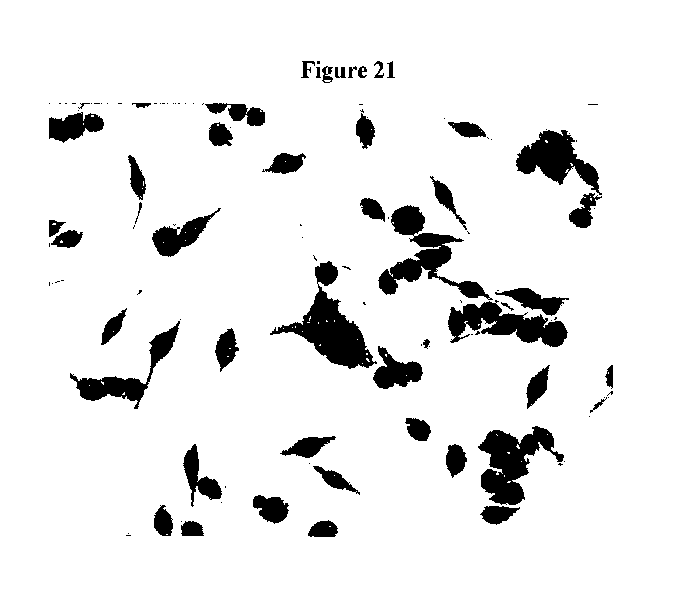

Figure 6 shows the cells after staining with the multinucleated cells being marked. As can be seen, most multinucleated cells are present in the wild-type RANKL treated sample. In contrast, the dimer treated samples show considerably less multinucleated cells and they are also less clustered than wild-type RANKL treated cells. RANKL dimer 1 (Figure 21) appears to be less potent blocker of osteoclastogenesis than dimer 2, the dimer 1 treated cells contain more nuclei and the osteoclasts are bigger than the ones observed in cells treated with RANKL dimer 2.

Upon treatment with RANKL dimer 1 and dimer 2 (100 ng/mL) the number of multinucleated cells remains similar to the untreated control samples. Importantly, treatment with RANKL dimers at a concentration of 100 ng/mL does not lead to an increase in the formation of multinucleated cells (Figure 6B).

In conclusion, the RANKL dimers are potent inhibitors of wtRANKL induced osteoclastogenesis.

### Numbered embodiments of the invention

1. A variant of a TNF family ligand which is mutated such that it is not capable of assembling into a trimer, wherein the variant ligand retains the ability to bind one or more of its cognate receptor(s), but wherein binding to the receptor does not activate the receptor.

2. The variant of a TNF family ligand of embodiment 1, wherein the variant does not homotrimerise with itself.

3. The variant of a TNF family ligand of embodiment 1 or embodiment 2, wherein the variant is capable of assembling into a dimer with another variant of the same ligand.

4. The variant of a TNF family ligand of embodiment 3, wherein the variant binds one or more of its cognate receptors within the cleft formed between two assembled ligand monomers.

5. The variant of a TNF family ligand of embodiment 4, wherein the TNF family ligand is selected from the group consisting of RANKL, TRAIL, APRIL, BAFF, TNFalpha, CD30L, CD40L, FasL, Light, and Tweak.

6. The variant of a TNF family ligand of any one of the preceding embodiments, wherein the variant comprises a mutation at one or more of positions 168, 194, 212, 220, 229, 256, 271, and 279.

7. The variant of a TNF family ligand of embodiment 6, wherein the TNF variant comprises one or more of the mutations T168V, K194D, F212Y, C220S, C220A, D229K, S229K, K256D, F271Y and F279Y.

8. The variant of a TNF family ligand of embodiment 7, wherein the variant comprises the mutations K194D and C220S and F271Y.

9. The variant of a TNF family ligand of embodiment 7, wherein the variant comprises the mutations F212Y, K256D and F279Y.

10. The variant of a TNF family ligand of embodiment 7, wherein the variant comprises the mutations T168V, S229K and F271Y.

11. The variant of a TNF family ligand of embodiment 7, wherein the variant comprises the mutations T168V, F212Y, C220A, S229K, K256D and F279Y.

12. The variant of a TNF family ligand of embodiment 1 or 2, wherein the variant is not capable of assembling into a dimer with the same or other TNF family ligands.

13. The variant of a TNF family ligand of embodiment 12, wherein the variant binds its cognate receptor on the solvent exposed surface of the TNF ligand variant.

14. The variant of a TNF family ligand of embodiment 13, wherein the variant is selected from the group consisting of APRIL and BAFF.

15. The variant of a TNF family ligand of any one of the preceding embodiments, wherein the variant has an increased binding affinity for one or more of its cognate receptor(s), compared to the wild-type TNF family ligand.

16. The variant of a TNF family ligand of any one of the preceding embodiments, wherein the variant of a TNF family ligand has an increased binding affinity for one or more of its cognate target receptor(s), compared to the wild-type TNF family ligand.

17. The variant of a TNF family ligand of any one of the preceding embodiments, wherein the variant of a TNF family ligand has a decreased binding affinity for one or more of its cognate non-target receptor(s), compared to the wild-type TNF family ligand.

18. The variant of a TNF family ligand according to any one of the preceding embodiments, wherein the variant is soluble.

19. A dimer comprising two variants of a TNF family ligand of any one of embodiments 1-11.

20. The dimer of embodiment 19, wherein the dimer is a heterodimer.

21. A dimer comprising the variant of a TNF family ligand of embodiment 8 and the variant of a TNF family ligand of embodiment 9.

22. A dimer comprising the variant of a TNF family ligand of embodiment 10 and the variant of a TNF family ligand of embodiment 11.

23. A complex comprising one or more of the variant of a TNF family ligand of embodiments 1-18 and one or more cognate receptors for the TNF family ligand.

24. The complex of embodiment 23, wherein the complex comprises two variants of a TNF family ligand of embodiments 1-18.

25. A complex comprising a dimer of any one of embodiments 19-22 and one or more cognate receptors for the TNF family ligand.

26. The complex of any one of embodiments 23-25, wherein the complex comprises two cognate receptors for the TNF family ligand.

27. A nucleotide sequence encoding the variant of a TNF family ligand of any one of embodiments 1-18 or the dimer of any one of embodiments 19-22.

28. A vector comprising the nucleotide sequence of embodiment 27.

29. A host cell comprising the nucleotide sequence of embodiment 27 or the vector of embodiment 28.

30. A pharmaceutical composition comprising the variant of a TNF family ligand of any one of embodiments 1-18, the dimer of any one of embodiments 19-22, the complex of any one of embodiments 23-26, the nucleotide sequence of embodiment 27, the vector of embodiment 28 or the host cell of embodiment 29.

31. The variant of a TNF family ligand of any one of embodiments 1-18, the dimer of any one of embodiments 19-22, the nucleotide sequence of embodiment 27, the vector of embodiment 28, the host cell of embodiment 29 or the pharmaceutical composition of embodiment 30 for use in medicine.

32. The variant of a TNF family ligand of any one of embodiments 1-18, the dimer of any one of embodiments 19-22, the nucleotide sequence of embodiment 27, the vector of embodiment 28, the host cell of embodiment 29 or the pharmaceutical composition of embodiment 30 for use in the treatment of osteoporosis, rheumatoid arthritis, Paget's disease, malignancy induced bone disease or cancer.

33. Use of the variant of a TNF family ligand of any one of embodiments 1-18, the dimer of any one of embodiments 19-22, the nucleotide sequence of embodiment 27, the vector of embodiment 28, the host cell of embodiment 29 or the pharmaceutical composition of embodiment 30 in the manufacture of a medicament for the treatment of osteoporosis, rheumatoid arthritis, Paget's disease, malignancy induced bone disease or cancer.

34. A method of treating osteoporosis, rheumatoid arthritis, Paget's disease, malignancy induced bone disease or cancer comprising administering a pharmaceutically effective amount of the variant of a TNF family ligand of any one of embodiments 1-18, the dimer of any one of embodiments 19-22, the nucleotide sequence of embodiment 27, the vector of embodiment 28, the host cell of embodiment 29 or the pharmaceutical composition of embodiment 30 to a patient in need of treatment.

35. A transgenic animal which expresses the variant of a TNF family ligand of any one of embodiments 1-18, or the dimer of any one of embodiments 19-22.

36. A method for producing a variant of a TNF family ligand comprising the steps of:

a) identifying amino acids in the TNF family ligand that are located in the trimerisation interface as candidates for mutation;

b) substituting each of one or more residues in the trimerisation interface; and

c) selecting amino acid substitutions which have a neutral or positive effect on the stability of the dimer but a negative effect on the stability of the trimer.

37. The method of embodiment 36, further comprising one or more of the steps of:

d) selecting amino acid substitutions to increase the affinity for one or more of the target receptor(s);

e) selecting amino acid substitutions to increase selectivity for one or more of the target receptors, either by increasing affinity for one or more of the target receptor(s) or decreasing affinity for one or more of the decoy receptors.

f) producing a variant of a TNF family ligand; and

g) Modifying the variant to improve its properties such as to decrease its immunogenicity or improve its pharmacokinetics. Such modifications may include one or more of pegylation, acetylation, formylation, alkylation such as methylation, and glycosylation.

38. The method of embodiment 36 or 37, wherein the variant of a TNF family ligand is an inhibitory variant of a TNF family ligand.

**References**

1. Kanazawa, K., and Kudo, A. (2005) J Bon. Min. Res., 20, 2053-2060

2. Parfitt, A.M. (2002) Bone, 30, 5-7

3. Teitelbaum, S.L. (2002) Science, 289, 1504-1508

4. Joseph Melton III, L. (2003) J. Bon. Min. Res., 18, 1139-1141

5. Boyce, B.F., and Xing, L. (2008) Arch. Biochem. Biophys., 474, 139-146

6. Anderson, D.M., Maraskovsky, E., Billingsley, W.L., Dougall, W.C., Tometsko, M.E., Roux, E.R., Teepe, M.C., DuBose, R.F., Cosman, D., Galibert, L. (1997) Nature, 390, 175-179

7. Wong, B.R., Rho, J., Arron, J., Robinson, E., Orlinick, J., Chao, M., Kalachikov, S., Cayani, E., Bartlett III, F.S., Frankel, W.N., Lee, S.Y., Choi, Y., J.B.C. (1997), 272, 25190-25194

8. Lacey, D.L., Timms, E., Tan, H.L., Kelley, M.J., Dunstan, C.R., Burgess, T., Elliott, R., Colombero, A., Elliott, G., Scully, S., Hsu, H., Sullivan, J., Hawkins, N., Davy, E., Capparelli, C., Eli, A., Qian, Y.X., Kaufman, S., Sarosi, I., Shalhoub, V., Senaldi, G., Guo, J., Delaney. J., Boyle, W.J. (1998) Cell, 93, 165-167

9. Wrigth, H.L., McCarhy, H.S., Middleton, J., Marshall, M.J. (2009) Curr Rev Musculoskelet Med, 2, 54-64

10. Ji, J.-D., Park-Min, K.-H., Shen, Z., Fajardo, R.J., Goldring, S.R., McHugh, K.P., Ivashkiv, L.B. (2009) J. Imm., 183, 7223-7233

11. Roato, I., Dámelio, P., Gorassini, E., Grimaldi, A., Bonello, L., Fiori, C., Delsedime, L., Tizzani, A., De Libero, A., Isaia, G., Ferracini, R., (2008) Plos One, 3, e3627

12. Mikami, S., Katsube, K., Oya, M., Ishida, M., Kosaka, T., Mizuno, R., Mochizuki, S., Ikeda, T., Mukai, M., Okada, Y. (2009) J. Pathol., 218, 530-539

13. Coleman, R.E. (2006) Clin. Canc. Res., 20s, 6243s-6249s

14. Kostenuit, P.J., Nguyen, H.Q., McCabe, J., Warmington K.S., Kurahara, C., Sun, N., Chen, C., Li, L., Cattley, R.C., Van, G., Scully, S., Elliott, R., Grisanti, M., Morony, S., Tan, H.L, Asuncion, F., Li, X., Ominsky, M.S., Stolina, M., Dwyer, D., Dougall, W.C., Hawkins, N., Boyle, W.J., Simonet, W.S., Sullivan J.K. (2009) J. Bon. Min. Res., 24, 182-195

15. Sloot, A.M. van der, Tur, V., Szegezdi, E., Mullaly, M.M., Cool, R.H., Samali, A., Serrano, L., Quax, W.J. (2006) PNAS, 103, 8634-8639

16. Ito, S., Hata, T., (2004) Vitam. Horm., 67, 19-33

17. Bossen, C., Ingold, K., Tardivel, A., Bodmer, J.-L., Gaide, O., Hertig, S., Ambrose, C., Tschopp, J., Schneider, P. (2006) J.B.C., 281, 13964-13971

18. Sambrook, J., Fritsch, E.F., Maniatis T. (1989) Molecular Cloning: A Laboratory Manual, 2nd Ed., Cold Spring Harbor Laboratory, Cold Spring Harbor, NY

19. Duan, L., Vos, P. de, Fan, M., Ren, Y. (2008) Front. Biosci., 13, 7064-7071

20. Walter, T.S., Liu, C., Huang P., Zhang, S., Wedderbum, L.R., Gao B., Owens, R.J., Stuart, D.I., Tang, P., Ren, J., (2009) Acta Cryst., F65, 597-600

21. Lewit-Bentley, A., Fourme, R., Kahn, R., Prangé, T., Vachette, P., Tavernier, J., Hauquier, G., Niers, W., (1988) J. Mol Biol, 199, 389-392

22. Lam, J., Nelson, C.A., Ross, F.P., Teitelbaum, S.L., Fremont, D.H., (2001) JCI, 108, 971-979

24. Guerois, R., Nielsen, J.E., Serrano, L., (2002) J Mol Biol, 320, 369-387

25. Schymkowitz, J., Borg, J., Stricher, F., Nys, R., Rousseau, F., Serrano, L., (2005) Nucleic Acid Res., 33, W382-W388

26. Sloot, A.M. van der, Mullally, M.M., Fernandez-Ballester, G., Serrano, L., Quax, W.J., (2004) Prot.Eng.Des.Sel., 9, 673-680

27. Reis, C.R., Sloot A.M. van der, Szegezdi, E., Natoni, A., Tur, V., Cool, R.H., Samali, A., Serrano, L., Quax, W.J., (2009) Biochemistry, 10, 2180-2191

28. Tur, V., Sloot, A.M. van der, Reis, C.R., Szegzdi, E., Cool, R.H., Samali, A., Serrano, L., Quax, W.J., (2008) JBC, 29, 20560-20568

29. Nobbmann, U., Connah, M., Fish, B., Varley, P., Gee, C., Mulot, S., Chen, J., Zhou, L., Lu, Y., Sheng, F., Yi, J., Harding, S.E., (2007) Biotech.Gen.Eng.Rev., 24, 117-128

30. Reis, C.R., Assen, A.H.G. van, Quax, W.J., Cool, R.H., (2010) Mol Cell Prot, Sep 17 (epub ahead of print)

31. Ta et al. (2010) PNAS, 47, 20281-20286

32. Sato et al. (2010) Current Opinions in Biotechnology, 17, 638-642

33. Locksley et al. (2001) Cell, 104, 487-501

34. Bodmer et al. (2002) Trends Biochem. Sci., 27, 19-26

35. Rieux-Laucat et al. (2003) Current Opinion in Immunology, 15, 325

36. Mackay and Ambrose (2003) Cytokine and growth factor reviews, 14, 311

37. Mackay and Kalled (2002) Current opinion in Immunology, 14, 783-790

38. Remington's Pharmaceutical Sciences

39. Peppel K et al. (1991) J Exp Med, 174(6), 1483-9

40. US 5,447,851

41. Steed PM et al. (2003) Science, 301(5641), 1895-8

42. Bekker PJ et al. (2004) J Bone Miner Res, 19(7), 1059-66

43. McClung MR et al. (2006) N Engl J Med, 354(8), 821-31

44. Clinical development of anti-RANKL therapy. Arthritis Research & Therapy 2007, 9(Suppl 1):S7.

45. Schramek et al. (2010) Nature, 0(0), 1-7

46. Gonzalez-Suarez et al. (2010) Nature, 0(0), 1-7

47. US7,381,792

**Sequences**

SEQ ID NO: 1 (human RANKL)

ggccaaagccgggctccaagtcggcgccccacgtcgaggctccgccgcagcctccggagttggccgcagacaagaaggg

gagggagcgggagagggaggagagctccgaagcgagagggccgagcgccatgcgccgcgccagcagagactacacca

agtacctgcgtggctcggaggagatgggcggcggccccggagccccgcacgagggcccctgcacgccccgccgccgc

ctgcgccgcaccagcccccgccgcctcccgctccatgttcgtggccctcctggggctggggctgggccaggttgtctgcag

cgtcgccctgttcttctatttcagagcgcagatggatcctaatagaatatcagaagatggcactcactgcatttatagaattttgaga

ctccatgaaaatgcagattttcaagacacaactctggagagtcaagatacaaaattaatacctgattcatgtaggagaattaaaca

ggcctttcaaggagctgtgcaaaaggaattacaacatatcgttggatcacagcacatcagagcagagaaagcgatggtggatg

gctcatggttagatctggccaagaggagcaagcttgaagctcagccttttgctcatctcactattaatgccaccgacatcccatct

ggttcccataaagtgagtctgtcctcttggtaccatgatcggggttgggccaagatctccaacatgacttttagcaatggaaaact

aatagttaatcaggatggctttattacctgtatgccaacatttgctttcgacatcatgaaacttcaggagacctagctacagagtat

cttcaactaatggtgtacgtcactaaaaccagcatcaaaatcccaagttctcataccctgatgaaaggaggaagcaccaagtatt

ggtcagggaattctgaattccattttattccataaacgttggtggattttttaagttacggtctggagaggaaatcagcatcgaggt

ctccaacccctccttactggatccggatcaggatgcaacatactttggggcttttaaagttcgagatatagattgagccccagttttt

ggagtgttatgtatttcctggatgtttggaaacatttttttaaaacaagccaagaaagatgtatataggtgtgtgagactactaagagg

catggccccaacggtacacgactcagtatccatgctcttgaccttgtagagaacacgcgtatttacctgccagtgggagatgtta

gactcatggtgtgttacacaatggttttttaaattttgtaatgaattcctagaattaaaccagattggagcaattacgggttgaccttatg

agaaactgcatgtgggctatgggagggggttggtccctggtcatgtgcccccttcgcagctgaagtggagagggtgtcatctagc

gcaattgaaggatcatctgaaggggcaaattcttttgaattgttacatcatgctggaacctgcaaaaaatactttttctaatgaggag

agaaaatatatgtattttatataatatctaaagttatatttcagatgtaatgttttctttgcaaagtattgtaaattatatttgtgctatagta

tttgattcaaaatatttaaaaatgtcttgctgttgacatatttaatgttttaaatgtacagacatatttaactggtgcactttgtaaattccc

tggggaaaacttgcagctaaggaggggaaaaaaatgttgtttcctaatatcaaatgcagtatatttcttcgttcttttttaagttaatag

atttttttcagacttgtcaagcctgtgcaaaaaaattaaaatggatgccttgaataataagcaggatgttggccaccaggtgcctttc

aaatttagaaactaattgactttagaaagctgacattgccaaaaaggatacataatgggccactgaaatttgtcaagagtagttata

taattgttgaacaggtgttttccacaagtgccgcaaattgtacctttttttttttttcaaaatagaaaagttattagtggtttatcagcaa

aaaagtccaattttaatttagtaaatgttattttatactgtacaataaaaacattgcctttgaatgttaattttttggtacaaaaataaattt

atatgaaaaaaaaaaaaaaaaaaaaaaaaaaaaaaaa

SEQ ID NO: 2 (human RANKL)

MRRASRDYTKYLRGSEEMGGGPGAPHEGPLHAPPPPAPHQPPAASRSMFVALL

GLGLGQVVCSVALFFYFRAQMDPNRISEDGTHCIYRILRLHENADFQDTTLESQ

DTKLIPDSCRRIKQAFQGAVQKELQHIVGSQHIRAEKAMVDGSWLDLAKRSKLE

AQPFAHLTINATDIPSGSHKVSLSSWYHDRGWAKISNMTFSNGKLIVNQDGFYY

LYANICFRHHETSGDLATEYLQLMVYVTKTSIKIPSSHTLMKGGSTKYWSGNSE
FHFYSINVGGFFKLRSGEEISIEVSNPSLLDPDQDATYFGAFKVRDID

SEQ ID NO: 3 (mouse RANKL)

CCCACGTCCCGGGGAGCCACTGCCAGGACCTTTGTGAACCGGTCGGGGCGG
GGGCCGTGGCGGAGTCTGCTCGGCGGTGGGTGGCCCGAGAAGGGAGAGAAC
GATCGCGGAGCAGGGCGCCCGAACTCCGGGCGCCGCGCCATGCGCCGGGCC
AGCCGAGACTACGGCAAGTACCTGCGCAGCTCGGAAGAGATGGGCAGCGGC
CCCGGCGTCCCACACGAAGGTCCGCTGCACCCCGCGCCTTCTGCACCGGCTC
CGGCGCCGCCACCCGCCGCCTCCCGCTCCATGTTCCTGGCCCTCCTGGGGCT
GGGACTGGGCCAGGTGGTCTGCAGCATCGCTCTGTTCCTGTACTTTCGAGCG
CAGATGGATCCTAACAGAATATCAGAAGACAGCACTCACTGCTTTATAGAA
TCCTGAGACTCCATGAAAACGCAGGTTTGCAGGACTCGACTCTGGAGAGTGA
AGACACTACCTGACTCCTGCAGGAGGATGAAACAAGCCTTTCAGGGGGC
CGTGCAGAAGGAACTGCAACACATTGTGGGGCCACAGCGCTTCTCAGGAGC
TCCAGCTATGATGGAAGGCTCATGGTTGGATGTGGCCCAGCGAGGCAAGCCT
GAGGCCCAGCCATTTGCACACCTCACCATCAATGCTGCCAGCATCCCATCGG
GTTCCCATAAAGTCACTCTGTCCTCTTGGTACCACGATCGAGGCTGGGCCAA
GATCTCTAACATGACGTTAAGCAACGGAAAACTAAGGGTTAACCAAGATGG
CTTCTATTACCTGTACGCCAACATTTGCTTTCGGCATCATGAAACATCGGGA
AGCGTACCTACAGACTATCTTCAGCTGATGGTGTATGTCGTTAAAACCAGCA
TCAAAATCCCAAGTTCTCATAACCTGATGAAAGGAGGGAGCACGAAAAACT
GGTCGGGCAATTCTGAATTCCACTTTTATTCCATAAATGTTGGGGGATTTTTC
AAGCTCCGAGCTGGTGAAGAAATTAGCATTCAGGTGTCCAACCCTTCCCTGC
TGGATCCGGATCAAGATGCGACGTACTTTGGGGCTTTCAAAGTTCAGGACAT
AGACTGAGACTCATTTCGTGGAACATTAGCATGGATGTCCTAGATGTTTGGA
AACTTCTTAAAAAATGGATGATGTCTATACATGTGTAAGACTACTAAGAGAC
ATGGCCCACGGTGTATGAAACTCACAGCCCTCTCTCTTGAGCCCTGTACAGG
TTGTGTATATGTAAAGTCCATAGGTGATGTTAGATTCATGGTGATTACACAA
CGGTTTTACAATTTTGTAATGATTTCCTAGAATTGAACCAGATTGGGAGAGG
TATTCCGATGCTTATGAAAAACTTACACGTGAGCTATGGAAGGGGGTCACAG
TCTCTGGTCTAACCCCTGGACATGTGCCACTGAGAACCTTGAAATTAAGAGG
ATGCCATGTCATTGCATAGAAATGATAGTGTGAAGGGTTAAGTTCTTTTGAA
TTGTTACATTGCGCTGGGACCTGCAAATAAGTTCTTTTTTTCTAATGAGGAGA

AAAATATATGTATTTTTATATAATGTCTAAAGTTATATTTCAGGTGTAATGTT
TTCTGTGCAAAGTTTTGTAAATTATATTTGTGCTATAGTATTTGATTCAAAAT
ATTTAAAAATGTCTCACTGTTGACATATTTAATGTTTTAAATGTACAGATGTA
TTTAACTGGTGCACTTTGTAATTCCCCTGAAGGTACTCGTAGCTAAGGGGGC
AGAATACTGTTTCTGGTGACCACATGTAGTTTATTTCTTTATTCTTTTTAACTT
AATAGAGTCTTCAGACTTGTCAAAACTATGCAAGCAAAATAAATAAATAAA
AATAAAATGAATACCTTGAATAATAAGTAGGATGTTGGTCACCAGGTGCCTT
TCAAATTTAGAAGCTAATTGACTTTAGGAGCTGACATAGCCAAAAAGGAAC
ATAATAGGCTACTGAAATCTGTCAGGAGTATTTATGCAATTATTGAACAGGT
GTCTTTTTTACAAGAGCTACAAATTGTAAATTTTGGTTTCTTTTTTTTCCCAT
AGAAAATGTACTATAGTTTATCAGCCAAAAAACAATCCACTTTTTAATTTAG
TGAAAGTTATTTTATTATACTGTACAATAAAAGCATTGTCTCTGAATGTTAAT
TTTTTGGTACAAAAAATAAATTTGTACGAAAAAAAAAAAAAAAAAAAAAAAA
AA

SEQ ID NO: 4 (mouse RANKL)

MRRASRDYGKYLRSSEEMGSGPGVPHEGPLHPAPSAPAPAPPPAASRSMFLALL
GLGLGQVVCSIALFLYFRAQMDPNRISEDSTHCFYRILRLHENADLQDSTLESED
TLPDSCRRMKQAFQGAVQKELQHIVGPQRFSGAPAMMEGSWLDVAQRGKPEA
QPFAHLTINAASIPSGSHKVTLSSWYHDRGWAKISNMTLSNGKLRVNQDGFYYL
YANICFRHHETSGSVPTDYLQLMVYVVKTSIKIPSSHNLMKGGSTKNWSGNSEF
HFYSINVGGFFKLRAGEEISIQVSNPSLLDPDQDATYFGAFKVQDID

SEQ ID NO: 5 (human TRAIL)

cctcactgactataaaagaatagagaaggaagggcttcagtgaccggctgcctggctgacttacagcagtcagactctgacaggatcatgg
ctatgatggaggtccagggggggacccagcctgggacagacctgcgtgctgatcgtgatcttcacagtgctcctgcagtctctctgtgtggct
gtaacttacgtgtactttaccaacgagctgaagcagatgcaggacaagtactccaaaagtggcattgcttgtttcttaaaagaagatgacagtt
attgggaccccaatgacgaagagagtatgaacagcccctgctggcaagtcaagtggcaactccgtcagctcgttagaaagatgattttgag
aacctctgaggaaaccatttctacagttcaagaaaagcaacaaaatatttctccctagtgagagaaagaggtcctcagagagtagcagctc
acataactgggaccagaggaagaagcaacacattgtcttctccaaactccaagaatgaaaaggctctgggccgcaaaataaactcctggg
aatcatcaaggagtgggcattcattcctgagcaacttgcacttgaggaatggtgaactggtcatccatgaaaaagggttttactacatctattcc
caaacatactttcgatttcaggaggaaataaaagaaaacacaaagaacgacaaacaaatggtccaatatatttacaaatacacaagttatcct
gaccctatattgttgatgaaaagtgctagaaatagttgttggtctaaagatgcagaatatggactctattccatctatcaagggggaatatttgag
cttaaggaaaatgacagaattttgtttctgtaacaaatgagcacttgatagacatggaccatgaagccagttttttcggggcctttttagttggct
aactgacctggaaagaaaaagcaataacctcaaagtgactattcagttttcaggatgatacactatgaagatgtttcaaaaaatctgaccaaaa
caaacaaacagaaaacagaaaacaaaaaaacctctatgcaatctgagtagagcagccacaaccaaaaaattctacaacacacactgttctg

aaagtgactcacttatcccaagaaaatgaaattgctgaaagatctttcaggactctacctcatatcagtttgctagcagaaatctagaagactgt

cagcttccaaacattaatgcaatggttaacatcttctgtctttataatctactccttgtaaagactgtagaagaaagcgcaacaatccatctctcaa

gtagtgtatcacagtagtagcctccaggtttccttaagggacaacatccttaagtcaaaagagagaagaggcaccactaaaagatcgcagtt

tgcctggtgcagtggctcacacctgtaatcccaacattttgggaacccaaggtgggtagatcacgagatcaagagatcaagaccatagtga

ccaacatagtgaaaccccatctctactgaaagtgcaaaaattagctgggtgtgttggcacatgcctgtagtcccagctacttgagaggctgag

gcaggagaatcgtttgaacccgggaggcagaggttgcagtgtggtgagatcatgccactacactccagcctggcgacagagcgagactt

ggtttcaaaaaaaaaaaaaaaaaaaaaacttcagtaagtacgtgttattttttttcaataaaattctattacagtatgtcaaaaaaaaaaaaaaaaaa

SEQ IS NO: 6 (human TRAIL)

MAMMEVQGGPSLGQTCVLIVIFTVLLQSLCVAVTYVYFTNELKQMQDKYSKSG
IACFLKEDDSYWDPNDEESMNSPCWQVKWQLRQLVRKMILRTSEETISTVQEK
QQNISPLVRERGPQRVAAHITGTRGRSNTLSSPNSKNEKALGRKINSWESSRSGH
SFLSNLHLRNGELVIHEKGFYYIYSQTYFRFQEEIKENTKNDKQMVQYIYKYTSY
PDPILLMKSARNSCWSKDAEYGLYSIYQGGIFELKENDRIFVSVTNEHLIDMDHE
ASFFGAFLVG

SEQ ID NO: 7 (human APRIL)

agcatcctgagtaatgagtggcctgggccggagcaggcgaggtggccggagccgtgtggaccaggaggagcgctttccac

agggcctgtggacgggggtggctatgagatcctgccccgaagagcagtactgggatcctctgctgggtacctgcatgtcctgc

aaaaccatttgcaaccatcagagccagcgcacctgtgcagccttctgcaggtcactcagctgccgcaaggagcaaggcaagtt

ctatgaccatctcctgagggactgcatcagctgtgcctccatctgtggacagcaccctaagcaatgtgcatacttctgtgagaac

aagctcaggagcccagtgaaccttccaccagagctcaggagacagcggagtggagaagttgaaaacaattcagacaactcg

ggaaggtaccaaggattggagcacagaggctcagaagcaagtccagctctcccggggctgaagctgagtgcagatcaggtg

gccctggtctacagcacgctggggctctgcctgtgtgccgtcctctgctgcttcctggtggcggtggcctgcttcctcaagaaga

gggggggatccctgctcctgccagccccgctcaaggccccgtcaaagtccggccaagtcttcccaggatcacgcgatggaag

ccggcagccctgtgagcacatcccccgagccagtggagacctgcagcttctgcttccctgagtgcagggcgcccacgcagg

agagcgcagtcacgcctgggacccccgaccccacttgtgctggaaggtgggggtgccacaccaggaccacagtcctgcag

ccttgcccacacatcccagacagtggccttggcattgtgtgtgtgcctgcccaggaggggggcccaggtgcataaatgggggt

cagggagggaaaggaggagggagagagatggagaggaggggagagagaaagagaggtggggagaggggagagagat

atgaggagagagagacagaggaggcagagagggagagaaacagaggagacagagagggagagagagacagagggag

agagagacagaggggaagagaggcagagagggaaagaggcagagaaggaaagagacaggcagagaaggagagagg

cagagagggagagaggcagagagggagagaggcagagagacagagagggagagagggacagagagagatagagcag

gaggtcggggcactctgagtcccagttcccagtgcagctgtaggtcgtcatcacctaaccacacgtgcaataaagtcctcgtgc

ctgctgctcacagcccccgagagcccctcctcctggagaataaaacctttggcagctgcccttcctcaaaaaaaaaaaaaaaaaa
aaa

SEQ ID NO: 8 (human APRIL)

MPASSPFLLAPKGPPGNMGGPVREPALSVALWLSWGAALGAVACAMALLTQQ
TELQSLRREVSRLQGTGGPSQNGEGYPWQSLPEQSSDALEAWENGERSRKRRA
VLTQKQKKQHSVLHLVPINATSKDDSDVTEVMWQPALRRGRGLQAQGYGVRI
QDAGVYLLYSQVLFQDVTFTMGQVVSREGQGRQETLFRCIRSMPSHPDRAYNS
CYSAGVFHLHQGDILSVIIPRARAKLNLSPHGTFLGFVKL

SEQ ID NO: 9 (human BAFF)

Agctcagcctcagtccccgcagcttgtgcggcggcgtcggcaccatgaggcgagggccccggagcctgcggggcaggga
cgcgccagccccacgccctgcgtcccggccgagtgcttcgacctgctggtccgccactgcgtggcctgcgggctcctgcgc
acgccgcggccgaaaccggccgggggccagcagccctgcgcccaggacggcgctgcagccgcaggagtcggtgggcgc
ggggggccggcgaggcggcgctgccctgcccgggctgctctttggcgcccccgcgctgctgggcctggcactggtcctggc
gctggtcctggtgggtctggtgagctggaggcggcgacagcggcggcttcgcggcgcgtcctccgcagaggccccccgacg
gagacaaggacgccccagagccctggacaaggtcatcattctgtctccgggaatctctgatgccacagctcctgcctggcct
cctcctggggaagacccaggaaccaccccacctggccacagtgtccctgtgccagccacagagctgggctccactgaactg
gtgaccaccaagacggccggccctgagcaacaatagcagggagccggcaggaggtggcccctgccctccctctggacccc
cagccaggggcttggaaatcaaattcagctcttcactccagcatgcacatgccctctttctgggaccaggctaactctgcagaag
cacagacactacagaccacagcattcagcccccatggagtttggtgtgcttgcctttggcttcagacctcaccatctttgacagcc
cttgaaggtggtagcccagctcctgttcctgtgccttcaaaaggctggggcactatgagtaaaagaccgcttttaaaatggggaa
ggcaccattaagccaaaatgaatctgaaaaaagac

SEQ ID NO: 10 (human BAFF)

MDDSTEREQSRLTSCLKKREEMKLKECVSILPRKESPSVRSSKDGKLLAATLLLA
LLSCCLTVVSFYQVAALQGDLASLRAELQGHHAEKLPAGAGAPKAGLEEAPAV
TAGLKIFEPPAPGEGNSSQNSRNKRAVQGPEETVTQDCLQLIADSETPTIQKGSY
TFVPWLLSFKRGSALEEKENKILVKETGYFFIYGQVLYTDKTYAMGHLIQRKKV
HVFGDELSLVTLFRCIQNMPETLPNNSCYSAGIAKLEEGDELQLAIPRENAQISLD
GDVTFFGALKLL

SEQ ID NO: 11 (TNFalpha)

ctccctcagcaaggacagcagaggaccagctaagagggagagaagcaactacagaccccccctgaaaacaaccctcagac
gccacatccctgacaagctgccaggcaggttctcttcctctcacatactgacccacggctccaccctctctccctggaaagg
acaccatgagcactgaaagcatgatccgggacgtggagctggccgaggaggcgctccccaagaagacaggggggcccca
gggctccaggcggtgcttgttcctcagcctcttctccttcctgatcgtggcaggcgccaccacgctcttctgcctgctgcactttg

gagtgatcggcccccagagggaagagttccccagggacctctctctaatcagccctctggcccaggcagtcagatcatcttctc gaaccccgagtgacaagcctgtagcccatgttgtagcaaaccctcaagctgaggggcagctccagtggctgaaccgccggg ccaatgccctcctggccaatggcgtggagctgagagataaccagctggtggtgccatcagagggcctgtacctcatctactcc caggtcctcttcaagggccaaggctgcccctccacccatgtgctcctcacccacaccatcagccgcatcgccgtctcctaccag accaaggtcaacctcctctctgccatcaagagccctgccagagggagaccccagaggggggctgaggccaagccctggtat gagcccatctatctgggaggggtcttccagctggagaagggtgaccgactcagcgctgagatcaatcggcccgactatctcga ctttgccgagtctgggcaggtctactttgggatcattgccctgtgaggaggacgaacatccaaccttcccaaacgcctcccctgc cccaatcccttattaccccctccttcagacaccctcaacctcttctggctcaaaaagagaattggggggcttagggtcggaaccca agcttagaactttaagcaacaagaccaccacttcgaaacctgggattcaggaatgtgtggcctgcacagtgaagtgctggcaac cactaagaattcaaactggggcctccagaactcactggggcctacagctttgatccctgacatctggaatctggagaccaggga gcctttggttctggccagaatgctgcaggacttgagaagacctcacctagaaattgacacaagtggaccttaggccttcctctctc cagatgtttccagacttccttgagacacggagcccagccctccccatggagccagctccctctatttatgtttgcacttgtgattatt tattatttatttattatttatttatttacagatgaatgtatttatttgggagaccggggtatcctggggggacccaatgtaggagctgcctt ggctcagacatgttttccgtgaaaacggagctgaacaataggctgttcccatgtagcccccctggcctctgtgccttcttttgattat gttttttaaaatatttatctgattaagttgtctaaacaatgctgatttggtgaccaactgtcactcattgctgagcctctgctcccccagg ggagttgtgtctgtaatcgccctactattcagtggcgagaaataaagtttgcttagaaaagaa

SEQ ID NO: 12 (TNFalpha)

MSTESMIRDVELAEEALPKKTGGPQGSRRCLFLSLFSFLIVAGATTLFCLLHFGVI GPQREEFPRDLSLISPLAQAVRSSSRTPSDKPVAHVVANPQAEGQLQWLNRRAN ALLANGVELRDNQLVVPSEGLYLIYSQVLFKGQGCPSTHVLLTHTISRIAVSYQT KVNLLSAIKSPCQRETPEGAEAKPWYEPIYLGGVFQLEKGDRLSAEINRPDYLDF AESGQVYFGIIAL

SEQ ID NO: 13 (CD30L)

CCCTTGCTGCCGCCTCTCGCCTCACGCCCCCAGAGAAGAGTTTCTCCACCAG GCAGCAGGTGAAGGTTTTTTTCCAAGTCACATGATTCAGGATTCAGGGGGAG AATCCTTCTTGGAACAGAGATGGGCCCAGAACTGAATCAGATGAAGAGAGA TAAGGTGTGATGTGGGGAAGACTATATAAAGAATGGACCCAGGGCTGCAGC AAGCACTCAACGGAATGGCCCCTCCTGGAGACACAGCCATGCATGTGCCGG CGGGCTCCGTGGCCAGCCACCTGGGGACCACGAGCCGCAGCTATTTCTATTT GACCACAGCCACTCTGGCTCTGTGCCTTGTCTTCACGGTGGCCACTATTATGG TGTTGGTCGTTCAGAGGACGGACTCCATTCCCAACTCACCTGACAACGTCCC CCTCAAAGGAGGAAATTGCTCAGAAGACCTCTTATGTATCCTGAAAAGGGCT

CCATTCAAGAAGTCATGGGCCTACCTCCAAGTGGCAAAGCATCTAAACAAA

ACCAAGTTGTCTTGGAACAAAGATGGCATTCTCCATGGAGTCAGATATCAGG

ATGGGAATCTGGTGATCCAATTCCCTGGTTTGTACTTCATCATTTGCCAACTG

CAGTTTCTTGTACAATGCCCAAATAATTCTGTCGATCTGAAGTTGGAGCTTCT

CATCAACAAGCATATCAAAAAACAGGCCCTGGTGACAGTGTGTGAGTCTGG

AATGCAAACGAAACACGTATACCAGAATCTCTCTCAATTCTTGCTGGATTAC

CTGCAGGTCAACACCACCATATCAGTCAATGTGGATACATTCCAGTACATAG

ATACAAGCACCTTTCCTCTTGAGAATGTGTTGTCCATCTTCTTATACAGTAAT

TCAGACTGAACAGTTTCTCTTGGCCTTCAGGAAGAAAGCGCCTCTCTACCAT

ACAGTATTTCATCCCTCCAAACACTTGGGCAAAAGAAAACTTTAGACCAAG

ACAAACTACACAGGGTATTAAATAGTATACTTCTCCTTCTGTCTCTTGGAAA

GATACAGCTCCAGGGTTAAAAAGAGAGTTTTTAGTGAAGTATCTTTCAGATA

GCAGGCAGGGAAGCAATGTAGTGTGGTGGGCAGAGCCCCACACAGAATCAG

AAGGGATGAATGGATGTCCCAGCCCAACCTCTAATTCACTGTATGGTCTTGA

TCTATTTCTTCTGTTTTGAGAGCCTCCAGTTAAAATGGGGCTTCAGTACCAGA

GCAGCTAGCAACTCTGCCCTAATGGGAAATGAAGGGGAGCTGGGTGTGAGT

GTTTACACTGTGCCCTTCACGGGATACTTCTTTTATCTGCAGATGGCCTAATA

CTTAGTTGTCCAAGTCGCGATCAAGGACTCTCTCACACAGGAAACTTCCCTA

TACTGGCAGATACACTTGTGACTGAACCATGCCCAGTTTATGCCTGTCTGAC

TGTCACTCTGGCACTAGGAGGCTGATCTTGTACTCCATATGACCCCACCCCT

AGGAACCCCCAGGGAAAACCAGGCTGGGACAGCCCCCTGTTCCTGAGATGG

AAAGCACAAATTTAATACACCACCACAATGGAAAACAAGTTCAAAGACTTT

TACTTACAGATCCTGGACAGAAAGGGCATAATGAGTCTGAAGGGCAGTCCT

CCTTCTCTAGGTTACATGAGGCAGGAATAAGAAGTCAGACAGAGACAGCAA

GACAGTTAACAATGTAGGTAAAGAAATAGGGTGTGGTCACTCTCAATTCACT

GGCAAATGCCTGAATGGTCTGTCTGAAGGAAGCAACAGAGAAGTGGGGAAT

CCAGTCTGCTAGGCAGGAAAGATGCCTCTAAGTTCTTGTCTCTGGCCAGAGG

TGTGGTATAGAACCAGAAACCCATATCAAGGGTGACTAAGCCCGGCTTCTGG

TATGAGAAATTAAACTTGTATACAAATGGTTGCCAAGGCAACATAAAATTA

TAAGAATTC

SEQ ID NO: 14 (CD30L)

MDPGLQQALNGMAPPGDTAMHVPAGSVASHLGTTSRSYFYLTTATLALCLVFT
VATIMVLVVQRTDSIPNSPDNVPLKGGNCSEDLLCILKRAPFKKSWAYLQVAKH

LNKTKLSWNKDGILHGVRYQDGNLVIQFPGLYFIICQLQFLVQCPNNSVDLKLE
LLINKHIKKQALVTVCESGMQTKHVYQNLSQFLLDYLQVNTTISVNVDTFQYID
TSTFPLENVLSIFLYSNSD

SEQ ID NO: 15 (human CD40L)

actttgacagtcttctcatgctgcctctgccaccttctctgccagaagataccatttcaactttaacacagcatgatcgaaacataca
accaaacttctccccgatctgcggccactggactgcccatcagcatgaaaatttttatgtatttacttactgtttttcttatcacccaga
tgattgggtcagcactttttgctgtgtatcttcatagaaggttggacaagatagaagatgaaaggaatcttcatgaagattttgtattc
atgaaaacgatacagagatgcaacacaggagaaagatccttatccttactgaactgtgaggagattaaaagccagtttgaaggc
tttgtgaaggatataatgttaaacaaagaggagacgaagaaagaaaacagctttgaaatgcaaaaaggtgatcagaatcctcaa
attgcggcacatgtcataagtgaggccagcagtaaaacaacatctgtgttacagtgggctgaaaaaggatactacaccatgagc
aacaacttggtaaccctggaaaatgggaaacagctgaccgttaaaagacaaggactctattatatctatgcccaagtcaccttct
gttccaatcgggaagcttcgagtcaagctccatttatagccagcctctgcctaaagtcccccggtagattcgagagaatcttactc
agagctgcaaatacccacagttccgccaaaccttgcgggcaacaatccattcacttgggaggagtatttgaattgcaaccaggt
gcttcggtgtttgtcaatgtgactgatccaagccaagtgagccatggcactggcttcacgtcctttggcttactcaaactctgaaca
gtgtcaccttgcaggctgtggtggagctgacgctgggagtcttcataatacagcacagcggttaagcccacccccctgttaactg
cctatttataaccctaggatcctccttatggagaactatttattatacactccaaggcatgtagaactgtaataagtgaattacaggtc
acatgaaaccaaaacgggccctgctccataagagcttatatatctgaagcagcaaccccactgatgcagacatccagagagtc
ctatgaaaagacaaggccattatgcacaggttgaattctgagtaaacagcagataacttgccaagttcagttttgtttcttgcgtgc
agtgtctttccatggataatgcatttgatttatcagtgaagatgcagaagggaaatggggagcctcagctcacattcagttatggtt
gactctgggttcctatggccttgttggaggggggccaggctctagaacgtctaacacagtggagaaccgaaaccccccccccccc
ccccgccaccctctcggacagttattcattctctttcaatctctctctctccatctctctctttcagtctctctctctcaacctctttcttcc
aatctctctttctcaatctctctgtttcccttttgtcagtctcttccctcccccagtctctcttctcaatcccccttctaacacacacacac
acacacacacacacacacacacacacacacacacacacacagagtcaggccgttgctagtcagttctcttctttccaccctgtc
cctatctctaccactatagatgagggtgaggagtagggagtgcagccctgagcctgcccactcctcattacgaaatgactgtattt
aaaggaaatcattgtatctacctgcagtctccattgtttccagagtgaacttgtaattatcttgttatttatttttttgaataataaagacct
cttaacattaa

SEQ ID NO: 16 (human CD40L)

MIETYNQTSPRSAATGLPISMKIFMYLLTVFLITQMIGSALFAVYLHRRLDKIEDE
RNLHEDFVFMKTIQRCNTGERSLSLLNCEEIKSQFEGFVKDIMLNKEETKKENSF
EMQKGDQNPQIAAHVISEASSKTTSVLQWAEKGYYTMSNNLVTLENGKQLTVK
RQGLYYIYAQVTFCSNREASSQAPFIASLCLKSPGRFERILLRAANTHSSAKPCGQ
QSIHLGGVFELQPGASVFVNVTDPSQVSHGTGFTSFGLLKL

SEQ ID NO: 17 (FasL)

gaggtgtttcccttagctatggaaactctataagagagatccagcttgcctcctcttgagcagtcagcaacagggtcccgtccttg

acacctcagcctctacaggactgagaagaagtaaaaccgtttgctggggctggcctgactcaccagctgccatgcagcagccc

ttcaattacccatatccccagatctactgggtggacagcagtgccagctctccctgggcccctccaggcacagttcttccctgtcc

aacctctgtgcccagaaggcctggtcaaaggaggccaccaccaccaccgccaccgccaccactaccacctccgccgccgcc

gccaccactgcctccactaccgctgccacccctgaagaagagagggaaccacagcacaggcctgtgtctccttgtgatgttttt

catggttctggttgccttggtaggattgggcctggggatgtttcagctcttccacctacagaaggagctggcagaactccgagag

tctaccagccagatgcacacagcatcatctttggagaagcaaataggccaccccagtccacccctgaaaaaaaggagctga

ggaaagtggcccatttaacaggcaagtccaactcaaggtccatgcctctggaatgggaagacacctatggaattgtcctgctttc

tggagtgaagtataagaagggtggccttgtgatcaatgaaactgggctgtactttgtatattccaaagtatacttccggggtcaatc

ttgcaacaacctgcccctgagccacaaggtctacatgaggaactctaagtatccccaggatctggtgatgatggagggggaaga

tgatgagctactgcactactgggcagatgtgggcccgcagcagctacctggggggcagtgttcaatcttaccagtgctgatcattt

atatgtcaacgtatctgagctctctctggtcaattttgaggaatctcagacgtttttcggcttatataagctctaagagaagcactttg

ggattctttccattatgattctttgttacaggcaccgagaatgttgtattcagtgagggtcttcttacatgcatttgaggtcaagtaaga

agacatgaaccaagtggaccttgagaccacagggttcaaaatgtctgtagctcctcaactcacctaatgtttatgagccagacaa

atggaggaatatgacggaagaacatagaactctgggctgccatgtgaagagggagaagcatgaaaaagcagctaccaggtg

ttctacactcatcttagtgcctgagagtatttaggcagattgaaaaggacaccttttaactcacctctcaaggtgggccttgctacct

caaggggggactgtctttcagatacatggttgtgacctgaggatttaagggatggaaaaggaagactagaggcttgcataataag

ctaaagaggctgaaagaggccaatgccccactggcagcatcttcacttctaaatgcatatcctgagccatcggtgaaactaaca

gataagcaagagagatgttttgggggactcatttcattcctaacacagcatgtgtatttccagtgcaattgtagggggtgtgtgtgtg

tgtgtgtgtgtgtgtgtgtatgactaaagagagaatgtagatattgtgaagtacatattaggaaaatatgggttgcatttggtcaaga

ttttgaatgcttcctgacaatcaactctaatagtgcttaaaaatcattgattgtcagctactaatgatgttttcctataatataataaatatt

tatgtagatgtgcattttgtgaaatgaaaacatgtaataaaaagtatatgttaggatacaaaaaaaaaaaaaaaaaaaaaaaaaaa

aaaaaaaaaaaaaaaaaaaaaaa

SEQ ID NO: 18 (FasL)

MQQPFNYPYPQIYWVDSSASSPWAPPGTVLPCPTSVPRRPGQRRPPPPPPPPLPP
PPPPPPLPPLPLPPLKKRGNHSTGLCLLVMFFMVLVALVGLGLGMFQLFHLQKEL
AELRESTSQMHTASSLEKQIGHPSPPPEKKELRKVAHLTGKSNSRSMPLEWEDT
YGIVLLSGVKYKKGGLVINETGLYFVYSKVYFRGQSCNNLPLSHKVYMRNSKY
PQDLVMMEGKMMSYCTTGQMWARSSYLGAVFNLTSADHLYVNVSELSLVNFE
ESQTFFGLYKL

SEQ ID NO: 19 (Light)

MEESVVRPSVFVVDGQTDIPFTRLGRSHRRQSCSVARVGLGLLLLLMGAGLAV
QGWFLLQLHWRLGEMVTRLPDGPAGSWEQLIQERRSHEVNPAAHLTGANSSLT
GSGGPLLWETQLGLAFLRGLSYHDGALVVTKAGYYYIYSKVQLGGVGCPLGLA
STITHGLYKRTPRYPEELELLVSQQSPCGRATSSSRVWWDSSFLGGVVHLEAGE
KVVVRVLDERLVRLRDGTRSYFGAFMV

SEQ ID NO: 20 (Light)

GAGGTTGAAGGACCCAGGCGTGTCAGCCCTGCTCCAGAGACCTTGGGCATG
GAGGAGAGTGTCGTACGGCCCTCAGTGTTTGTGGTGGATGGACAGACCGAC
ATCCCATTCACGAGGCTGGGACGAAGCCACCGGAGACAGTCGTGCAGTGTG
GCCCGGGTGGGTCTGGGTCTCTTGCTGTTGCTGATGGGGGCTGGGCTGGCCG
TCCAAGGCTGGTTCCTCCTGCAGCTGCACTGGCGTCTAGGAGAGATGGTCAC
CCGCCTGCCTGACGGACCTGCAGGCTCCTGGGAGCAGCTGATACAAGAGCG
AAGGTCTCACGAGGTCAACCCAGCAGCGCATCTCACAGGGGCCAACTCCAG
CTTGACCGGCAGCGGGGGGCCGCTGTTATGGGAGACTCAGCTGGGCCTGGC
CTTCCTGAGGGGCCTCAGCTACCACGATGGGGCCCTTGTGGTCACCAAAGCT
GGCTACTACTACATCTACTCCAAGGTGCAGCTGGGCGGTGTGGGCTGCCCGC
TGGGCCTGGCCAGCACCATCACCCACGGCCTCTACAAGCGCACACCCCGCTA
CCCCGAGGAGCTGGAGCTGTTGGTCAGCCAGCAGTCACCCTGCGGACGGGC
CACCAGCAGCTCCCGGGTCTGGTGGGACAGCAGCTTCCTGGGTGGTGTGGTA
CACCTGGAGGCTGGGGAGGAGGTGGTCGTCCGTGTGCTGGATGAACGCCTG
GTTCGACTGCGTGATGGTACCCGGTCTTACTTCGGGGCTTTCATGGTGTGAA
GGAAGGAGCGTGGTGCATTGGACATGGGTCTGACACGTGGAGAACTCAGAG
GGTGCCTCAGGGGAAAGAAAACTCACGAAGCAGAGGCTGGGCGTGGTGGCT
CTCGCCTGTAATCCCAGCACTTTGGGAGGCCAAGGCAGGCGGATCACCTGAG
GTCAGGAGTTCGAGACCAGCCTGGCTAACATGGCAAAACCCCATCTCTACTA
AAAATACAAAAATTAGCCGGAC

SEQ ID NO: 21 (Tweak)

CACAGCCCCCGCCCCCATGGCCGCCCGTCGGAGCCAGAGGCGGAGGGGGC
GCCGGGGGGAGCCGGGCACCGCCCTGCTGGTCCCGCTCGCGCTGGGCCTGG
GCCTGGCGCTGGCCTGCCTCGGCCTCCTGCTGGCCGTGGTCAGTTTGGGGAG
CCGGGCATCGCTGTCCGCCCAGGAGCCTGCCCAGGAGGAGCTGGTGGCAGA
GGAGGACCAGGACCCGTCGGAACTGAATCCCCAGACAGAAGAAAGCCAGG

ATCCTGCGCCTTTCCTGAACCGACTAGTTCGGCCTCGCAGAAGTGCACCTAA

AGGCCGGAAAACACGGGCTCGAAGAGCGATCGCAGCCCATTATGAAGTTCA

TCCACGACCTGGACAGGACGGAGCGCAGGCAGGTGTGGACGGGACAGTGAG

TGGCTGGGAGGAAGCCAGAATCAACAGCTCCAGCCCTCTGCGCTACAACCG

CCAGATCGGGGAGTTTATAGTCACCCGGGCTGGGCTCTACTACCTGTACTGT

CAGGTGCACTTTGATGAGGGGAAGGCTGTCTACCTGAAGCTGGACTTGCTGG

TGGATGGTGTGCTGGCCCTGCGCTGCCTGGAGGAATTCTCAGCCACTGCGGC

CAGTTCCCTCGGGCCCCAGCTCCGCCTCTGCCAGGTGTCTGGGCTGTTGGCC

CTGCGGCCAGGGTCCTCCCTGCGGATCCGCACCCTCCCCTGGGCCCATCTCA

AGGCTGCCCCCTTCCTCACCTACTTCGGACTCTTCCAGGTTCACTGAGGGGCC

CTGGTCTCCCCACAGTCGTCCCAGGCTGCCGGCTCCCCTCGACAGCTCTCTG

GGCACCCGGTCCCCTCTGCCCCACCCTCAGCCGCTCTTTGCTCCAGACCTGCC

CCTCCCTCTAGAGGCTGCCTGGGCCTGTTCACGTGTTTTCCATCCCACATAAA

TACAGTATTCCCACTCTTATCTTACAACTCCCCCACCGCCCACTCTCCACCTC

ACTAGCTCCCCAATCCCTGACCCTTTGAGGCCCCCAGTGATCTCGACTCCCCC

CTGGCCACAGACCCCCAGGGCATTGTGTTCACTGTACTCTGTGGGCAAGGAT

GGGTCCAGAAGACCCCACTTCAGGCACTAAGAGGGGCTGGACCTGGCGGCA

GGAAGCCAAAGAGACTGGGCCTAGGCCAGGAGTTCCCAAATGTGAGGGGCG

AGAAACAAGACAAGCTCCTCCCTTGAGAATTCCCTGTGGATTTTTAAAACAG

ATATTATTTTTATTATTATTGTGACAAATGTTGATAAATGGATATTAAATAG

AATAAGTCAG

SEQ ID NO: 22 (Tweak)

MAARRSQRRRGRRGEPGTALLVPLALGLGLALACLGLLLAVVSLGSRASLSAQ

EPAQEELVAEEDQDPSELNPQTEESQDPAPFLNRLVRPRRSAPKGRKTRARRAIA

AHYEVHPRPGQDGAQAGVDGTVSGWEEARINSSSPLRYNRQIGEFIVTRAGLYY

LYCQVHFDEGKAVYLKLDLLVDGVLALRCLEEFSATAASSLGPQLRLCQVSGLL

ALRPGSSLRIRTLPWAHLKAAPFLTYFGLFQVH

SEQ ID NO: 23 (TNFβ)

GAGGTTTATTGGGCCTCGGTCCTCCTGCACCTGCTGCCTGGATCCCCGGCCTG

CCTGGGCCTGGGCCTTGGTTCTCCCCATGACACCACCTGAACGTCTCTTCCTC

CCAAGGGTGTGTGGCACCACCCTACACCTCCTCCTTCTGGGGCTGCTGCTGG

TTCTGCTGCCTGGGGCCCAGGGGCTCCCTGGTGTTGGCCTCACACCTTCAGCT

GCCCAGACTGCCCGTCAGCACCCCAAGATGCATCTTGCCCACAGCACCCTCA

AACCTGCTGCTCACCTCATTGGAGACCCCAGCAAGCAGAACTCACTGCTCTG

GAGAGCAAACACGGACCGTGCCTTCCTCCAGGATGGTTTCTCCTTGAGCAAC

AATTCTCTCCTGGTCCCCACCAGTGGCATCTACTTCGTCTACTCCCAGGTGGT

CTTCTCTGGGAAAGCCTACTCTCCCAAGGCCACCTCCTCCCCACTCTACCTGG

CCCATGAGGTCCAGCTCTTCTCCTCCCAGTACCCCTTCCATGTGCCTCTCCTC

AGCTCCCAGAAGATGGTGTATCCAGGGCTGCAGGAACCCTGGCTGCACTCG

ATGTACCACGGGGCTGCGTTCCAGCTCACCCAGGGAGACCAGCTATCCACCC

ACACAGATGGCATCCCCCACCTAGTCCTCAGCCCTAGTACTGTCTTCTTTGGA

GCCTTCGCTCTGTAGAACTTGGAAAAATCCAGAAAGAAAAAATAATTGATTT

CAAGACCTTCTCCCCATTCTGCCTCCATTCTGACCATTTCAGGGGTCGTCACC

ACCTCTCCTTTGGCCATTCCAACAGCTCAAGTCTTCCCTGATCAAGTCACCGG

AGCTTTCAAAGAAGGAATTCTAGGCATCCCAGGGGACCCACACTCCCTGAAC

CATCCCTGATGTCTGTCTGGCTGAGGATTTCAAGCCTGCCTAGGAATTCCCA

GCCCAAAGCTGTTGGTCTTGTCCACCAGCTAGGTGGGGCCTAGATCCACACA

CAGAGGAAGAGCAGGCACATGGAGGAGCTTGGGGGATGACTAGAGGCAGG

GAGGGGACTATTTATGAAGGCAAAAAAATTAAATTATTTATTTATGGAGGAT

GGAGAGAGGGAATAATAGAAGAACATCCAAGGAGAAACAGAGACAGGCCC

AAGAGATGAAGAGTGAGAGGGCATGCGCACAAGGCTGACCAAGAGAGAAA

GAAGTAGGCATGAGGGATCACAGGGCCCCAGAAGGCAGGGAAAGGCTCTG

AAAGCCAGCTGCCGACCAGAGCCCCACACGGAGGCATCTGCACCCTCGATG

AAGCCCAATAAACCTCTTTTCTCTGA

SEQ ID NO: 24 (TNFβ)

MTPPERLFLPRVCGTTLHLLLLGLLLVLLPGAQGLPGVGLTPSAAQTARQHPKM

HLAHSTLKPAAHLIGDPSKQNSLLWRANTDRAFLQDGFSLSNNSLLVPTSGIYFV

YSQVVFSGKAYSPKATSSPLYLAHEVQLFSSQYPFHVPLLSSQKMVYPGLQEPW

LHSMYHGAAFQLTQGDQLSTHTDGIPHLVLSPSTVFFGAFAL

SEQ ID NO: 25 (murine APRIL)

CATGCCGAGTGCTTTGTGTGTGTTACCTGCTCTAAGAAGCTGGCTGGGCAGC

GTTTCACCGCTGTGGAGGACCAGTATTACTGCGTGGATTGCTACAAGAACTT

TGTGGCCAAGAAGTGTGCTGGATGCAAGAACCCCATCACTGGGTTTGGTAAA

GGCTCCAGTGTGGTGGCCTATGAAGGACAATCCTGGCACGACTACTGCTTCC

ACTGCAAAAAATGCTCCGTGAATCTGGCCAACAAGCGCTTTGTATTTCATAA
TGAGCAGGTGTATTGCCCTGACTGTGCCAAAAAGCTGTAACTTGACGGCTGC
CCTGTCCTTCCTAGATAATGGCACCAAATTCTCCTGAGGCTAGGGGGGAAGG
AGTGTCAGAGTGTCACTAGCTCGACCCTGGGGACAAGGGGGACTAATAGTA
CCCTAGCTTGATTTCTTCCTATTCTCAAGTTCCTTTTTATTTCTCCCTTGCGTA
ACCCGCTCTTCCCTTCTGTGCCTTTGCCTGTATTCCCACCCTCCCTGCTACCTC
TTGGCCACCTCACTTCTGAGACCACAGCTGTTGGCAGGGTCCCTAGCTCATG
CCAGCCTCATCTCCAGGCCACATGGGGGGCTCAGTCAGAGAGCCAGCCCTTT
CGGTTGCTCTTTGGTTGAGTTGGGGGGCAGTTCTGGGGGCTGTGACTTGTGC
TGTCGCACTACTGATCCAACAGACAGAGCTGCAAAGCCTAAGGCGGGAGGT
GAGCCGGCTGCAGCGGAGTGGAGGGCCTTCCCAGAAGCAGGGAGAGCGCCC
ATGGCAGAGCCTCTGGGAGCAGAGTCCTGATGTCCTGGAAGCCTGGAAGGA
TGGGGCGAAATCTCGGAGAAGGAGAGCAGTACTCACCCAGAAGCACAAGAA
GAAGCACTCAGTCCTGCATCTTGTTCCAGTTAACATTACCTCCAAGGACTCT
GACGTGACAGAGGTGATGTGGCAACCAGTACTTAGGCGTGGGAGAGGCCTG
GAGGCCCAGGGAGACATTGTACGAGTCTGGGACACTGGAATTTATCTGCTCT
ATAGTCAGGTCCTGTTTCATGATGTGACTTTCACAATGGGTCAGGTGGTATCT
CGGGAAGGACAAGGGAGAAGAGAAACTCTATTCCGATGTATCAGAAGTATG
CCTTCTGATCCTGACCGTGCCTACAATAGCTGCTACAGTGCAGGTGTCTTTCA
TTTACATCAAGGGGATATTATCACTGTCAAAATTCCACGGGCAAACGCAAAA
CTTAGCCTTTCTCCGCATGGAACATTCCTGGGGTTTGTGAAACTATGATTGTT
ATAAAGGGGGTGGGGATTTCCCATTCCAAAAACTGGCTAGACAAAGGACAA
GGAACGGTCAAGAACAGCTCTCCATGGCTTTGCCTTGACTGTTGTTCCTCCCT
TTGCCTTTCCCGCTCCCACTATCTGGGCTTTGACTCCATGGATATTAAAAAAG
TAGAATATTTTGGTTTATCTCCCAAAAA

SEQ ID NO: 26 (murine APRIL)

MPASSPGHMGGSVREPALSVALWLSWGAVLGAVTCAVALLIQQTELQSLRREV
SRLQRSGGPSQKQGERPWQSLWEQSPDVLEAWKDGAKSRRRRAVLTQKHKKK
HSVLHLVPVNITSKDSDVTEVMWQPVLRRGRGLEAQGDIVRVWDTGIYLLYSQ
VLFHDVTFTMGQVVSREGQGRRETLFRCIRSMPSDPDRAYNSCYSAGVFHLHQ
GDIITVKIPRANAKLSLSPHGTFLGFVKL

SEQ ID NO: 27 (Human OX40L)

ccgcaaggaaaacccagactctggcgacagcagagacgaggatgtgcgtgggggctcggcggctgggccgcgggccgtg tgcggctctgctcctcctgggcctggggctgagcaccgtgacggggctccactgtgtcggggacacctaccccagcaacgac cggtgctgccacgagtgcaggccaggcaacgggatggtgagccgctgcagccgctcccagaacacggtgtgccgtccgtg cgggccgggcttctacaacgacgtggtcagctccaagccgtgcaagccctgcacgtggtgtaacctcagaagtgggagtgag cggaagcagctgtgcacggccacacaggacacagtctgccgctgccgggcgggcacccagcccctggacagctacaagc ctggagttgactgtgcccccctgccctccagggcacttctccccaggcgacaaccaggcctgcaagccctggaccaactgcac cttggctgggaagcacaccctgcagccggccagcaatagctcggacgcaatctgtgaggacagggacccccagccacgc agccccaggagacccagggccccccggccaggcccatcactgtccagcccactgaagcctggcccagaacctcacaggga ccctccacccggcccgtggaggtccccggggggccgtgcggttgccgccatcctgggcctgggcctggtgctggggctgctg ggccccctggccatcctgctggccctgtacctgctccggagggaccagaggctgcccccccgatgcccacaagccccctggg ggaggcagtttccggacccccatccaagaggagcaggccgacgcccactccaccctggccaagatctgacctgggcccacc aaggtggacgctgggccccgccaggctggagcccggagggtctgctgggcgagcagggcaggtgcaggccgcctgcccc gccacgctcctgggccaactctgcaccgttctaggtgccgatggctgcctccggctctctgcttacgtatgccatgcatacctcct gccccgcgggaccacaataaaaaaccttggcagacgggagtctccgaccggcaaaaaaaaaaaaaaaaa

SEQ ID NO: 28 (Human OX40L)

MERVQPLEENVGNAARPRFERNKLLLVASVIQGLGLLLCFTYICLHFSALQVSH
RYPRIQSIKVQFTEYKKEKGFILTSQKEDEIMKVQNNSVIINCDGFYLISLKGYFS
QEVNISLHYQKDEEPLFQLKKVRSVNSLMVASLTYKDKVYLNVTTDNTSLDDF
HVNGGELILIHQNPGEFCVL

SEQ ID NO: 29 (Human GITRL)

Catgacattgcatccttcacccatcacttgtgaattttttgttttccacagctctcatttctccaaaaatgtgtttgagccacttggaaaatatgcct ttaagccattcaagaactcaaggagctcagagatcatcctggaagctgtggctcttttgctcaatagttatgttgctatttctttgctccttcagttg gctaatctttattttctccaattagagactgctaaggagccctgtatggctaagtttggaccattaccctcaaaatggcaaatggcatcttctgaa cctccttgcgtgaataaggtgtctgactggaagctggagatacttcagaatggcttatatttaatttatggccaagtggctcccaatgcaaacta caatgatgtagctccttttgaggtgcggctgtataaaaacaaagacatgatacaaactctaacaaacaaatctaaaatccaaatgtaggagg gacttatgaattgcatgttggggacaccatagacttgatattcaactctgagcatcaggttctaaaaaataatacatactggggtatcattttacta gcaaatccccaattcatctcctagagacttgatttgatctcctcattcccttcagcacatgtagaggtgccagtgggtggattggagggagaag atattcaatttctagagtttgtctgtctacaaaaaatcaacacaaacagaactcctctgcacgtgaattttcatctat

SEQ ID NO: 30 (Human GITRL)

MCLSHLENMPLSHSRTQGAQRSSWKLWLFCSIVMLLFLCSFSWLIFIFLQLETAK
EPCMAKFGPLPSKWQMASSEPPCVNKVSDWKLEILQNGLYLIYGQVAPNANYN

DVAPFEVRLYKNKDMIQTLTNKSKIQNVGGTYELHVGDTIDLIFNSEHQVLKNN
TYWGIILLANPQFIS

SEQ ID NO: 31 (human EDA-A1)

ATTCCCTCGGCGGGCCGAGCCTCCCCTCTCTCCCGCCCCTCCTCCTCCCTTTC
CCACCCCTCGGAGTAGAGCTGCACATGCGGCTGCTCCCTGCTCCGTCCCGCC
CAGCCACTGTCGCGCAGGAACGGGTCCCTGCAGCCCCCAGCCGATGGCAGG
ACAGTAGCCGCCTGTCAGAGGTCGTGAACGGCTGAGGCAGACGCAGCGGCT
CCCGGGCCTCAAGAGAGTGGGTGTCTCCGGAGGCCATGGGCTACCCGGAGG
TGGAGCGCAGGGAACTCCTGCCTGCAGCAGCGCCGCGGGAGCGAGGGAGCC
AGGGCTGCGGGTGTGGCGGGGCCCCTGCCCGGGCGGGCGAAGGGAACAGCT
GCCTGCTCTTCCTGGGTTTCTTTGGCCTCTCGCTGGCCCTCCACCTGCTGACG
TTGTGCTGCTACCTAGAGTTGCGCTCGGAGTTGCGGCGGGAACGTGGAGCCG
AGTCCCGCCTTGGCGGCTCGGGCACCCCTGGCACCTCTGGCACCCTAAGCAG
CCTCGGTGGCCTCGACCCTGACAGCCCCATCACCAGTCACCTTGGGCAGCCG
TCACCTAAGCAGCAGCCATTGGAACCGGGAGAAGCCGCACTCCACTCTGACT
CCCAGGACGGGCACCAGATGGCCCTATTGAATTTCTTCTTCCCTGATGAAAA
GCCATACTCTGAAGAAGAAAGTAGGCGTGTTCGCCGCAATAAAAGAAGCAA
AAGCAATGAAGGAGCAGATGGCCCAGTTAAAAACAAGAAAAAGGGAAAGA
AAGCAGGACCTCCTGGACCCAATGGCCCTCCAGGACCCCCAGGACCTCCAG
GACCCCAGGGACCCCCAGGAATTCCAGGGATTCCTGGAATTCCAGGAACAA
CTGTTATGGGACCACCTGGTCCTCCAGGTCCTCCTGGTCCTCAAGGACCCCCT
GGCCTCCAGGGACCTTCTGGTGCTGCTGATAAAGCTGGAACTCGAGAAAACC
AGCCAGCTGTGGTGCATCTACAGGGCCAAGGGTCAGCAATTCAAGTCAAGA
ATGATCTTTCAGGTGGAGTGCTCAATGACTGGTCTCGCATCACTATGAACCC
CAAGGTGTTTAAGCTACATCCCCGCAGCGGGGAGCTGGAGGTACTGGTGGA
CGGCACCTACTTCATCTATAGTCAGGTAGAAGTATACTACATCAACTTCACT
GACTTTGCCAGCTATGAGGTGGTGGTGGATGAGAAGCCCTTCCTGCAGTGCA
CACGCAGCATCGAGACGGGCAAGACCAACTACAACACTTGCTATACCGCAG
GCGTCTGCCTCCTCAAGGCCCGGCAGAAGATCGCCGTCAAGATGGTGCACGC
TGACATCTCCATCAACATGAGCAAGCACCACGTTCTTTGGGGCCATCAGG
CTGGGTGAAGCCCCTGCATCCTAGATTCCCCCCATTTTGCCTCTGTCCGTGCC
CCTTCCCTGGGTTTGGGAGCCAGGACTCCCAGAACCTCTAAGTGCTGCTGTG
GAGTGAGGTGTATTGGTGTTGCAGCCGCAGAGAAATGCCCCAGTGTTATTTA

TTCCCCAGTGACTCCAGGGTGACAAGGCCTGCTTGACTTTCCAGAATGACCT
TGAGTTAACAGGACAGTTGATGGAGCCCCAGGGTTTACATGAAGCAGAACC
TTCTTTGGTTCCATGTTGACTGACTTATGGCATGACTCTTCAACCCCGAGGTC
CCTGTTGTCAGATCTATTGTTTGTTGCACTAAAATGAGGATCCAGGGCAGCA
GGCCAGAGAAAGCAAAGGTGCACTCCAGACTCTGGGGGTGGACATCTGACC
CCAAGGGGGCTGCTGCTCCTCTCTTGGGTAGGGTAGTGGCTGGGGTGGAGTG
GGAAGGGAGCATTGCAGCCTAAGAAGAAGGCCAGAGAGGGAAAAGGCAGG
TGCTTTTGGCAGAGACCATAAGAGAAACCTGCCAAGGAGCATCCTTGGCAGT
GGGAATGTTCTTTCTGCTCTATACTGTGGCCTGCAGGAGGGTTGGAGTGCTC
TTCCCACTCCAGCTGACAGCCACACCGTGGCAGCTTGCTGGGCTTTGGGAAG
TTTGCTGTGCTTTGGAACAATCACAGGGAATGGCCACAAACCTGCCCGCCTA
AGACCCTGAATCCGTACTTGGGTCACATGACTCTCATTTTATTTACAGCTGTG
CTCCACACTCAGAAAATTCCCTGGGGTCACCTTCTAGTTGCCCCCATTCCCAG
CCTGACTAGAACTCCTGTCTTCTTTCTCCATGGAGCCTACCTCTGTCTGAGAC
AGGTGCCTAACCTGGGACCTGTGGTCATGTGAGTCTGGGATATTCTTTAGCT
TACCTGGGCACAAACAGAATTTTCCATTTATTAAGCAGTACAAATGTTTTTC
ATCCATTCCTAATCAAATTCTGTCTGGGGACGAAGGGTTGGACGGGATGACC
TCCAGAAGTCCCTTCAATTTCTAGTACCTGTGACTCTTAGCCCTCACCACAGC
CTTCTAAATTCCCAAATCCTAGACTGCTCCTGGGCATTAGCAAGGCAGAGCC
TTTTTACCTGGCCTAGAAAGGGCAAGGGGTGAGGATAGGACAGAGGGATTT
TGTTCAAGTTTGCTGCAACCCAAGTGGACGTTAGGCCAGGCCTTATCTGAAA
GGCCAGCAGCTGATGCTGTACTAACCCAGTCTTTCTTCACTCTGGCTTCAAA
AAGCCACAGCAGAGCATTGTCACCGCAGGTGCTCATGCTGCTCCCCTAAAGC
CAGGCTCAGGAGAAGCCAGTGTCTAGGCACTGAGCAGGGATCTGCCCCCTA
GTTCAGGTCCAAATTCACCTTCCCCTAAACCCCAAGCTTCCCAACAGATCAT
ATGGTAGGACCCTCGAGAGCCTTACTTCAAAGTGCCTGGGCTCAGCCTGGTT
TCTGGGTGCTAGATCCAGCCCAAACCTGGGAAGGCCAGCCTTGTACAGTCTG
CTCCTCTTGTTCCTGAAATGTGTTTCCTTTTCAGGAGATGGGGAATAATTTCC
TTCAGGCAGCTGAAATTCACCAAGAACAGCGGGTACTTATTTCTCAGCTGTG
CCTTCCCTTTCTAAGCAACCACACTGCTTGGCCCTTCAAGGGTCAGGGTGAG
ACGTGATGGGCTAGGCCTCCGTTGTCTGGTTGCTAATGACAGCCTTGCAACC
CAAGGTGAGGTGAACTCCAGGCATGTGTCTGGCCCTAACTCCTATAAAGTGC
CTCGGACAGTCCGCAGTTGTAGCAGAAACCAACAAGAACCACTCCTTCATGT

TTGGAAAATAATTTCTCTTGTATTATCTCCTTTGAAGAAGGCAAGGCTGATA

ATATGACAAACATCATTGTTTAGATGAGGCTCAGAGAGGTAGCACTCTCAGA

GTGTTTTGACCAGTTTAAGCCGCAGACCTGGAGCTTCAGCCAGGTCTGACTC

CAAAGCTGTTCCATTACACCACAGCATTGTGTGGAATTTGAGGTCTAGAGAG

AACCAATAAAAGTGGTAATTGGGAACTGAAATCCTTGAGAGTTCCGGGGAG

AAACCCAGAGATGCCTGATTTCATTCCTCGATGGTAATACCCGTCCTCTCGG

CTGCCAGGGGCTCTGTGGCAAAAGAGTCAGACATTTCTTTGGAAAACAGC

GAACAGCCTTAGAGCTCTTGTGTTCAGAAGAATCTTCCTGGCACAATGTTGG

AGCAGCAGGCCTCTGGGACCCACAGAACTTGTGGCCTTTATGTTCTTTCACC

CATCCTAGGAACCAGCCAACCATCATGTGTAGAGCCCCTACTGTGGGCAAAG

TCCTCCTTTCATTACCCTACAGACAGCTTACAGGAGCCAGCCTGCTTCCCACA

ACTACTAGTGTGACTCCTTATCTCTTTCCACCATACCTTAGAGACTTTGATAC

TACCAGGGTCTCTCAGGGATGGAGGGAAGACCTGAAAGAGAGGACTGGTTC

TGAGGCCAGAAAGGTGTGAGGAGAGAGGAGGAAAAGTCTTCCTAATTGTGC

CCCTAAAGAGCATCCTGATACCATTCTATTCTCCAGACATGGAGGGGATGAT

AAAGGAAATAGGATCTCCACTGGACCCTTGATTCATTCTGAACCCTCCAAAG

GAACTCTAGAGGGCGAGGGATGATGAGGGAAGCAATAGGTAGCTGGGGAG

CCCTATTGCTGCTAAGTCATTGGCAAAGTGACAAAGCAATTTACTGATGAGA

GAATGTGGAAATAGATGTGCAGTTTGGAATTATGTTGGTGTGAATTTGCCAG

AGGACCAATGCTTGCATGGAGAATGGGACGAGGACATTTGTGGGCAAGCAG

ATGACAGAGGTTTGAAGGAGAATGGCATGGCAGGAGTCTCTGCCAGTTACTT

GGGCTTCAACAGCCAAGCTGGCACAAAAGACAGCTGGCGGAGGCTGCTCGG

CTACTGGTTACCTGGAGAAGTAGTATTTGCCTATTCCCCCTTCATCCATCCT

GAGCCAAATTTCTTTTGCTGAACAGGAAAGAGCTAGGAACCCTGGAGGTAA

ACAAAGACTTTGATCCATGTATGAGTGTATGTGTTTATGTAACTTCCTGTGGA

TGCAAATAGATTCAGAGAAATTTAGAGCTAAAAAGGCCCTTAGAGGGAATC

TAGCCCAACCTACATTCCACCCTGTTACTTATGTAGAAACTGAGGCCCAGAG

AGGGAAGATGACCTGCCCCAAGTGGTGAGCAAGCACCAACCTCCAGACTCA

GCAGAGTGAGGGGGTAAAGCAGTTCCTGTCCCACATGGCCATCTTCTTTCTT

CCACCCACAAACTCCAGGCTGGAAGTACTTGGCCCCCTTCAGGAGCCTGGCC

AGGCAGGGAGAGAGTAGCTGCAGCCTTCATCAGAACTCTTCCTCCTCCCAAG

GCATTCTCCCAGCTCTAGCCTCTGGACTGGAAAGCACAAGACTGGCCCAGTG

CCAGCAAGTCCTTAGGCTACTGTAATGCTGCCTCAGGACCCATCCCTGCCTG

GAGGCTCCTCTAGGCCCTGTGAGCACAAAGAAGAAAGCTGATTTTTGTCTTT
TAATCCATTTCAGGACTCTCTCCAGGAGGGCTCGGGGTGTGTCATTTCTATAT
TCCTCCAGCTGGGATTGGGGGGTGGGCTTTGTTGTGAGAATGGCCTGGAGCA
GGCCCAATGCTGCTTTTGGGGGTCAGCATCCAGTGTGAGATACTGTGTATAT
AAACTATATATAATGTATATAAACTGGGATGTAAGTTTGTGTAAATTAATGG
TTTATTCTTTGCAAATAAAACGCTTTCCCCGTCTGTTCTTGAAAAAAAAAAA
AAAAAA

SEQ ID NO: 32 (human EDA-A1)

GSHMGPSGAADKAGTRENQPAVVHLQGQGSAIQVKNDLSGGVLNDWSRITMN
PKVFKLHPRSGELEVLVDGTYFIYSQVEVYYINFTDFASYEVVVDEKPFLQCTRS
IETGKTNYNTCYTAGVCLLKARQKIAVKMVHADISINMSKHTTFFGAIRLGEAP
AS

SEQ ID NO: 33 (human EDA-A2)

MGYPEVERRELLPAAAPRERGSQGCGCGGAPARAGEGNSCLLFLGFFGLSLALH
LLTLCCYLELRSELRRERGAESRLGGSGTPGTSGTLSSLGGLDPDSPITSHLGQPS
PKQQPLEPGEAALHSDSQDGHQMALLNFFFPDEKPYSEEESRRVRRNKRSKSNE
GADGPVKNKKKGKKAGPPGPNGPPGPPGPPGPQGPPGIPGIPGIPGTTVMGPPGP
PGPPGPQGPPGLQGPSGAADKAGTRENQPAVVHLQGQGSAIQVKNDLSGGVLN
DWSRITMNPKVFKLHPRSGELEVLVDGTYFIYSQVYYINFTDFASYEVVVDEKP
FLQCTRSIETGKTNYNTCYTAGVCLLKARQKIAVKMVHADISINMSKHTTFFGAI
RLGEAPAS

SEQ ID NO: 34 (human EDA-A2)

ATTCCCTCGGCGGGCCGAGCCTCCCCTCTCTCCCGCCCCTCCTCCTCCCTTTC
CCACCCCTCGGAGTAGAGCTGCACATGCGGCTGCTCCCTGCTCCGTCCCGCC
CAGCCACTGTCGCGCAGGAACGGGTCCCTGCAGCCCCCAGCCGATGGCAGG
ACAGTAGCCGCCTGTCAGAGGTCGTGAACGGCTGAGGCAGACGCAGCGGCT
CCCGGGCCTCAAGAGAGTGGGTGTCTCCGGAGGCCATGGGCTACCCGGAGG
TGGAGCGCAGGGAACTCCTGCCTGCAGCAGCGCCGCGGGAGCGAGGGAGCC
AGGGCTGCGGGTGTGGCGGGGCCCCTGCCCGGGCGGGCGAAGGGAACAGCT
GCCTGCTCTTCCTGGGTTTCTTTGGCCTCTCGCTGGCCCTCCACCTGCTGACG
TTGTGCTGCTACCTAGAGTTGCGCTCGGAGTTGCGGCGGGAACGTGGAGCCG
AGTCCCGCCTTGGCGGCTCGGGCACCCCTGGCACCTCTGGCACCCTAAGCAG

CCTCGGTGGCCTCGACCCTGACAGCCCCATCACCAGTCACCTTGGGCAGCCG

TCACCTAAGCAGCAGCCATTGGAACCGGGAGAAGCCGCACTCCACTCTGACT

CCCAGGACGGGCACCAGATGGCCCTATTGAATTTCTTCTTCCCTGATGAAAA

GCCATACTCTGAAGAAGAAAGTAGGCGTGTTCGCCGCAATAAAAGAAGCAA

AAGCAATGAAGGAGCAGATGGCCCAGTTAAAAACAAGAAAAAGGGAAAGA

AAGCAGGACCTCCTGGACCCAATGGCCCTCCAGGACCCCCAGGACCTCCAG

GACCCCAGGGACCCCCAGGAATTCCAGGGATTCCTGGAATTCCAGGAACAA

CTGTTATGGGACCACCTGGTCCTCCAGGTCCTCCTGGTCCTCAAGGACCCCCT

GGCCTCCAGGGACCTTCTGGTGCTGCTGATAAAGCTGGAACTCGAGAAAACC

AGCCAGCTGTGGTGCATCTACAGGGCCAAGGGTCAGCAATTCAAGTCAAGA

ATGATCTTTCAGGTGGAGTGCTCAATGACTGGTCTCGCATCACTATGAACCC

CAAGGTGTTTAAGCTACATCCCCGCAGCGGGGAGCTGGAGGTACTGGTGGA

CGGCACCTACTTCATCTATAGTCAGGTATACTACATCAACTTCACTGACTTTG

CCAGCTATGAGGTGGTGGTGGATGAGAAGCCCTTCCTGCAGTGCACACGCA

GCATCGAGACGGGCAAGACCAACTACAACACTTGCTATACCGCAGGCGTCT

GCCTCCTCAAGGCCCGGCAGAAGATCGCCGTCAAGATGGTGCACGCTGACA

TCTCCATCAACATGAGCAAGCACACCACGTTCTTTGGGGCCATCAGGCTGGG

TGAAGCCCCTGCATCCTAGATTCCCCCCATTTTGCCTCTGTCCGTGCCCCTTC

CCTGGGTTTGGGAGCCAGGACTCCCAGAACCTCTAAGTGCTGCTGTGGAGTG

AGGTGTATTGGTGTTGCAGCCGCAGAGAAATGCCCCAGTGTTATTTATTCCC

CAGTGACTCCAGGGTGACAAGGCCTGCTTGACTTTCCAGAATGACCTTGAGT

TAACAGGACAGTTGATGGAGCCCCAGGGTTTACATGAAGCAGAACCTTCTTT

GGTTCCATGTTGACTGACTTATGGCATGACTCTTCAACCCCGAGGTCCCTGTT

GTCAGATCTATTGTTTGTTGCACTAAAATGAGGATCCAGGGCAGCAGGCCAG

AGAAAGCAAAGGTGCACTCCAGACTCTGGGGGTGGACATCTGACCCCAAGG

GGGCTGCTGCTCCTCTCTTGGGTAGGGTAGTGGCTGGGGTGGAGTGGGAAGG

GAGCATTGCAGCCTAAGAAGAAGGCCAGAGAGGGAAAAGGCAGGTGCTTTT

GGCAGAGACCATAAGAGAAACCTGCCAAGGAGCATCCTTGGCAGTGGGAAT

GTTCTTTCTGCTCTATACTGTGGCCTGCAGGAGGGTTGGAGTGCTCTTCCCAC

TCCAGCTGACAGCCACACCGTGGCAGCTTGCTGGGCTTTGGGAAGTTTGCTG

TGCTTTGGAACAATCACAGGGAATGGCCACAAACCTGCCCGCCTAAGACCCT

GAATCCGTACTTGGGTCACATGACTCTCATTTTATTTACAGCTGTGCTCCACA

CTCAGAAAATTCCCTGGGGTCACCTTCTAGTTGCCCCCATTCCCAGCCTGACT

AGAACTCCTGTCTTCTTTCTCCATGGAGCCTACCTCTGTCTGAGACAGGTGCC
TAACCTGGGACCTGTGGTCATGTGAGTCTGGGATATTCTTTAGCTTACCTGG
GCACAAACAGAATTTTCCATTTATTAAGCAGTACAAATGTTTTTCATCCATTC
CTAATCAAATTCTGTCTGGGGACGAAGGGTTGGACGGGATGACCTCCAGAA
GTCCCTTCAATTTCTAGTACCTGTGACTCTTAGCCCTCACCACAGCCTTCTAA
ATTCCCAAATCCTAGACTGCTCCTGGGCATTAGCAAGGCAGAGCCTTTTTAC
CTGGCCTAGAAAGGGCAAGGGGTGAGGATAGGACAGAGGGATTTTGTTCAA
GTTTGCTGCAACCCAAGTGGACGTTAGGCCAGGCCTTATCTGAAAGGCCAGC
AGCTGATGCTGTACTAACCCAGTCTTTCTTCACTCTGGCTTCAAAAAGCCAC
AGCAGAGCATTGTCACCGCAGGTGCTCATGCTGCTCCCCTAAAGCCAGGCTC
AGGAGAAGCCAGTGTCTAGGCACTGAGCAGGGATCTGCCCCCTAGTTCAGG
TCCAAATTCACCTTCCCCTAAACCCCAAGCTTCCCAACAGATCATATGGTAG
GACCCTCGAGAGCCTTACTTCAAAGTGCCTGGGCTCAGCCTGGTTTCTGGGT
GCTAGATCCAGCCCAAACCTGGGAAGGCCAGCCTTGTACAGTCTGCTCCTCT
TGTTCCTGAAATGTGTTTCCTTTTCAGGAGATGGGGAATAATTTCCTTCAGGC
AGCTGAAATTCACCAAGAACAGCGGGTACTTATTTCTCAGCTGTGCCTTCCC
TTTCTAAGCAACCACACTGCTTGGCCCTTCAAGGGTCAGGGTGAGACGTGAT
GGGCTAGGCCTCCGTTGTCTGGTTGCTAATGACAGCCTTGCAACCCAAGGTG
AGGTGAACTCCAGGCATGTGTCTGGCCCTAACTCCTATAAAGTGCCTCGGAC
AGTCCGCAGTTGTAGCAGAAACCAACAAGAACCACTCCTTCATGTTTGGAAA
ATAATTTCTCTTGTATTATCTCCTTTGAAGAAGGCAAGGCTGATAATATGAC
AAACATCATTGTTTAGATGAGGCTCAGAGAGGTAGCACTCTCAGAGTGTTTT
GACCAGTTTAAGCCGCAGACCTGGAGCTTCAGCCAGGTCTGACTCCAAAGCT
GTTCCATTACACCACAGCATTGTGTGGAATTTGAGGTCTAGAGAGAACCAAT
AAAAGTGGTAATTGGGAACTGAAATCCTTGAGAGTTCCGGGGAGAAACCCA
GAGATGCCTGATTTCATTCCTCGATGGTAATACCCGTCCTCTCGGCTGCCAG
GGGCTCTGTGGCAAAAGAGTCAGACATTTCTTTGGAAAACAGCGAACAGC
CTTAGAGCTCTTGTGTTCAGAAGAATCTTCCTGGCACAATGTTGGAGCAGCA
GGCCTCTGGGACCCACAGAACTTGTGGCCTTTATGTTCTTTCACCCATCCTAG
GAACCAGCCAACCATCATGTGTAGAGCCCCTACTGTGGGCAAAGTCCTCCTT
TCATTACCCTACAGACAGCTTACAGGAGCCAGCCTGCTTCCCACAACTACTA
GTGTGACTCCTTATCTCTTTCCACCATACCTTAGAGACTTTGATACTACCAGG
GTCTCTCAGGGATGGAGGGAAGACCTGAAAGAGAGGACTGGTTCTGAGGCC

AGAAAGGTGTGAGGAGAGAGGAGGAAAAGTCTTCCTAATTGTGCCCCTAAA
GAGCATCCTGATACCATTCTATTCTCCAGACATGGAGGGGATGATAAAGGAA
ATAGGATCTCCACTGGACCCTTGATTCATTCTGAACCCTCCAAAGGAACTCT
AGAGGGCGAGGGATGATGAGGGAAGCAATAGGTAGCTGGGGAGCCCTATTG
CTGCTAAGTCATTGGCAAAGTGACAAAGCAATTTACTGATGAGAATGTG
GAAATAGATGTGCAGTTTGGAATTATGTTGGTGTGAATTTGCCAGAGGACCA
ATGCTTGCATGGAGAATGGGACGAGGACATTTGTGGGCAAGCAGATGACAG
AGGTTTGAAGGAGAATGGCATGGCAGGAGTCTCTGCCAGTTACTTGGGCTTC
AACAGCCAAGCTGGCACAAAAGACAGCTGGCGGAGGCTGCTCGGCTACTGG
TTACCTGGAGAAGTAGTATTTGCCTATTTCCCCCTTCATCCATCCTGAGCCAA
ATTTCTTTTGCTGAACAGGAAAGAGCTAGGAACCCTGGAGGTAAACAAAGA
CTTTGATCCATGTATGAGTGTATGTGTTTATGTAACTTCCTGTGGATGCAAAT
AGATTCAGAGAAATTTAGAGCTAAAAAGGCCCTTAGAGGGAATCTAGCCCA
ACCTACATTCCACCCTGTTACTTATGTAGAAACTGAGGCCCAGAGAGGGAAG
ATGACCTGCCCCAAGTGGTGAGCAAGCACCAACCTCCAGACTCAGCAGAGT
GAGGGGGTAAAGCAGTTCCTGTCCCACATGGCCATCTTCTTTCTTCCACCCA
CAAACTCCAGGCTGGAAGTACTTGGCCCCCTTCAGGAGCCTGGCCAGGCAG
GGAGAGAGTAGCTGCAGCCTTCATCAGAACTCTTCCTCCTCCCAAGGCATTC
TCCCAGCTCTAGCCTCTGGACTGGAAAGCACAAGACTGGCCCAGTGCCAGCA
AGTCCTTAGGCTACTGTAATGCTGCCTCAGGACCCATCCCTGCCTGGAGGCT
CCTCTAGGCCCTGTGAGCACAAAGAAGAAAGCTGATTTTTGTCTTTTAATCC
ATTTCAGGACTCTCTCCAGGAGGGCTCGGGGTGTGTCATTTCTATATTCCTCC
AGCTGGGATTGGGGGGTGGGCTTTGTTGTGAGAATGGCCTGGAGCAGGCCC
AATGCTGCTTTTGGGGGTCAGCATCCAGTGTGAGATACTGTGTATATAAACT
ATATATAATGTATATAAACTGGGATGTAAGTTTGTGTAAATTAATGGTTTATT
CTTTGCAAATAAAACGCTTTCCCCGTCTGTTCTTGAAAAAAAAAAAAAAAAA
A

SEQ ID NO: 35 (murine RANKL variant D1 monomer A)

MRRASRDYGKYLRSSEEMGSGPGVPHEGPLHPAPSAPAPAPPPAASRSMFLALL
GLGLGQVVCSIALFLYFRAQMDPNRISEDSTHCFYRILRLHENADLQDSTLESED
TLPDSCRRMKQAFQGAVQKELQHIVGPQRFSGAPAMMEGSWLDVAQRGKPEA
QPFAHLTINAASIPSGSHKVTLSSWYHDRGWADISNMTLSNGKLRVNQDGFYYL

YANISFRHHETSGSVPTDYLQLMVYVVKTSIKIPSSHNLMKGGSTKNWSGNSEF

HYYSINVGGFFKLRAGEEISIQVSNPSLLDPDQDATYFGAFKVQDID

SEQ ID NO: 36 (murine RANKL variant D1 monomer B)

MRRASRDYGKYLRSSEEMGSGPGVPHEGPLHPAPSAPAPAPPPAASRSMFLALL

GLGLGQVVCSIALFLYFRAQMDPNRISEDSTHCFYRILRLHENADLQDSTLESED

TLPDSCRRMKQAFQGAVQKELQHIVGPQRFSGAPAMMEGSWLDVAQRGKPEA

QPFAHLTINAASIPSGSHKVTLSSWYHDRGWAKISNMTLSNGKLRVNQDGYYY

LYANICFRHHETSGSVPTDYLQLMVYVVKTSIKIPSSHNLMDGGSTKNWSGNSE

FHFYSINVGGYFKLRAGEEISIQVSNPSLLDPDQDATYFGAFKVQDID

SEQ ID NO: 37 (murine RANKL variant D2 monomer A)

MRRASRDYGKYLRSSEEMGSGPGVPHEGPLHPAPSAPAPAPPPAASRSMFLALL

GLGLGQVVCSIALFLYFRAQMDPNRISEDSTHCFYRILRLHENADLQDSTLESED

TLPDSCRRMKQAFQGAVQKELQHIVGPQRFSGAPAMMEGSWLDVAQRGKPEA

QPFAHLVINAASIPSGSHKVTLSSWYHDRGWAKISNMTLSNGKLRVNQDGFYY

LYANICFRHHETSGKVPTDYLQLMVYVVKTSIKIPSSHNLMKGGSTKNWSGNSE

FHYYSINVGGFFKLRAGEEISIQVSNPSLLDPDQDATYFGAFKVQDID

SEQ ID NO: 38 (murine RANKL variant D2 monomer B)

MRRASRDYGKYLRSSEEMGSGPGVPHEGPLHPAPSAPAPAPPPAASRSMFLALL

GLGLGQVVCSIALFLYFRAQMDPNRISEDSTHCFYRILRLHENADLQDSTLESED

TLPDSCRRMKQAFQGAVQKELQHIVGPQRFSGAPAMMEGSWLDVAQRGKPEA

QPFAHLVINAASIPSGSHKVTLSSWYHDRGWAKISNMTLSNGKLRVNQDGYYY

LYANIAFRHHETSGKVPTDYLQLMVYVVKTSIKIPSSHNLMDGGSTKNWSGNSE

FHFYSINVGGYFKLRAGEEISIQVSNPSLLDPDQDATYFGAFKVQDID

SEQ ID NO: 39 (human RANKL variant D1 monomer A)

MRRASRDYTKYLRGSEEMGGGPGAPHEGPLHAPPPPAPHQPPAASRSMFVALL

GLGLGQVVCSVALFFYFRAQMDPNRISEDGTHCIYRILRLHENADFQDTTLESQ

DTKLIPDSCRRIKQAFQGAVQKELQHIVGSQHIRAEKAMVDGSWLDLAKRSKLE

AQPFAHLTINATDIPSGSHKVSLSSWYHDRGWARISNMTFSNGKLIVNQDGFYY

LYANISFRHHETSGDLATEYLQLMVYVTKTSIKIPSSHTLMKGGSTKYWSGNSEF

HYYSINVGGFFKLRSGEEISIEVSNPSLLDPDQDATYFGAFKVRDID

SEQ ID NO: 40 (human RANKL variant D1 monomer B)

MRRASRDYTKYLRGSEEMGGGPGAPHEGPLHAPPPPAPHQPPAASRSMFVALL
GLGLGQVVCSVALFFYFRAQMDPNRISEDGTHCIYRILRLHENADFQDTTLESQ
DTKLIPDSCRRIKQAFQGAVQKELQHIVGSQHIRAEKAMVDGSWLDLAKRSKLE
AQPFAHLTINATDIPSGSHKVSLSSWYHDRGWAKISNMTFSNGKLIVNQDGYYY
LYANICFRHHETSGDLATEYLQLMVYVTKTSIKIPSSHTLMDGGSTKYWSGNSE
FHFYSINVGGYFKLRSGEEISIEVSNPSLLDPDQDATYFGAFKVRDID

SEQ ID NO: 41 (human RANKL variant D2 monomer A)

MRRASRDYTKYLRGSEEMGGGPGAPHEGPLHAPPPPAPHQPPAASRSMFVALL
GLGLGQVVCSVALFFYFRAQMDPNRISEDGTHCIYRILRLHENADFQDTTLESQ
DTKLIPDSCRRIKQAFQGAVQKELQHIVGSQHIRAEKAMVDGSWLDLAKRSKLE
AQPFAHLVINATDIPSGSHKVSLSSWYHDRGWAKISNMTFSNGKLIVNQDGFYY
LYANICFRHHETSGKLATEYLQLMVYVTKTSIKIPSSHTLMKGGSTKYWSGNSE
FHYYSINVGGFFKLRSGEEISIEVSNPSLLDPDQDATYFGAFKVRDID

SEQ ID NO: 42 (human RANKL variant D2 monomer B)

MRRASRDYTKYLRGSEEMGGGPGAPHEGPLHAPPPPAPHQPPAASRSMFVALL
GLGLGQVVCSVALFFYFRAQMDPNRISEDGTHCIYRILRLHENADFQDTTLESQ
DTKLIPDSCRRIKQAFQGAVQKELQHIVGSQHIRAEKAMVDGSWLDLAKRSKLE
AQPFAHLVINATDIPSGSHKVSLSSWYHDRGWAKISNMTFSNGKLIVNQDGYYY
LYANIAFRHHETSGKLATEYLQLMVYVTKTSIKIPSSHTLMDGGSTKYWSGNSE
FHFYSINVGGYFKLRSGEEISIEVSNPSLLDPDQDATYFGAFKVRDID

SEQ ID NO: 43 (human trail variant D1 monomer A)

MAMMEVQGGPSLGQTCVLIVIFTVLLQSLCVAVTYVYFTNELKQMQDKYSKSG
IACFLKEDDSYWDPNDEESMNSPCWQVKWQLRQLVRKMILRTSEETISTVQEK
QQNISPLVRERGPQRVAAHITGTRGRSNTLSSPNSKNEKALGRKINSWESSRSGH
SDLSNLHLRNGELVIHEKGFYYIYSQTSFRFQEEIKENTKNDKQMVQYIYKYTSY
PDPILLMKSARNSCWSKDAEYGYYSIYQGGIFELKENDRIFVSVTNEHLIDMDHE
ASFFGAFLVG

SEQ ID NO: 44 (human trail variant D1 monomer B)

MAMMEVQGGPSLGQTCVLIVIFTVLLQSLCVAVTYVYFTNELKQMQDKYSKSG
IACFLKEDDSYWDPNDEESMNSPCWQVKWQLRQLVRKMILRTSEETISTVQEK
QQNISPLVRERGPQRVAAHITGTRGRSNTLSSPNSKNEKALGRKINSWESSRSGH

SFLSNLHLRNGELVIHEKGYYYIYSQTYFRFQEEIKENTKNDKQMVQYIYKYTS
YPDPILLMDSARNSCWSKDAEYGLYSIYQGGYFELKENDRIFVSVTNEHLIDMD
HEASFFGAFLVG

SEQ ID NO: 45 (human trail variant D2 monomer A)

MAMMEVQGGPSLGQTCVLIVIFTVLLQSLCVAVTYVYFTNELKQMQDKYSKSG
IACFLKEDDSYWDPNDEESMNSPCWQVKWQLRQLVRKMILRTSEETISTVQEK
QQNISPLVRERGPQRVAAHIVGTRGRSNTLSSPNSKNEKALGRKINSWESSRSGH
SFLSNLHLRNGELVIHEKGFYYIYSQTYFRFQEEIKENTKNDKQMVQYIYKYTSY
PDPILLMKSARNSCWSKDAEYGYYSIYQGGIFELKENDRIFVSVTNEHLIDMDHE
ASFFGAFLVG

SEQ ID NO: 46 (human trail variant D2 monomer B)

MAMMEVQGGPSLGQTCVLIVIFTVLLQSLCVAVTYVYFTNELKQMQDKYSKSG
IACFLKEDDSYWDPNDEESMNSPCWQVKWQLRQLVRKMILRTSEETISTVQEK
QQNISPLVRERGPQRVAAHITGVRGRSNTLSSPNSKNEKALGRKINSWESSRSGH
SFLSNLHLRNGELVIHEKGYYYIYSQTAFRFQEEIKENTKNDKQMVQYIYKYTS
YPDPILLMDSARNSCWSKDAEYGLYSIYQGGYFELKENDRIFVSVTNEHLIDMD
HEASFFGAFLVG

SEQ ID NO: 47 (human BAFF variant D1 monomer A)

MDDSTEREQSRLTSCLKKREEMKLKECVSILPRKESPSVRSSKDGKLLAATLLLA
LLSCCLTVVSFYQVAALQGDLASLRAELQGHHAEKLPAGAGAPKAGLEEAPAV
TAGLKIFEPPAPGEGNSSQNSRNKRAVQGPEETVTQDCLQLIADSETPTIQKGSY
TFVPWLLSYKRGSALEEKENKILVKETGYFFIYGQVSYTDKTYAMGHLIQRKKV
HVFGDELSLVTLFRCIQNMPETLPYNSCYSAGIAKLEEGDELQLAIPRENAQISLD
GDVTFFGALKLL

SEQ ID NO: 48 (human BAFF variant D1 monomer B)

MDDSTEREQSRLTSCLKKREEMKLKECVSILPRKESPSVRSSKDGKLLAATLLLA
LLSCCLTVVSFYQVAALQGDLASLRAELQGHHAEKLPAGAGAPKAGLEEAPAV
TAGLKIFEPPAPGEGNSSQNSRNKRAVQGPEETVTQDCLQLIADSETPTIQKGSY
TFVPWLLSFKRGSALEEKENKILVKETGYFFIYGQVLYTDKTYAMGHLIQRKKV
HVFGDELSLVTLFDCIQNMPETLPNNSCYSAGYAKLEEGDELQLAIPRENAQISL
DGDVTFFGALKLL

SEQ ID NO: 49 (human BAFF variant D2 monomer A)

MDDSTEREQSRLTSCLKKREEMKLKECVSILPRKESPSVRSSKDGKLLAATLLLA
LLSCCLTVVSFYQVAALQGDLASLRAELQGHHAEKLPAGAGAPKAGLEEAPAV
TAGLKIFEPPAPGEGNSSQNSRNKRAVQGPEETVTQDCLQLVADSETPTIQKGSY
TFVPWLLSFKRGSALEEKENKILVKETGYFFIYGQVLYTDKTYAMGHLIQRKKV
HVFGDELSLVTLFRCIQNMPETLPYNSCYSAGIAKLEEGDELQLAIPRENAQISLD
GDVTFFGALKLL

SEQ ID NO: 50 (human BAFF variant D2 monomer B)

MDDSTEREQSRLTSCLKKREEMKLKECVSILPRKESPSVRSSKDGKLLAATLLLA
LLSCCLTVVSFYQVAALQGDLASLRAELQGHHAEKLPAGAGAPKAGLEEAPAV
TAGLKIFEPPAPGEGNSSQNSRNKRAVQGPEETVTQDCLQLDADSETPTIQKGSY
TFVPWLLSFKRGSALEEKENKILVKETGYFFIYGQVAYTDKTYAMGHLIQRKKV
HVFGDELSLVTLFDCIQNMPETLPNNSCYSAGYAKLEEGDELQLAIPRENAQISL
DGDVTFFGALKLL

SEQ ID NO: 51 (Murine APRIL variant D1 monomer A)

MPASSPGHMGGSVREPALSVALWLSWGAVLGAVTCAVALLIQQTELQSLRREV
SRLQRSGGPSQKQGERPWQSLWEQSPDVLEAWKDGAKSRRRRAVLTQKHKKK
HSVLHLVPVNITSKDSDVTEVMWQPVDRRGRGLEAQGDIVRVWDTGIYLLYSQ
VSFHDVTFTMGQVVSREGQGRRETLFRCIRSMPSDPDRAYNSCYSAGVFHLHQ
GDIITVKIPRANAKLSLSPHGTFLGFVKL

SEQ ID NO: 52 (Murine APRIL variant D1 monomer B)

MPASSPGHMGGSVREPALSVALWLSWGAVLGAVTCAVALLIQQTELQSLRREV
SRLQRSGGPSQKQGERPWQSLWEQSPDVLEAWKDGAKSRRRRAVLTQKHKKK
HSVLHLVPVNITSKDSDVTEVMWQPVLRRGRGLEAQGDIVRVWDTGYYLLYSQ
VLFHDVTFTMGQVVSREGQGRRETLFDCIRSMPSDPDRAYNSCYSAGYFHLHQ
GDIITVKIPRANAKLSLSPHGTFLGFVKL

SEQ ID NO: 53 (Murine APRIL variant D2 monomer A)

MPASSPGHMGGSVREPALSVALWLSWGAVLGAVTCAVALLIQQTELQSLRREV
SRLQRSGGPSQKQGERPWQSLWEQSPDVLEAWKDGAKSRRRRAVLTQKHKKK
HSVLHLVPVNITSKDSDVTEVMWQPVLRRGRGLEAQGDIVRVWDTGIYLLYSQ

VLFHDVTFTMGQVVSREGQGRRETLFRCIRSMPSDPDRAYNSCYSAGVFHLHQ
GDIITVKIPRANAKLSLSPHGTFLGFVKL

SEQ ID NO: 54 (Murine APRIL variant D2 monomer B)

MPASSPGHMGGSVREPALSVALWLSWGAVLGAVTCAVALLIQQTELQSLRREV
SRLQRSGGPSQKQGERPWQSLWEQSPDVLEAWKDGAKSRRRRAVLTQKHKKK
HSVLHLVPVNITSKDSDVTEVMWQPVLRRGRGLEAQGDIVRVWDTGYYLLYSQ
VAFHDVTFTMGQVVSREGQGRRETLFDCIRSMPSDPDRAYNSCYSAGYFHLHQ
GDIITVKIPRANAKLSLSPHGTFLGFVKL

SEQ ID NO: 55 (TNFalpha variant D1 monomer A)

MSTESMIRDVELAEEALPKKTGGPQGSRRCLFLSLFSFLIVAGATTLFCLLHFGVI
GPQREEFPRDLSLISPLAQAVRSSSRTPSDKPVAHVVANPQAEGQLQWLNRRAN
ASLANGVELRDNQLVVPSEGLYLIYSQVSFKGQGCPSTHVLLTHTISRIAVSYQT
KVNLLSAIKSPCQRETPEGAEAKPWYEPIYLGGVFQLEKGDRLSAEINRPDYLDF
AESGQVYFGIIAL

SEQ ID NO: 56 (TNFalpha variant D1 monomer B)

MSTESMIRDVELAEEALPKKTGGPQGSRRCLFLSLFSFLIVAGATTLFCLLHFGVI
GPQREEFPRDLSLISPLAQAVRSSSRTPSDKPVAHVVANPQAEGQLQWLNRRAN
ALLANGVELRDNQLVVPSEGYYLIYSQVLFKGQGCPSTHVLLTHTIDRIAVSYQT
KVNLLSAIKSPCQRETPEGAEAKPWYEPIYLGGYFQLEKGDRLSAEINRPDYLDF
AESGQVYFGIIAL

SEQ ID NO: 57 (TNFalpha variant D2 monomer A)

MSTESMIRDVELAEEALPKKTGGPQGSRRCLFLSLFSFLIVAGATTLFCLLHFGVI
GPQREEFPRDLSLISPLAQAVRSSSRTPSDKPVAHVVANPQAEGQLQWLNRRAN
ALLANGVELRDNQLVVPSEGLYLIYSQVLFKGQGCPSTHVLLTHTISRIAVSYQT
KVNLLSAIKSPCQRETPEGAEAKPWYEPIYLGGVFQLEKGDRLSAEINRPDYLDF
AESGQVYFGIIAL

SEQ ID NO: 58 (TNFalpha variant D2 monomer B)

MSTESMIRDVELAEEALPKKTGGPQGSRRCLFLSLFSFLIVAGATTLFCLLHFGVI
GPQREEFPRDLSLISPLAQAVRSSSRTPSDKPVAHVVANPQAEGQLQWLNRRAN
ALLANGVELRDNQLVVPSEGYYLIYSQVAFKGQGCPSTHVLLTHTIDRIAVSYQ

TKVNLLSAIKSPCQRETPEGAEAKPWYEPIYLGGYFQLEKGDRLSAEINRPDYLD
FAESGQVYFGIIAL

SEQ ID NO: 59 (TNFbeta variant D1 monomer A)

MTPPERLFLPRVCGTTLHLLLLGLLLVLLPGAQGLPGVGLTPSAAQTARQHPKM
HLAHSTLKPAAHLIGDPSKQNSLLWRANTDRADLQDGFSLSNNSLLVPTSGIYF
VYSQVSFSGKAYSPKATSSPLYLAHEVQLFSSQYPFHVPLLSSQKMVYPGLQEP
WYHSMYHGAAFQLTQGDQLSTHTDGIPHLVLSPSTVFFGAFAL

SEQ ID NO: 60 (TNFbeta variant D1 monomer B)

MTPPERLFLPRVCGTTLHLLLLGLLLVLLPGAQGLPGVGLTPSAAQTARQHPKM
HLAHSTLKPAAHLIGDPSKQNSLLWRANTDRAFLQDGFSLSNNSLLVPTSGYYF
VYSQVVFSGKAYSPKATSSPLYLAHEVQLFSSQYPFHVPLLDSQKMVYPGLQEP
WLHSMYHGAYFQLTQGDQLSTHTDGIPHLVLSPSTVFFGAFAL

SEQ ID NO: 61 (TNFbeta variant D2 monomer A)

MTPPERLFLPRVCGTTLHLLLLGLLLVLLPGAQGLPGVGLTPSAAQTARQHPKM
HLAHSTLKPAAHLVGDPSKQNSLLWRANTDRAFLQDGFSLSNNSLLVPTSGIYF
VYSQVVFSGKAYSPKATSSPLYLAHEVQLFSSQYPFHVPLLSSQKMVYPGLQEP
WYHSMYHGAAFQLTQGDQLSTHTDGIPHLVLSPSTVFFGAFAL

SEQ ID NO: 62 (TNFbeta variant D2 monomer B)

MTPPERLFLPRVCGTTLHLLLLGLLLVLLPGAQGLPGVGLTPSAAQTARQHPKM
HLAHSTLKPAAHLVGDPSKQNSLLWRANTDRAFLQDGFSLSNNSLLVPTSGYYF
VYSQVAFSGKAYSPKATSSPLYLAHEVQLFSSQYPFHVPLLDSQKMVYPGLQEP
WLHSMYHGAYFQLTQGDQLSTHTDGIPHLVLSPSTVFFGAFAL

SEQ ID NO: 63 (human CD40L variant D1 monomer A)

MIETYNQTSPRSAATGLPISMKIFMYLLTVFLITQMIGSALFAVYLHRRLDKIEDE
RNLHEDFVFMKTIQRCNTGERSLSLLNCEEIKSQFEGFVKDIMLNKEETKKENSF
EMQKGDQNPQIAAHVISEASSKTTSVLQWAEKGYYDMSNNLVTLENGKQLTV
KRQGLYYIYAQVDFCSNREASSQAPFIASLCLKSPGRFERILLRAANTHSSAKPC
GYQSIHLGGVFELQPGASVFVNVTDPSQVSHGTGFTSFGLLKL

SEQ ID NO: 64 (human CD40L variant D1 monomer B)

MIETYNQTSPRSAATGLPISMKIFMYLLTVFLITQMIGSALFAVYLHRRLDKIEDE
RNLHEDFVFMKTIQRCNTGERSLSLLNCEEIKSQFEGFVKDIMLNKEETKKENSF
EMQKGDQNPQIAAHVISEASSKTTSVLQWAEKGYYTMSNNLVTLENGKQLTVK
RQGYYYIYAQVTFCSNREASSQAPFIASLCLKSPGRFERILLDAANTHSSAKPCG
QQSIHLGGYFELQPGASVFVNVTDPSQVSHGTGFTSFGLLKL

SEQ ID NO: 65 (human CD40L variant D2 monomer A)

MIETYNQTSPRSAATGLPISMKIFMYLLTVFLITQMIGSALFAVYLHRRLDKIEDE
RNLHEDFVFMKTIQRCNTGERSLSLLNCEEIKSQFEGFVKDIMLNKEETKKENSF
EMQKGDQNPQIAAHVVSEASSKTTSVLQWAEKGYYTMSNNLVTLENGKQLTV
KRQGLYYIYAQVTFCSNREASSQAPFIASLCLKSPGRFERILLRAANTHSSAKPCG
YQSIHLGGVFELQPGASVFVNVTDPSQVSHGTGFTSFGLLKL

SEQ ID NO: 66 (human CD40L variant D2 monomer B)

MIETYNQTSPRSAATGLPISMKIFMYLLTVFLITQMIGSALFAVYLHRRLDKIEDE
RNLHEDFVFMKTIQRCNTGERSLSLLNCEEIKSQFEGFVKDIMLNKEETKKENSF
EMQKGDQNPQIAAHVVSEASSKTTSVLQWAEKGYYTMSNNLVTLENGKQLTV
KRQGYYYIYAQVAFCSNREASSQAPFIASLCLKSPGRFERILLDAANTHSSAKPC
GQQSIHLGGYFELQPGASVFVNVTDPSQVSHGTGFTSFGLLKL

SEQ ID NO: 67 (human APRIL variant D1 monomer A)

MPASSPFLLAPKGPPGNMGGPVREPALSVALWLSWGAALGAVACAMALLTQQ
TELQSLRREVSRLQGTGGPSQNGEGYPWQSLPEQSSDALEAWENGERSRKRRA
VLTQKQKKQHSVLHLVPINATSKDDSDVTEVMWQPADRRGRGLQAQGYGVRI
QDAGVYLLYSQVSFQDVTFTMGQVVSREGQGRQETLFRCIRSMPSHPDRAYNS
CYSAGVFHLHQGDILSVIIPRARAKLNLSPHGTFLGFVKL

SEQ ID NO: 68 (human APRIL variant D1 monomer B)

MPASSPFLLAPKGPPGNMGGPVREPALSVALWLSWGAALGAVACAMALLTQQ
TELQSLRREVSRLQGTGGPSQNGEGYPWQSLPEQSSDALEAWENGERSRKRRA
VLTQKQKKQHSVLHLVPINATSKDDSDVTEVMWQPALRRGRGLQAQGYGVRI
QDAGYYLLYSQVLFQDVTFTMGQVVSREGQGRQETLDRCIRSMPSHPDRAYNS
CYSAGYFHLHQGDILSVIIPRARAKLNLSPHGTFLGFVKL

SEQ ID NO: 69 (human APRIL variant D2 monomer A)

MPASSPFLLAPKGPPGNMGGPVREPALSVALWLSWGAALGAVACAMALLTQQ
TELQSLRREVSRLQGTGGPSQNGEGYPWQSLPEQSSDALEAWENGERSRKRRA
VLTQKQKKQHSVLHLVPINATSKDDSDVTEVMWQPALRRGRGLQAQGYGVRI
QDAGVYLLYSQVLFQDVTFTMGQVVSREGQGRQETLFRCIRSMPSHPDRAYNS
CYSAGVFHLHQGDILSVIIPRARAKLNLSPHGTFLGFVKL

SEQ ID NO: 70 (human APRIL variant D2 monomer B)

MPASSPFLLAPKGPPGNMGGPVREPALSVALWLSWGAALGAVACAMALLTQQ
TELQSLRREVSRLQGTGGPSQNGEGYPWQSLPEQSSDALEAWENGERSRKRRA
VLTQKQKKQHSVLHLVPINATSKDDSDVTEVMWQPALRRGRGLQAQGYGVRI
QDAGYYLLYSQVAFQDVTFTMGQVVSREGQGRQETLDRCIRSMPSHPDRAYNS
CYSAGYFHLHQGDILSVIIPRARAKLNLSPHGTFLGFVKL

SEQ ID NO: 71 (human OX40L variant D1 monomer A)

MERVQPLEENVGNAARPRFERNKLLLVASVIQGLGLLLCFTYICLHFSALQVSH
RYPRIQSIKVQFTEYKKEKGFILTSQKEDEIMKVQNNSVIINCDGDYLISLKGYFS
QEVNISLHYQKDEEPLFSLKKVRSVNSLMVASLTYKDKVYLNVTTDNTSLDDFH
VNGGELILIHQNPGEFCVL

SEQ ID NO: 72 (human OX40L variant D1 monomer B)

MERVQPLEENVGNAARPRFERNKLLLVASVIQGLGLLLCFTYICLHFSALQVSH
RYPRIQSIKVQFTEYKKEKGFILTSQKEDEIMKVQNNSVIINCDGFYLISLKGYFS
QEVNISLHYQKDEEPLFQLKKVRSVNSLMDASLTYKDKVYLNVTTDNTSLDDF
HVNGGELILIHQNPGEFCVL

SEQ ID NO: 73 (human OX40L variant D2 monomer A)

MERVQPLEENVGNAARPRFERNKLLLVASVIQGLGLLLCFTYICLHFSALQVSH
RYPRIQSIKVVFTEYKKEKGFILTSQKEDEIMKVQNNSVIINCDGFYLISLKGYFS
QEVNISLHYQKDEEPLFQLKKVRSVNSLMVASLTYKDKVYLNVTTDNTSLDDF
HVNGGELILIHQNPGEFCVL

SEQ ID NO: 74 (human OX40L variant D2 monomer B)

MERVQPLEENVGNAARPRFERNKLLLVASVIQGLGLLLCFTYICLHFSALQVSH
RYPRIQSIKVVFTEYKKEKGFILTSQKEDEIMKVQNNSVIINCDGFYLISLKGYFS

QEVNISLHYQKDEEPLFALKKVRSVNSLMDASLTYKDKVYLNVTTDNTSLDDF
HVNGGELILIHQNPGEFCVL

SEQ ID NO: 75 (human GITRL variant D1 monomer A)

MCLSHLENMPLSHSRTQGAQRSSWKLWLFCSIVMLLFLCSFSWLIFIFLQLETAK
EPCMAKFGPLPSKWQMASSEPPCVNKVSDWKLEILQNGLYLIYGQVAPNANYN
DVAPFEVRLYKNKDMIQTLTNKSKIQNVGGTYELHVGDTIDLIFNSEHQVLKNN
TYWGIILLANPQFIS

SEQ ID NO: 76 (human GITRL variant D1 monomer B)

MCLSHLENMPLSHSRTQGAQRSSWKLWLFCSIVMLLFLCSFSWLIFIFLQLETAK
EPCMAKFGPLPSKWQMASSEPPCVNKVSDWKLEILQNGDYLIYGQVAPNANYN
DVAPFEVRLYKNKDMIQSLTNKSKIQNVGGTYELHVGDTIDLIFNSEHQVLKNN
TYWGIILLANPQFIS

SEQ ID NO: 77 (human GITRL variant D2 monomer A)

MCLSHLENMPLSHSRTQGAQRSSWKLWLFCSIVMLLFLCSFSWLIFIFLQLETAK
EPCMAKFGPLPSKWQMASSEPPCVNKVSDWKLEILQNGLYLIYGQVYPNANYN
DVAPFEVRLYKNKDMIQTLTNKSKIQNVGGTYELHVGDTIDLIFNSEHQVLKNN
TYWGIILLANPQFIS

SEQ ID NO: 78 (human GITRL variant D2 monomer B)

MCLSHLENMPLSHSRTQGAQRSSWKLWLFCSIVMLLFLCSFSWLIFIFLQLETAK
EPCMAKFVPLPSKWQMASSEPPCVNKVSDWKLEILQNGLYLIYGQVYPNANYN
DVAPFEVRLYKNKDMIQALTNKSKIQNVGGDYELHVGDTIDLIFNSEHQVLKNN
TYWGIILLANPQFIS

**Claims**

1. A variant of a TNF family ligand which is mutated such that it is not capable of assembling into a trimer, wherein the variant ligand retains the ability to bind one or more of its cognate receptor(s), but wherein binding to the receptor does not activate the receptor.

2. The variant of a TNF family ligand of claim 1, wherein the variant is capable of assembling into a dimer with another variant of the same ligand.

3. The variant of a TNF family ligand of claim 2, wherein the TNF family ligand is selected from the group consisting of RANKL, TRAIL, APRIL, BAFF, TNFalpha, CD30L, CD40L, FasL, Light, and Tweak.

4. The variant of a TNF family ligand of any one of the preceding claims, wherein the variant comprises a mutation at one or more of positions 168, 194, 212, 220, 229, 256, 271, and 279 in the murine RANKL sequence (169, 195, 213, 221, 230, 257, 272 and 280 in the human RANKL sequence), or an equivalent position as set out in Table 1 herein.

5. The variant of a TNF family ligand of claim 4, wherein the murine RANKL variant comprises one or more of the mutations T168V, K194D, F212Y, C220S, C220A, D229K, S229K, K256D, F271Y and F279Y (T169V, K195D, F213Y, C221S, S230K, K257D, F272Y and F280Y in human RANKL).

6. The variant of a TNF family ligand of claim 1, wherein the variant is not capable of assembling into a dimer with the same or other TNF family ligands.

7. The variant of a TNF family ligand of claim 6, wherein the variant is selected from the group consisting of APRIL and BAFF.

8. A dimer comprising two variants of a TNF family ligand of any one of claims 1-5.

9. The dimer of claim 8, wherein the dimer is a heterodimer.

10. A complex comprising one or more of the variant of a TNF family ligand of claims 1-5 and one or more cognate receptors for the TNF family ligand.

11. A nucleotide sequence encoding the variant of a TNF family ligand of any one of claims 1-7 or the dimer of claims 8 or 9.

12. A vector comprising the nucleotide sequence of claim 10.

13. A pharmaceutical composition comprising the variant of a TNF family ligand of any one of claims 1-7, the dimer of claim 8 or claim 9, the complex of claim 10, the nucleotide sequence of claim 11, or the vector of claim 12.

14. The variant of a TNF family ligand of any one of claims 1-7, the dimer of claim 8 or claim 9, the complex of claim 10, the nucleotide sequence of claim 11, the vector of claim 12 or the pharmaceutical composition of claim 13 for use in the treatment of osteoporosis, rheumatoid arthritis, Paget's disease, malignancy induced bone disease or cancer.

15. A method for producing a variant of a TNF family ligand comprising the steps of:

a) identifying amino acids in the TNF family ligand that are located in the trimerisation interface as candidates for mutation;
b) substituting each of one or more residues in the trimerisation interface; and
c) selecting amino acid substitutions which have a neutral or positive effect on the stability of the dimer but a negative effect on the stability of the trimer.

Figure 1.

**Figure 1D**

Figure 2

Figure 3

Figure 4(A)

Figure 4(B)

Figure 4(C)

EP 2 468 764 A1

**Figure 5**

EP 2 468 764 A1

Figure 6

100 ng/mL WT RANK-L

Figure 6(contd.)

100 ng/mL RANK - L Dimer 2

Figure 6(contd.)

100 ng/mL wtRANK-L
+ 100 ng/mL RANK-L Dimer2

Figure 6(contd.)

untreated

Figure 7.

Figure 8

Figure 9

EP 2 468 764 A1

EP 2 468 764 A1

**Figure 10(A)**

20100732f f1agRANKL wt 160610 superdex 75 GFC analytical001:10_UV001.PEAK

**Figure 10(B)**

20100731c flagRANKL 1 1 1 0903 superdex 75 GFC analytical001:10_UV001.PEAK

Figure 10(C)

20100732f f1agRANKL 2HI superdex75 GFC analytical001:10_UV802.PEAK

Figure 11

EP 2 468 764 A1

**Figure 12**

# Figure 13

# Figure 14 A

```
soluble mRANKL   M G K P E A Q P F A H L T I N A A S I P S G S H K V T L S S W Y H D R G W A[D]K I S N M T L S N G K L R V
soluble hRANKL   - - . L . . . . . . . . . . . . T D . . . . . . . . S . . . . . . . . . . . F . . . . . I .
                 100%
Conservation
                 0%

soluble mRANKL   N Q D G[Y]F Y L Y A N I[S]C F R H H E T S G S V P T D Y L Q L M V Y V V K T S I K I P S S H N L M[D]K G G S
soluble hRANKL   . . . . . . . . . . . . . . . . . . D L A . E . . . . . . . . . T . . . . . . . . T . . . . . .
                 100%
Conservation
                 0%

soluble mRANKL   T K N W S G N S E F H[Y]F Y S I N V G G[Y]F K L R A G E E I S I Q V S N P S L L D P D Q D A T Y F G A F K
soluble hRANKL   . . Y . . . . . . . . . . . . . . . . . . . . . . . S . . . . . . E . . . . . . . . . . . . . . . . .
                 100%
Conservation
                 0%

soluble mRANKL   V Q D I D   316
soluble hRANKL   . R . . .   317
                 100%
Conservation
                 0%
```

Figure 14 B

```
soluble mRANKL   MGKPEAQPFAHL T NAAS I PSGSHKVTLSSWYHDRGWAK I SNMTLSNGKLRV
                            D
soluble hRANKL   - - . L . . . . . . . . . .   . . . TD . . . . . . . . S . . . . . . . . . . . . F . . . . . . I .
100%
Conservation
0%
```

```
soluble mRANKL   NQDG F Y YLYAN I C RHHETSG S VPTDYLQLMVYVVKTS IK I PSSHNLM KGGS
                      Y       S       K                                        D
soluble hRANKL   . . . . . . . . . . . . . . . . . . . . . DLA . E . . . . . . . . . . T . . . . . . . . . . T . . . . . .
100%
Conservation
0%
```

```
soluble mRANKL   TKNWSGNSEFH F YS I NVGG F KLRAGEE I S I QVSNPSLLDPDQDATYFGAFK
                          Y       Y
soluble hRANKL   . . . Y . . . . . . . . . . . . . . . . . . S . . . . . E . . . . . . . . . . . . . . . . . . . .
100%
Conservation
0%
```

```
soluble mRANKL   VQD I D   316
soluble hRANKL   . R . . .   317
100%
Conservation
0%
```

Figure 15

Figure 16

Figure 17

Figure 18(A)

Figure 18(B)

Figure 18(C)

Figure 19

WT

EP 2 468 764 A1

Figure 19(contd.)

D1

| | |
|---|---|
| ——— 090304 D1 4 nM | ——— 090304 D1 31.25 pM |
| ——— 090304 D1 2 nM | ——— 090304 D1 62.5 pM |
| ——— 090304 D1 1 nM | - - - - - 090304 D1 125 pM |
| ——— 090304 D1 500 pM | ·········· 090304 D1 250 pM |
| —·—·— 090304 D1 250 pM | ——— 090304 D1 500 pM |
| -··-··- 090304 D1 125 pM | ——— 090304 D1 1 nM |
| ——— 090304 D1 62.5 pM | ——— 090304 D1 2 nM |
| ———— 090304 D1 31.25 pM | ——— 090304 D1 4 nM |

EP 2 468 764 A1

Figure 19(contd.)

**D2**

Legend:

- 090310 D2 4 nM
- 090310 D2 2 nM
- 090310 D2 1 nM
- 090310 D2 500 pM
- 090310 D2 250 pM
- 090310 D2 125 pM
- 090310 D2 62.5 pM
- 090310 D2 31.25 pM
- 090310 D2 31.25 pM
- 090310 D2 62.5 pM
- 090310 D2 125 pM
- 090310 D2 250 pM
- 090310 D2 500 pM
- 090310 D2 1 nM
- 090310 D2 2 nM
- 090310 D2 4 nM

A

Figure 20

B

Figure 21

Figure 22

A

B

A) Structural alignment of murine RANKL (1s55_a,Query) and human CD40L (1aly_a,Hit):                    **Figure 23**

```
Query:    1 ----AQPFAHLSINAASIPSGSHK-----VTLSSWYHDRGWAEIS--NMTLS-NGKLRVNQDGTYLIANISFRHHETSGSVPTDYL---------QLMYYVKTSIKIPS----SHNLMQGGSTKNWSGNSEF---HE 111
            Q  AH:  :A          :L :W :  |G|  :S   :TL: : :L V:::G:YY|IA:| F               ::: :  S     :: L||:::T : :
Hit  :    1 GDQNPQIAAHVSSEA---------SSKTTSVL-QWAE-KGYYMSNNLVTLENCKQLTVKRQGTYIYAQVTFC------------SHREASSQAPFIASLCLKS-----PGRPERILLSRAANTHSSA-----KPCG 105


Query:  112 YSINVGGSFKLRAGEEISIQVSNPSLLDPDQDATYFGAFKVQDID 156
            SI::GG F:L: G :|  |:V::PS ::    F FG :K:
Hit  :  106 QSIHLGGSFELQPGASVFVNVTDPSQVSNGTGFTSFGLLKL---- 147
```

The above structural alignment between CD40L (1aly_a) and murine RANKL (1s55_a) has an RMSD of 1.305 Å over 118 aligned residues with 29.66% sequence identity.

B) Structural alignment of murine RANKL (1s55_a,Query) and human TRAIL (1d4v_b,Hit):

```
Query:    1 -AQPFAHLSIN---AASIPSGSH-----------KVTLSSWYHD-RGWAEISNMTLSNGKLRVNQDGTYLIANISFRNHETSGSVPT--------DYLQLMYYVKTSIKI--PSSHNLMQGGSTKNW--SGNSEFH 110
              : AH|F :   : :             |:SW :  G : |SN| L NG:L |:| GFYY|Y:: FR :        : QI: Y| K :   P: LMR:: ::   S ::E|
Hit  :    1 PQRVPAHISGTRGRSWT------LSSPNSKNEKALGRKINSWESSRSCGNSSLHLRANGELVIHEKGTYIYSQTSFRFQ--------EEIKENTKNDKQMVQYIYKYT---SYPDPILLMRSARNS--CNSKDAEYG 120


Query:  111 SYSINVGGSFKLRAGEEISIQVSNPSLLDPDQDATYFGAFKVQDID 156
            :YSI  GG:F:L| :|:I |:V:N  L|D D:|A:|FGAF V
Hit  :  121 SYSIYQGGSFELKENDRIFVSVTNEHLIDMDHEASFGAFLVG--- 164
```

The above structural alignment between human TRAIL (1d4v_b) and murine RANKL (1s55_a) has an RMSD of 1.349 Å over 134 aligned residues with 35.07% sequence identity.

Figure 23(contd.)

C) Structural alignment of murine RANKL (1s55_a,Query) and TNF-alpha (1tnf,Hit):

```
Query:   1 ----AQPFAHLTINAASIPSGSHKVTLS--SWYHDRGWARIS-NMTLSNGKLRVNQDGTYLYANITTRHHETSGSVP----TDYLQLMVYVVKTSIKIP-----SSHMIANGGSTKNWSGNS---------EFHRYS 113
             :P AH:: N :           :N : R: A |: :: L :::L V :|G:Y |Y::| F|        : |: L  | {        :: NL|:: ::              : |
Hit  :   1 RTPSDKPVAHVTANPQAEG--------QLQWLNRRANALLANGVELRDNQLVVPSEGTYLIYSQVTFKG--------QGCPSTHVLLTHTISRI-----AVSYQTKVNLLSAIKSPC-----QRETPEGAEAKPWYEP 112
```

Query: 114 INVGGTFKLRAGEEISIQVSNPSLLQP-DQDATYFGAFKVQDID 156
              I :GG F:L: G|:|S |{::P: L  |:  :YFG : :
Hit  : 113 IYLGGVFQLEKGDRLSAEINRPDYLLFAESGQVYFGIIAL---- 153


The above structural alignment between TNF-alpha (1tnf) and murine RANKL (1s55_a) has an RMSD of 1.207 Å over 125 aligned residues with 23.20% sequence identity.

D) Structural alignment of murine RANKL (1s55_a,Query) and TNF-beta (1tnr,Hit):

```
Query:   1 AQPFAHLTINAASIPSGSHKVTLS----SWYHDRGWARIS-NMTLSNGKLRVNQDGTYLYANITTRHHETSGSVPTD----------YLQLMVYVVKTSIKIPS------SHNLANGGSTKNWSGNSEFHRYSINVGG 118
             :P AHL : ::            N :   A |: :::LSN::L V :G:Y|:Y::| F          L L V          L|:: :  : :: ::S: G:
Hit  :   1 -KPAAHLTGDPSK----------QNSLLWRANTDRAFLQDGFSLSNWSLLVPTSGTYEVYSQVTFSG----------KAYSPKATSSPLYLAHEVQLF------SSQYPFHVPLLSSQKNVYPGLQE-PWLESNYNGA 110
```

Query: 119 TFKLRAGEEISIQVSNPSLLDPDQDATYFGAFKVQDID 156
              F:L  G|||S ::::  L  : :::|FGAF :
Hit  : 111 TPQLTQGDQLSTHTDGIPHLVLSPSTVFFGAFAL---- 145


The above structural alignment between TNF-beta (1tnr) and murine RANKL (1s55_a) has an RMSD of 1.041 Å over 123 aligned residues with 24.39% sequence identity.

**Figure 23(contd.)**

E) Structural alignment of murine RANKL (1s55_a,Query) and murine APRIL (1u5x,Hit):

```
Query:   1 A-QPFAHLIINAASIPSCSHK------VTLSSWYHDRGNARIS--NMTLSNGKLRVNQDGRYILYANIRFRHHETSGSVPTD--YLQLNVYVVKTSIKIP--SSHNLNRGGSTKNWSGNSEF---HEYSINVGGRFKLR 123
             :    HL:             :  :  W    :   :|  ::  :RV : G:Y LY::|  F:        :|  V |  :      ::L:|   :           | S  :G F L:
Hit  :   1 -KHSVLHLRPV---------NITSDVTEV-NWQP----VRRRGRGLERQGDIVRVWDTGRYLLYSQVRTH----------DVTFTMGQVVSREG----QGRRETLFRCIRSM--------PSDARNSCYSAGRFHLH 100
```

```
Query: 124 AGEEISIQVSNPS-----LLDPDQDATYFGAFKVQDID 156
             G| I:|:|         L: :  :I|:G  K:
Hit  : 101 QGDIITVKI----PRANAKLSLSPHGTFLGFVKL---- 131
```

The above structural alignment between murine APRIL (1u5x) and murine RANKL (1s55_a) has an RMSD of 1.410 Å over 109 aligned residues with 20.18% sequence identity.

F) Structural alignment of murine RANKL (1s55_a,Query) and a homology based model of human April (hApril,Hit):

```
Query:   1 A-QPFAHLIINAASIPSGS--------HKVTLSSWTHDRGWARIS---NMTLSNGKLRVNQDGRYILYANIRFRHHETSGSVPTDYL----QLNVYVVKTSIKIP--SSHNLNRGGSTKNWSGNS----EFHRYSINVG 117
             :    HL:           :  :  W    A    :|  ::  :R|:: G Y LY::|  F:        }   V |   :     ::L:|   :        | | S  :
Hit  :   1 -QHSVLHLRPI----------NATSKDDSDVTEV-NWQP----ARR-RGNGLQAQGYGVRIQDACRYLLYSQVRFQ------------DVTFTMGQVVSREG----QGRQETLFRCIRSM------PSHPDRARNSCYSA 101
```

```
Query: 118 GRFKLRAGEEISIQVSNPS-----LLDPDQDATYFGAFKVQDID 156
             G F L: G| |S| |        L: :  :W|:G  K:
Hit  : 102 GRFHLHQGDIISVII----PRARAKLNLSPHGTFLGFVKL---- 138
```

The above structural alignment between human April (hApril) and murine RANKL (1s55_a) has an RMSD of 1.485 Å over 110 aligned residues with 20.00% sequence identity.

Figure 23(contd.)

G) Structural alignment of murine RANKL (1s55_a,Query) and human BAFF (1oqe,Hit):

```
Query:   1 A--QPFAHLSINAASIPSGSH-----KVTLSSNYHDRGWAKISH---MYLSNGKLRVNQDGSYYLYANISFRHHETSGSVPTDYL----QLMYYVVKTSIKIP---------SSHNLSGGSTKNWSGNSE---FTMYS 113
           Q  :L  :: :          :: N     :    |  ::K| V:| G|||Y::| |             |  ] |      S :L:| ::      : S
Hit  :   1 -VTQDCLQLSADSETPT----IQKGSYTFV-PWLL----SSKR-GSALEEKENKILVKETGSFFIYGQVLT------------DKTYANGHLIQRK-----KVHVFGDELSLVTLFRCIQNM-------PETLPANS 103
```

```
Query: 114 INVGGSFKLRAGEEISIQVSRPS-----LLDPQDATYFGAFKVQDID- 157
           :G: KL: G|E|:| |        |: D D:T|FGA:K:
Hit  : 104 CYSAGSAKLEEGDELQLAI----PRENAQISLDGDVTFFGALKL----L 144
```

The above structural alignment between human BAFF (1oqe) and murine RANKL (1s55_a) has an RMSD of 1.330 A over 112 aligned residues with 19.64% sequence identity.

H) Structural alignment of murine RANKL (1s55_a,Query) and human OX40L (2hev,Hit):

```
Query:   1 AQPFAHLSINAASIPSGSHKVT-----LSSNYHDRGNAKIS-----NMTLSNGKLRVNQDGSYYLYANISFRHHETSGSVPTDYLQL---MVYVVKTSIKIPSS---HYLMSGGSTKWMSGNSEFHF---YSINVSGST 120
                    : ::           N ::::::| |N DGFY |  : F       |:        : L:: S|N
Hit  :   1 RIQSIKVSFTE-----------YKKEKGFILTS--------QKEDEIMKVQDNSVIIMCDGSYLISLKGSFS---------------QEVDISLHYQ-------KDEEPLFEL-------------KKVRSVNSLMA 84
```

```
Query: 121 KLRAGEEISIQVSNP-------SLLDPDQD-ATYFGAFKVQDID-------- 157
           :L  |:| |:V::          :       : : :
Hit  :  85 SLTYKDKVYLNVTT-DNTSLDDFHV-----NGGELILIHQ----NPGEFCVL 126
```

The above structural alignment between human OX40L (2hev) and murine RANKL (1s55_a) has an RMSD of 1.603 A over 91 aligned residues with 13.19% sequence identity.

Figure 23(contd.)

I) Structural alignment of murine RANKL (1s55_a,Query) and human GITRL (3b94,Hit):

```
Query:   1 A-QPFAHLIINDASIPSGSHKVTLSSWYHDRGNAKIS--------------NMTLSNG-KLRVNQDGYYLYANIIRHHETSGSVPTDYLQL-MVYVVKTSIKIPSSHNLMGGSTKNWSGNSEFRFYS-INVGGRK 121
           :A :                                             :: : KL:| Q:G:Y |Y::||               V : K:      : |: :             NVGG |:
Hit  :   1 -EPCMAKFG--------------------------PLPSKWQNASSEPPCVNKVSDWKLEILQNGHYLIYGQVG----------------FZVRLYKN------KDMIQTLTN--------------KQNVGGRYE 73
```

```
Query: 122 LRAGEEISIQVSNPSLLDPDQD---ATYFGAFKVQDID- 157
           L::G| I:| :           YY:G : : : :
Hit  :  74 LRVGDTTDLIFN----------LKNWTYWGIILLANPQF 102
```

The above structural alignment between human GITRL (3b94) and murine RANKL (1s55_a) has an RMSD of 1.526 A over 80 aligned residues with 23.75% sequence identity.

J) Structural alignment of murine RANKL (1s55_a,Query) and human EDA-A1 (1rj7,Hit):

```
Query:   1 A-QPFAHLIINPASIPSGSHK-----------VTLSSWYHDRGWAKIS--NMTLG--NGKLRVNQDGRYLYANIIFRHHETSGSVPTDYL------QLNVYVVKTSIKIPSSHNLMGGSTKNWSGNSE-FRHYSINV 116
             :HL ::                      :L::W :       :: : L :G:L:V DG Y||Y::|            VV       : :|: : : :       :| : :
Hit  :   1 -QPAVVHLIGQG---------SAIQVKNDLSGGVLNDWSR----IRMNPKVFKLHPRSGRLEVLVDGIYFIYSQVEVY-------------YINFTDFASYEVVVD------ERPFLQTRSIETG----KTNWNTCYT 102
```

```
Query: 117 GGIFKLRAGEEISIQVSNPS----LLDPDQDATYFGAFKVQDID- 157
           :G   L|A |:I:|::          |: :: :T|IGA:|: |
Hit  : 103 AGKCLLKRQKIAVRMV---HADISIRNSKHTTFFGAIRLGEAPA 144
```

The above structural alignment between human EDA-A1 (1rj7) and murine RANKL (1s55_a) has an RMSD of 1.264 A over 116 aligned residues with 18.97% sequence identity

**Figure 23(contd.)**

K) Structural alignment of murine RANKL (1s55_a,Query) and human EDA-A2 (1rj8,Hit):

```
Query:   1 AQPFAHLTINAASIP----SGSHK-----VTLSSWYHDRGWAKIS--NMTLS--NGKLRVNQDGTYYLYANICFRHHETSGSVPTDYL---QLMVYVVKTSIKIPSSHNLMEGGSTKNMSGNSEFH-----FTSINVGG 118
            :KL :      : |      :L:W :    :| : L :G:L:V  DG Y||Y::|  | :              W      : :|: :: |        |: ::G
Hit  :   1 -PAVVHLKGQ-----GSAIQVK--NDLEGGVLNDWSR----ITMNPKVFKLHPRSGELEVLVDGTYFIYSQVYYIN-----------FTDFASYEVVVD-----EKPFLRCYRSIE--------TGKTHTNTCYTAG 101
```

```
Query: 119 GFKLRAGEEISIQVSNPS----LLDPDQDATYFGAFKVQDID- 157
            L|A |:I:|::        |: :: :T|FGA:|: |
Hit  : 102 SCLLKARQKIAVKKV---EADISINVSKHTFFGAIRLGEA-P 140
```

The above structural alignment between human EDA-A2 (1rj8) and murine RANKL (1s55_a) has an RMSD of 1.369 A over 114 aligned residues with 19.30% sequence identity

Figure 24

EP 2 468 764 A1

**TNF-alpha**

Figure 24(contd.)

**TNF-beta**

Figure 24(contd.)

Figure 24(contd.)

Figure 24(contd.)

Figure 24(contd.)

EDA-A2

Europäisches
Patentamt

European
Patent Office

Office européen
des brevets

**EUROPEAN SEARCH REPORT**

Application Number

EP 10 25 2230

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/219864 A1 (DESJARLAIS JOHN R [US] ET AL) 27 November 2003 (2003-11-27) * [0114], [0135],[0234]- [0236]claim 20, Figures, Table 1. * | 1-6 | INV. C07K14/435 ADD. C07K14/00 |
| X | US 2006/014248 A1 (MARSHALL SHANNON A [US] ET AL) 19 January 2006 (2006-01-19) * Claim 6-9 * | 1-6 | |
| A | MELLIS D J ET AL: "A mutation that prevents rank trimer formation does not prevent signalling downstream of rank", BONE, PERGAMON PRESS., OXFORD, GB, vol. 47, 1 June 2010 (2010-06-01), pages S137-S138, XP027170511, ISSN: 8756-3282 [retrieved on 2010-06-01] * abstract * | 1 | |
| A | BODMER J-L ET AL: "The molecular architecture of the TNF superfamily", TRENDS IN BIOCHEMICAL SCIENCES, ELSEVIER, HAYWARDS, GB, vol. 27, no. 1, 1 January 2002 (2002-01-01), pages 19-26, XP004332356, ISSN: 0968-0004, DOI: DOI:10.1016/S0968-0004(01)01995-8 * the whole document * | 1-15 | TECHNICAL FIELDS SEARCHED (IPC) C07K |

-/--

~~The present search report has been drawn up for all claims~~

| Place of search | Date of completion of the search | Examiner |
|---|---|---|
| Munich | 6 July 2011 | Gómez Ortiz, Mariola |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 10 25 2230

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOYCE B F ET AL: "Functions of RANKL/RANK/OPG in bone modeling and remodeling", ARCHIVES OF BIOCHEMISTRY AND BIOPHYSICS, ACADEMIC PRESS, US, vol. 473, no. 2, 15 May 2008 (2008-05-15), pages 139-146, XP022668167, ISSN: 0003-9861, DOI: DOI:10.1016/J.ABB.2008.03.018 [retrieved on 2008-03-25] * the whole document * | 1 | |

TECHNICAL FIELDS
SEARCHED      (IPC)

~~The present search report has been drawn up for all claims~~

| Place of search | Date of completion of the search | Examiner |
|---|---|---|
| Munich | 6 July 2011 | Gómez Ortiz, Mariola |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

Europäisches
Patentamt
European
Patent Office
Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-15(partially)

   Variants of a TNF family ligand which is mutated at position 168 in the murine RANKL sequence (or equivalent) such that is not capable of assembling into a trimer.
   ---

2. claims: 1-15(partially)

   Variants of a TNF family ligand which is mutated at position 194 in the murine RANKL sequence (or equivalent) such that is not capable of assembling into a trimer.
   ---

3. claims: 1-15(partially)

   Variants of a TNF family ligand which is mutated at position 212  in the murine RANKL sequence (or equivalent) such that is not capable of assembling into a trimer.
   ---

4. claims: 1-15(partially)

   Variants of a TNF family ligand which is mutated at position 220 in the murine RANKL sequence (or equivalent) such that is not capable of assembling into a trimer.
   ---

5. claims: 1-15(partially)

   Variants of a TNF family ligand which is mutated at position 229 in the murine RANKL sequence (or equivalent) such that is not capable of assembling into a trimer.
   ---

6. claims: 1-15(partially)

   Variants of a TNF family ligand which is mutated at position 256 in the murine RANKL sequence (or equivalent) such that is not capable of assembling into a trimer.
   ---

7. claims: 1-15(partially)

   Variants of a TNF family ligand which is mutated at position 271 in the murine RANKL sequence (or equivalent) such that is not capable of assembling into a trimer.
   ---

8. claims: 1-15(partially)

Europäisches Patentamt

European Patent Office

Office européen des brevets

**LACK OF UNITY OF INVENTION
SHEET B**

Application Number

EP 10 25 2230

The Search Division considers that the present European patent application does not comply with the requirements of unity of invention and relates to several inventions or groups of inventions, namely:

```
Variants of a TNF family ligand which is mutated at position
279 in the murine RANKL sequence (or equivalent) such that
is not capable of assembling into a trimer.
                    ---
```

## ANNEX TO THE EUROPEAN SEARCH REPORT
## ON EUROPEAN PATENT APPLICATION NO.

EP 10 25 2230

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2011

| Patent document cited in search report | | Publication date | Patent family member(s) | | Publication date |
|---|---|---|---|---|---|
| US 2003219864 | A1 | 27-11-2003 | US 2004121363 | A1 | 24-06-2004 |
| US 2006014248 | A1 | 19-01-2006 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5447851 A
- US 7381792 B

### Non-patent literature cited in the description

- **KANAZAWA, K. ; KUDO, A.** *J Bon. Min. Res.,* 2005, vol. 20, 2053-2060
- **PARFITT, A.M.** *Bone,* 2002, vol. 30, 5-7
- **TEITELBAUM, S.L.** *Science,* 2002, vol. 289, 1504-1508
- **JOSEPH MELTON III, L.** *J. Bon. Min. Res.,* 2003, vol. 18, 1139-1141
- **BOYCE, B.F. ; XING, L.** *Arch. Biochem. Biophys.,* 2008, vol. 474, 139-146
- **ANDERSON, D.M. ; MARASKOVSKY, E. ; BILLINGSLEY, W.L. ; DOUGALL, W.C. ; TOMETSKO, M.E. ; ROUX, E.R. ; TEEPE, M.C. ; DUBOSE, R.F. ; COSMAN, D. ; GALIBERT, L.** *Nature,* 1997, vol. 390, 175-179
- **WONG, B.R. ; RHO, J. ; ARRON, J. ; ROBINSON, E. ; ORLINICK, J. ; CHAO, M. ; KALACHIKOV, S. ; CAYANI, E. ; BARTLETT III, F.S. ; FRANKEL, W.N.** *J.B.C.,* 1997, vol. 272, 25190-25194
- **LACEY, D.L. ; TIMMS, E. ; TAN, H.L. ; KELLEY, M.J. ; DUNSTAN, C.R. ; BURGESS, T. ; ELLIOTT, R. ; COLOMBERO, A. ; ELLIOTT, G. ; SCULLY, S.** *Cell,* 1998, vol. 93, 165-167
- **WRIGTH, H.L. ; MCCARHY, H.S. ; MIDDLETON, J. ; MARSHALL, M.J.** *Curr Rev Musculoskelet Med,* 2009, vol. 2, 54-64
- **JI, J.-D. ; PARK-MIN, K.-H. ; SHEN, Z. ; FAJARDO, R.J. ; GOLDRING, S.R. ; MCHUGH, K.P. ; IVASHKIV, L.B.** *J. Imm.,* 2009, vol. 183, 7223-7233
- **ROATO, I. ; DÁMELIO, P. ; GORASSINI, E. ; GRIMALDI, A. ; BONELLO, L. ; FIORI, C. ; DELSEDIME, L. ; TIZZANI, A. ; DE LIBERO, A. ; ISAIA, G.** *Plos One,* 2008, vol. 3, e3627
- **MIKAMI, S. ; KATSUBE, K. ; OYA, M. ; ISHIDA, M. ; KOSAKA, T. ; MIZUNO, R. ; MOCHIZUKI, S. ; IKEDA, T. ; MUKAI, M. ; OKADA, Y.** *J. Pathol.,* 2009, vol. 218, 530-539
- **COLEMAN, R.E.** *Clin. Canc. Res.,* 2006, vol. 20s, 6243s-6249s
- **KOSTENUIT, P.J. ; NGUYEN, H.Q. ; MCCABE, J. ; WARMINGTON K.S. ; KURAHARA, C. ; SUN, N. ; CHEN, C. ; LI, L. ; CATTLEY, R.C. ; VAN, G.** *J. Bon. Min. Res.,* 2009, vol. 24, 182-195
- **SLOOT, A.M. VAN DER ; TUR, V. ; SZEGEZDI, E. ; MULLALY, M.M. ; COOL, R.H. ; SAMALI, A. ; SERRANO, L. ; QUAX, W.J.** *PNAS,* 2006, vol. 103, 8634-8639
- **ITO, S. ; HATA, T.** *Vitam. Horm.,* 2004, vol. 67, 19-33
- **BOSSEN, C. ; INGOLD, K. ; TARDIVEL, A. ; BODMER, J.-L. ; GAIDE, O. ; HERTIG, S. ; AMBROSE, C. ; TSCHOPP, J. ; SCHNEIDER, P.** *J.B.C.,* 2006, vol. 281, 13964-13971
- **SAMBROOK, J. ; FRITSCH, E.F. ; MANIATIS T.** Molecular Cloning: A Laboratory Manual. Cold Spring Harbor Laboratory, 1989
- **DUAN, L. ; VOS, P. DE ; FAN, M. ; REN, Y.** *Front. Biosci.,* 2008, vol. 13, 7064-7071
- **WALTER, T.S. ; LIU, C. ; HUANG P. ; ZHANG, S. ; WEDDERBUM, L.R. ; GAO B. ; OWENS, R.J. ; STUART, D.I. ; TANG, P. ; REN, J.** *Acta Cryst.,* 2009, vol. F65, 597-600
- **LEWIT-BENTLEY, A. ; FOURME, R. ; KAHN, R. ; PRANGÉ, T. ; VACHETTE, P. ; TAVERNIER, J. ; HAUQUIER, G. ; NIERS, W.** *J. Mol Biol,* 1988, vol. 199, 389-392
- **LAM, J. ; NELSON, C.A. ; ROSS, F.P. ; TEITELBAUM, S.L. ; FREMONT, D.H.** *JCI,* 2001, vol. 108, 971-979
- **GUEROIS, R. ; NIELSEN, J.E. ; SERRANO, L.** *J Mol Biol,* 2002, vol. 320, 369-387
- **SCHYMKOWITZ, J. ; BORG, J. ; STRICHER, F. ; NYS, R. ; ROUSSEAU, F. ; SERRANO, L.** *Nucleic Acid Res.,* 2005, vol. 33, W382-W388
- **SLOOT, A.M. VAN DER ; MULLALLY, M.M. ; FERNANDEZ-BALLESTER, G. ; SERRANO, L. ; QUAX, W.J.** *Prot.Eng.Des.Sel.,* 2004, vol. 9, 673-680
- **REIS, C.R. ; SLOOT A.M. VAN DER ; SZEGEZDI, E. ; NATONI, A. ; TUR, V. ; COOL, R.H. ; SAMALI, A. ; SERRANO, L. ; QUAX, W.J.** *Biochemistry,* 2009, vol. 10, 2180-2191
- **TUR, V. ; SLOOT, A.M. VAN DER ; REIS, C.R. ; SZEGEZDI, E. ; COOL, R.H. ; SAMALI, A. ; SERRANO, L. ; QUAX, W.J.** *JBC,* 2008, vol. 29, 20560-20568

- **NOBBMANN, U. ; CONNAH, M. ; FISH, B. ; VARLEY, P. ; GEE, C. ; MULOT, S. ; CHEN, J. ; ZHOU, L. ; LU, Y. ; SHENG, F.** *Biotech.Gen.Eng.Rev.,* 2007, vol. 24, 117-128
- **REIS, C.R. ; ASSEN, A.H.G. VAN ; QUAX, W.J. ; COOL, R.H.** *Mol Cell Prot,* 17 September 2010
- **TA et al.** *PNAS,* 2010, vol. 47, 20281-20286
- **SATO et al.** *Current Opinions in Biotechnology,* 2010, vol. 17, 638-642
- **LOCKSLEY et al.** *Cell,* 2001, vol. 104, 487-501
- **BODMER et al.** *Trends Biochem. Sci.,* 2002, vol. 27, 19-26
- **RIEUX-LAUCAT et al.** *Current Opinion in Immunology,* 2003, vol. 15, 325
- **MACKAY ; AMBROSE.** *Cytokine and growth factor reviews,* 2003, vol. 14, 311
- **MACKAY ; KALLED.** *Current opinion in Immunology,* 2002, vol. 14, 783-790
- **PEPPEL K et al.** *J Exp Med,* 1991, vol. 174 (6), 1483-9
- **STEED PM et al.** *Science,* 2003, vol. 301 (5641), 1895-8
- **BEKKER PJ et al.** *J Bone Miner Res,* 2004, vol. 19 (7), 1059-66
- **MCCLUNG MR et al.** *N Engl J Med,* vol. 354 (8), 821-31
- *Clinical development of anti-RANKL therapy. Arthritis Research & Therapy,* 2007, vol. 9 (1), 7
- **SCHRAMEK et al.** *Nature,* 2010, vol. 0 (0), 1-7
- **GONZALEZ-SUAREZ et al.** *Nature,* 2010, vol. 0 (0), 1-7